# EUROPEAN PATENT APPLICATION

(11) **EP 3 375 781 A1**
(43) Date of publication of application: **19.09.2018**
(21) Application number: 16827180.7
(22) Date of filing: 12.07.2016
(51) Int. Cl.: C07D 409/14, C07D 413/14, C07D 417/14, A61K 31/438, A61K 31/538, A61P 11/06, A61P 11/00, A61P 29/00

(54) **BENZO RING DERIVATIVE WITH B2 RECEPTOR AGONIST AND M3 RECEPTOR ANTAGONIST ACTIVITIES AND USE THEREOF IN MEDICINE**

(30) Priority: 21.07.2015 CN 201510427700
(71) Applicant: Sichuan Haisco Pharmaceutical Co., Ltd., Sichuan 611130 (CN)
(72) Inventor: WEI, Yonggang, Chengdu City Sichuan 611130 (CN); QIU, Guanpeng, Chengdu City Sichuan 611130 (CN); LEI, Bolin, Chengdu City Sichuan 611130 (CN); LU, Yonghua, Sichuan 611130 (CN); ZHENG, Suxin, Chengdu City Sichuan 611130 (CN)
(74) Representative: Wibbelmann, Jobst
(86) International application number: PCT/CN2016/089753
(87) International publication number: WO 2017/012489

(57) **Abstract**

A compound represented by general formula (I), or a stereoisomer, a hydrate, a metabolite, a solvate, a pharmaceutically acceptable salt, a cocrystal, or a prodrug thereof, the preparation method thereof, and use thereof in the manufacture of a medicament for treatment of an airway obstructive disease, 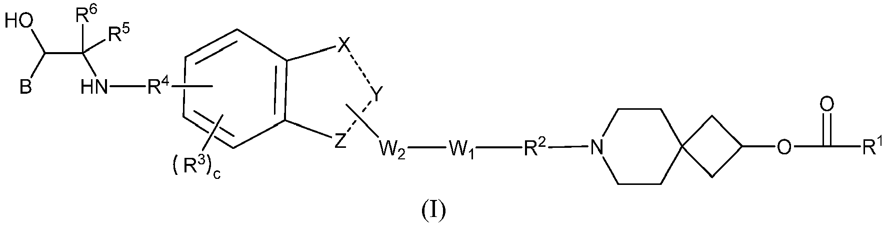 wherein the substituents are defined as in the specification.

## Description

### TECHNICAL FIELD

Embodiments of the present invention relate to a benzocyclic derivative and a preparation method and medical use thereof, and in particular relate to a new benzocyclic derivative having dual activity of a muscarine receptor M₃ antagonist and a β₂-adrenergic receptor agonist, or a stereoisomer, a hydrate, a solvate, a metabolite, a pharmaceutically acceptable salt, a cocrystal or a prodrug thereof, a pharmaceutical composition containing the same, and medical use thereof.

### BACKGROUND ART

Bronchodilators have been playing an important role in treatment of respiratory diseases such as the chronic obstructive pulmonary disease (COPD) and asthma. Bronchodilators widely used in clinical scenarios include muscarinic receptor antagonists and β₂-adrenergic agonists. Muscarinic receptor antagonists exert a bronchial dilating function by lowering the vagal cholinergic level in airway smooth muscle. Currently used inhalational muscarinic receptor antagonists include ipratropium bromide, oxitropium bromide, glycopyrronium bromide, tiotropium bromide, aclidinium bromide, and umeclidinium bromide. β₂-adrenergic agonists lead to bronchial dilatation by stimulating adrenergic receptors in airway smooth muscle, and reverse the effect of bronchial-constricting agents on various media such as acetylcholine. Currently used β₂-adrenergic agonists include salbutamol, salmeterol, arformoterol, formoterol, vilanterol and indacaterol. These drugs not only improve the function of lung, but also improve the quality of life of patients and arrest deterioration of the diseases.

Extensive clinical studies have shown that the combinational use of a muscarinic receptor antagonist and a β₂-adrenergic agonist is more effective than use of either of these therapeutic agents alone. Currently, muscarinic receptor antagonists and β₂-adrenergic agonists are clinically prepared into a combination formulation for treatment of asthma and moderate-to-severe COPDs. Such combination formulations mainly include Anoro Ellipta (umeclidinium bromide / vilanterol), Ultibro Breezhaler (glycopyrronium bromide / indacaterol), ipratropium bromide / salbutamol, and the like. Although the combination formulations have a better therapeutic effect than either individual drug contained therein, their preparation has strict requirements.

Therefore, it is desirable to develop a medicament having a dual effect of both muscarinic receptor antagonist and β₂-adrenergic agonist, which has the pharmacological advantages of both ingredients and also has a single molecular pharmacokinetics. These compounds are administered in the form of a single therapeutic agent, and can produce a bronchodilatory effect by two different and possibly synergistic modes of action. In addition, compounds having the dual effect of both muscarinic receptor antagonist and β₂-adrenergic agonist (MABA) can also be combined with corticosteroid (ICS) anti-inflammatory agents, to provide, as two therapeutic agents (MABA/ICS), a triplet therapeutic effect (Expert Opin. Investig. Drugs (2014) 23(4):453-456).

Therefore, it is necessary to develop a novel medicament having the dual activity of both muscarinic receptor antagonist and β₂-adrenergic agonist, to provide a single therapeutic agent or combination formulation that is more efficacious, thereby providing patients with more options of clinical medication.

### SUMMARY OF INVENTION

Embodiments of the present invention provide a compound represented by general formula (I), 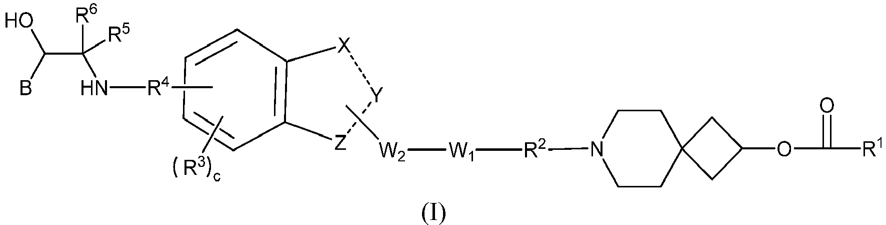 wherein
R¹ is selected from the group consisting of 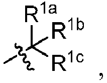 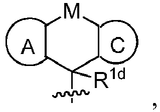 or 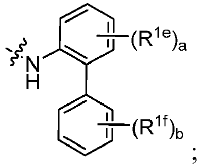
R^{1a} and R^{1b} are each independently selected from the group consisting of H, a C₂₋₆ alkenyl, a C₂₋₆ alkynyl, a C₃₋₁₀ carbocycle, a 3- to 8-membered heterocycle, a C₃₋₁₀ carbocyclyl-C₁₋₄ alkylene, or a 3- to 8-membered heterocyclyl-C₁₋₄ alkylene, wherein the alkenyl, alkynyl, alkylene, carbocycle or heterocycle is optionally further substituted with 0 to 4 substituents selected from the group consisting of F, Cl, Br, I, CH₂F, CHF₂, CF₃, OH, OCH₂F, OCHF₂, OCF₃, NH₂, carboxyl, cyano, nitro, a C₁₋₄ alkyl, a C₁₋₄ alkoxy, a -O(=O)C₁₋₄ alkyl, a -(=O)C₁₋₄ alkyl, a -OC₃₋₆ cycloalkyl or a C₁₋₄ alkylthio, wherein the heterocycle contains 1 to 3 hetero atoms selected from N, O or S; with the proviso that R^{1a} and R^{1b} are not both H;
R^{1c} is selected from the group consisting of H, hydroxy, cyano, NH₂, a C₁₋₆ alkyl, a C₁₋₆ alkoxy, a C₁₋₆ alkylthio or -C(=O)NH₂, wherein the NH₂, -C(=O)NH₂, or alkyl is optionally further substituted with 0 to 2 substituents selected from the group consisting of F, Cl, Br, I, hydroxy, cyano or a C₁₋₄ alkyl;
ring A and ring C are each independently selected from a C₆₋₁₀ carbocycle or a 5- to 10-membered heterocycle which is optionally further substituted with 0 to 4 substituents selected from the group consisting of F, Cl, Br, I, a C₁₋₄ alkyl or a C₁₋₄ alkoxy, wherein the heterocycle contains 1 to 3 heteroatoms selected from N, O or S;
M is selected from the group consisting of a bond, -CH₂-, -CH₂CH₂-, -O-, -S-, -OCH₂-, -SCH₂- or -CH=CH-;
R^{1d} is selected from the group consisting of H, hydroxy, -CH₂OH or a C₁₋₄ alkyl;
R^{1e} and R^{1f} are each independently selected from the group consisting of F, Cl, Br, I, CF₃, NH₂, OH, carboxyl, cyano, a C₁₋₄ alkyl, a C₁₋₄ alkoxy, a C₁₋₄ alkylthio, -NHC₁₋₄ alkyl, -N(C₁₋₄ alkyl)₂, -S(=O)-C₁₋₄ alkyl, -S(=O)₂-C₁₋₄ alkyl, or -C(=O)O-C₁₋₄ alkyl;
R² is selected from the group consisting of a C₁₋₆ alkylene, a C₂₋₆ alkenylene or a C₂₋₆ alkynylene, wherein the alkylene, alkenylene or alkynylene is optionally further substituted with 0 to 5 substituents selected from the group consisting of F, Cl, Br, I, OH, cyano, a C₁₋₄ alkyl, a C₁₋₄ alkoxy, phenyl or phenyl-C₁₋₄ alkylene;
W₁ is selected from the group consisting of a bond, -O-, -C(=O)O-, -OC(=O)-, -S-, -S(=O)-, -S(=O)₂-, -C(=O)NR^{w}-, -NR^{w}C(=O)-, -OC(=O)NR^{w}-, -NR^{w}C(=O)O-, -NR^{w}C(=O)NR^{w}-, -NR^{w}S(=O)₂-, -S(=O)₂NR^{w}-, -NR^{w}S(=O)₂NR^{w}-, or -NR^{w}-;
W₂ is selected from the group consisting of a bond or 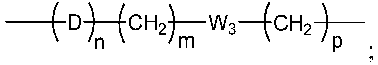
D is selected from a C₆₋₁₀ carbocyclene group or a 5- to 10-membered heterocyclene group, wherein the heterocyclene group contains 1 to 3 heteroatoms selected from N, O or S, and the carbocyclene or heterocyclene group is optionally further substituted with 0 to 4 substituents selected from the group consisting of F, Cl, Br, I, OH, NH₂, carboxyl, cyano, nitro, a C₁₋₄ alkyl, a C₂₋₄ alkynyl, a C₁₋₄ alkoxy, a -OC₃₋₆ cycloalkyl, a C₁₋₄ alkylthio, a -S(=O)-C₁₋₄ alkyl, a -S(=O)₂-C₁₋₄ alkyl, a -C(=O)-C₁₋₄ alkyl, a -C(=O)O-C₁₋₄ alkyl, a -OC(=O)-C₁₋₄ alkyl or a -C(=O)NH₂, where the alkyl, alkoxy, cycloalkyl, alkynyl, NH₂ and -C(=O)NH₂ are optionally further substituted with 0 to 4 substituents selected from the group consisting of F, Cl, Br, I, CF₃, a C₁₋₄ alkyl, a C₁₋₄ alkoxy or a -C(=O)-C₁₋₄ alkyl;
W₃ is selected from the group consisting of a bond, -O-, -C(=O)O-, -OC(=O)-, -S-, -S(=O)-, -S(=O)₂-, -C(=O)NR^{w}-, -NR^{w}C(=O)-, -OC(=O)NR^{w}-, -NR^{w}C(=O)O-, -NR^{w}C(=O)NR^{w}-, -NR^{w}S(=O)₂-, -S(=O)₂NR^{w}-, -NR^{w}S(=O)₂NR^{w}-, or -NR^{w}-;
R^{W} is selected from the group consisting of H or a C₁₋₄ alkyl;
X, Y and Z are each independently selected from the group consisting of -N-, -NR^{x1}-, -CR^{x1}R^{x2}-, -CR^{x1}R^{x2}CR^{x3}R^{x4}-, -CR^{x1}=CR^{x2}-, -S-, -O-, and -C(O)-;
R^{x1}, R^{x2}, R^{x3} or R^{x4} is each independently selected from the group consisting of H or a C₁₋₄ alkyl;
R³ is each independently selected from the group consisting of F, Cl, Br, I, OH, NH₂, carboxy, cyano, nitro, a C₁₋₄ alkyl, a C₂₋₄ alkenyl, a C₂₋₄ alkynyl, a C₁₋₄ alkoxy, a -OC₃₋₆ cycloalkyl, a C₁₋₄ alkylthio, a -S(=O)-C₁₋₄ alkyl, a -S(=O)₂-C₁₋₄ alkyl, a -C(=O)-C₁₋₄ alkyl, a -C(=O)O-C₁₋₄ alkyl, a -OC(=O)-C₁₋₄ alkyl or a -C(=O)NH₂, wherein the alkyl, alkoxy, cycloalkyl, alkenyl, alkynyl, NH₂ and -C(=O)NH₂ are optionally further substituted with 0 to 4 substituents selected from the group consisting of F, Cl, Br, I, CF₃, a C₁₋₄ alkyl, a C₁₋₄ alkoxy or a -C(=O)-C₁₋₄ alkyl;
R⁴ is selected from a C₁₋₆ alkylene which is optionally further substituted with 0 to 5 substituents selected from R^{4a};
R^{4a} is selected from the group consisting of F, Cl, Br, I, cyano, OH, a C₁₋₄ alkyl, a C₁₋₄ alkoxy, phenyl or phenyl-C₁₋₄ alkylene;
alternatively, two R^{4a} may form a 3- to 6-membered carbocycle together with the atoms to which they are attached, wherein the carbocycle is optionally further substituted with 0 to 5 substituents selected from the group consisting of F, Cl, Br, I, cyano, OH, a C₁₋₄ alkyl or a C₁₋₄ alkoxy;
R⁵ and R⁶ are each independently selected from the group consisting of H or a C₁₋₄ alkyl; 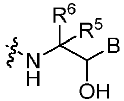 represents a β-adrenergic receptor binding group;
in formula (I), "------" is a single or double bond;
a is 0, 1, 2, 3, 4 or 5;
b is 0, 1, 2, 3 or 4;
c is 0, 1, 2, 3 or 4;
n is 0 or 1;
m is 0, 1, 2, 3, 4, 5 or 6, with the proviso that when n is 0, m is 1, 2, 3, 4, 5 or 6; and p is 0, 1, 2, 3, 4, 5 or 6,
or a stereoisomer, a hydrate, a metabolite, a solvate, a pharmaceutically acceptable salt, a cocrystal, or a prodrug thereof.

A preferred embodiment of the present invention is a compound represented by general formula (I), wherein: 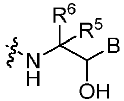 represents a β-adrenergic receptor binding group;
B is preferably selected from the group consisting of 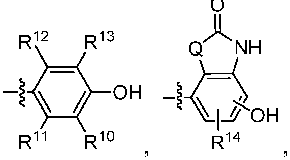 or 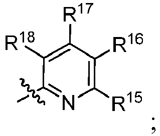 wherein R¹⁰, R¹¹, R¹², R¹³, R¹⁴, R¹⁵, R¹⁶, R¹⁷ or R¹⁸ are each independently selected from the group consisting of H, F, Cl, Br, I, CF₃, OH, -CH₂OH, cyano, carboxy, a C₁₋₄ alkyl, a C₁₋₄ alkoxy, a -C(=O)C₁₋₄ alkyl, a -C(=O)OC₁₋₄ alkyl, -NHC(=O)H, -NHS(=O)₂-C₁₋₄ alkyl, -NHS(=O)₂-NH₂ or -NHS(=O)₂-NHC₁₋₄ alkyl; and Q is selected from the group consisting of -CR^{q1}=CR^{q2}-, -CR^{q1}R^{q2}CR^{q3}R^{q4}-, -O-, -S-, -OCR^{q1}R^{q2}-, -CR^{q1}R^{q2}O-, -SCR^{q1}R^{q2}-, or -CR^{q1}R^{q2}S-, wherein R^{q1}, R^{q2}, R^{q3} and R^{q4} are each independently selected from the group consisting of H, F, Cl, Br, I or a C₁₋₄ alkyl;
B is more preferably selected from the group consisting of 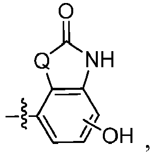 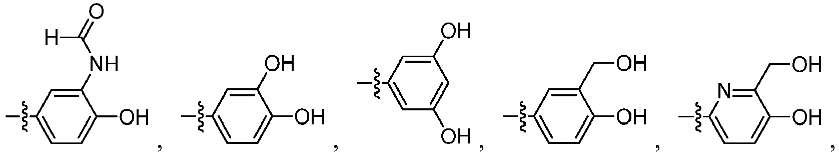 or 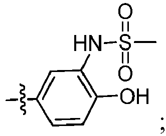 Q is selected from the group consisting of -CH=CH-, -CH₂CH₂-, -O-, -S-, -CH₂O-, -OCH₂-, -C(CH₃)₂O- or -OC(CH₃)₂-;
B is even more preferably selected from the group consisting of 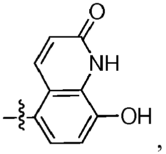 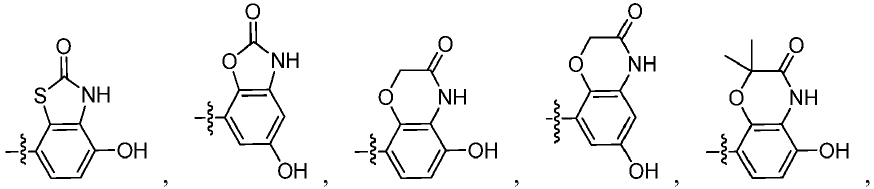 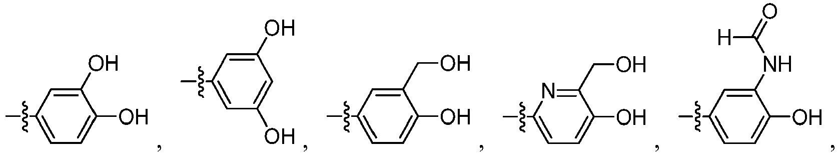 or 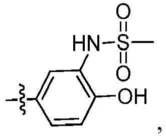
or a stereoisomer, a hydrate, a metabolite, a solvate, a pharmaceutically acceptable salt, a cocrystal, or a prodrug thereof.

A preferred embodiment of the present invention is a compound represented by general formula (I), wherein:
R¹ is selected from the group consisting of 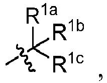 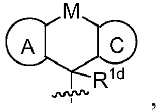 or 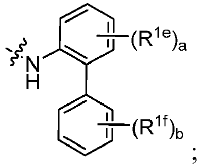
R^{1a} and R^{1b} are each independently selected from the group consisting of H, a C₂₋₄ alkenyl, a C₂₋₄ alkynyl, a C₃₋₇ carbocycle, a 3- to 6-membered heterocycle, a C₃₋₇ carbocyclyl-C₁₋₄ alkylene, or a 3- to 6-membered heterocyclyl-C₁₋₄ alkylene, preferably vinyl, ethynyl, propynyl, phenyl, cyclopropyl, cyclobutyl, cyclopentyl, cyclohexyl, cycloheptyl, benzyl, thienyl, furyl or pyridyl, wherein the alkenyl, alkynyl, alkylene, carbocycle, heterocycle, vinyl, ethynyl, propynyl, phenyl, cyclopropyl, cyclobutyl, cyclopentyl, cyclohexyl, cycloheptyl, benzyl, thienyl, furyl or pyridyl is optionally further substituted with 0 to 4 substituents selected from the group consisting of F, Cl, Br, I, CF₃, OH, NH₂, carboxyl, cyano, nitro, a C₁₋₄ alkyl, a C₁₋₄ alkoxy, a -O(=O)C₁₋₄ alkyl, a -(=O)C₁₋₄ alkyl, a -OC₃₋₆ cycloalkyl or a C₁₋₄ alkylthio, wherein the heterocycle contains 1 to 3 hetero atoms selected from N, O or S; with the proviso that R^{1a} and R^{1b} are not both H;
R^{1c} is selected from the group consisting of H, hydroxy, cyano, NH₂, a C₁₋₄ alkyl, a C₁₋₄ alkoxy, a C₁₋₄ alkylthio or -C(=O)NH₂, wherein the NH₂, -C(=O)NH₂, or alkyl is optionally further substituted with 0 to 2 substituents selected from the group consisting of F, Cl, Br, I, hydroxy, cyano or a C₁₋₄ alkyl;
ring A and ring C are each independently selected from a thiophene ring, a furan ring, a pyridine ring or a benzene ring, preferably a benzene ring, wherein the thiophene ring, furan, pyridine or benzene ring is optionally further substituted with 0 to 4 substituents selected from the group consisting of F, Cl, Br, I, a C₁₋₄ alkyl or a C₁₋₄ alkoxy, preferably with 0 to 4 substituents selected from the group consisting of F, Cl, Br, I, methyl, ethyl, methoxy or ethoxy;
M is selected from the group consisting of a bond, -CH₂-, -CH₂CH₂-, -O-, -S-, -OCH₂-, -SCH₂- or -CH=CH-; preferably a bond, -O- or -S-;
R^{1d} is selected from the group consisting of H, hydroxy, -CH₂OH or a C₁₋₄ alkyl; preferably H, hydroxy, -CH₂OH, methyl, ethyl, or propyl;
R^{1e} and R^{1f} are each independently selected from the group consisting of F, Cl, Br, I, CF₃, NH₂, OH, cyano, a C₁₋₄ alkyl, a C₁₋₄ alkoxy, a C₁₋₄ alkylthio, -NHC₁₋₄ alkyl, or -N(C₁₋₄ alkyl)₂;
R² is selected from the group consisting of a C₁₋₆ alkylene, a C₂₋₆ alkenylene or a C₂₋₆ alkynylene, preferably a C₁₋₆ alkylene, wherein the alkylene, alkenylene or alkynylene is optionally further substituted with 0 to 5 substituents selected from the group consisting of F, Cl, Br, I, OH, cyano, a C₁₋₄ alkyl, a C₁₋₄ alkoxy, phenyl or phenyl-C₁₋₄ alkylene;
W₁ is selected from the group consisting of a bond, -O-, -C(=O)O-, -OC(=O)-, -S-, -S(=O)-, -S(=O)₂-, -C(=O)NR^{w}-, -NR^{w}C(=O)-, -OC(=O)NR^{w}-, -NR^{w}C(=O)O-, -NR^{w}C(=O)NR^{w}-, -NR^{w}S(=O)₂-, -S(=O)₂NR^{w}-, -NR^{w}S(=O)₂NR^{w}-, or -NR^{w}-; preferably a bond, -O-, -C(=O)NR^{w}-, -NR^{w}C(=O)-, -OC(=O)NR^{w}- or -NR^{w}C(=O)O-; W₂ is selected from the group consisting of a bond or 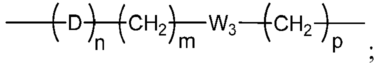
D is a phenylene which is optionally further substituted with 0 to 4 substituents selected from the group consisting of F, Cl, Br, I, OH, NH₂, carboxyl, cyano, nitro, a C₁₋₄ alkyl, a C₂₋₄ alkynyl, a C₁₋₄ alkoxy, a -OC₃₋₆ cycloalkyl, a C₁₋₄ alkylthio, a -S(=O)-C₁₋₄ alkyl, a -S(=O)₂-C₁₋₄ alkyl, a -C(=O)-C₁₋₄ alkyl, or a -C(=O)O-C₁₋₄ alkyl, wherein the alkyl, alkoxy, cycloalkyl, alkynyl, and NH₂ are optionally further substituted with 0 to 4 substituents selected from the group consisting of F, Cl, Br, I, CF₃, a C₁₋₄ alkyl, a C₁₋₄ alkoxy or a -C(=O)-C₁₋₄ alkyl;
W₃ is selected from the group consisting of a bond, -O-, -C(=O)O-, -OC(=O)-, -S-, -S(=O)-, -S(=O)₂-, -C(=O)NR^{w}-, -NR^{w}C(=O)-, -OC(=O)NR^{w}-, -NR^{w}C(=O)O-, -NR^{w}C(=O)NR^{w}-, -NR^{w}S(=O)₂-, -S(=O)₂NR^{w}-, -NR^{w}S(=O)₂NR^{w}-, or -NR^{w}-; preferably a bond, -O-, -C(=O)NR^{w}-, -NR^{w}C(=O)-, -OC(=O)NR^{w}- or -NR^{w}C(=O)O-;
R^{W} is selected from the group consisting of H or a C₁₋₄ alkyl;
X, Y and Z are each independently selected from the group consisting of -N-, -NR^{x1}-, -CR^{x1}R^{x2}-, -CR^{x1}R^{x2}CR^{x3}R^{x4}-, -CR^{x1}=CR^{x2}-, -S-, -O-, and -C(O)-;
R^{x1}, R^{x2}, R^{x3} or R^{x4} is each independently selected from the group consisting of H or a C₁₋₄ alkyl;
R³ is each independently selected from the group consisting of F, Cl, Br, I, OH, NH₂, carboxy, cyano, nitro, a C₁₋₄ alkyl, a C₂₋₄ alkynyl, a C₁₋₄ alkoxy, a -OC₃₋₆ cycloalkyl, a C₁₋₄ alkylthio, a -S(=O)-C₁₋₄ alkyl, a -S(=O)₂-C₁₋₄ alkyl, a -C(=O)-C₁₋₄ alkyl, or a -C(=O)O-C₁₋₄ alkyl, wherein the alkyl, alkoxy, cycloalkyl, alkynyl, and NH₂ are optionally further substituted with 0 to 4 substituents selected from the group consisting of F, Cl, Br, I, CF₃, a C₁₋₄ alkyl, a C₁₋₄ alkoxy or a -C(=O)-C₁₋₄ alkyl;
R⁴ is selected from a C₁₋₆ alkylene which is optionally further substituted with 0 to 5 substituents selected from R^{4a};
R^{4a} is selected from the group consisting of F, Cl, Br, I, cyano, OH, a C₁₋₄ alkyl, a C₁₋₄ alkoxy, phenyl or phenyl-C₁₋₄ alkylene;
alternatively, two R^{4a}s may form a 3- to 6-membered carbocycle together with the atoms to which they are attached, wherein the carbocycle is optionally further substituted with 0 to 5 substituents selected from the group consisting of F, Cl, Br, I, cyano, OH, a C₁₋₄ alkyl or a C₁₋₄ alkoxy;
R⁵ and R⁶ are each independently selected from the group consisting of H or a C₁₋₄ alkyl;
B is selected from the group consisting of 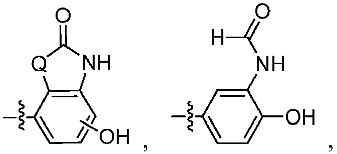 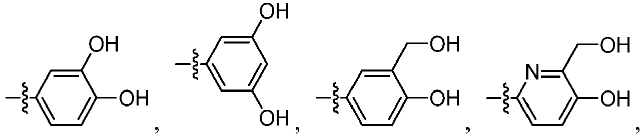 or 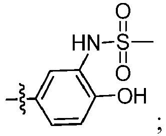 Q is selected from the group consisting of -CH=CH-, -CH₂CH₂-, -O-, -S-, -CH₂O-, -OCH₂-, -C(CH₃)₂O- or -OC(CH₃)₂-;
in formula (I), "------" is a single or double bond;
a is 0, 1, 2, 3, 4 or 5;
b is 0, 1, 2, 3 or 4;
c is 0, 1, 2, 3 or 4;
n is 0 or 1;
m is 0, 1, 2, 3, 4, 5 or 6, with the proviso that when n is 0, m is 1, 2, 3, 4, 5 or 6; and
p is 0, 1, 2, 3, 4, 5 or 6,
or a stereoisomer, a hydrate, a metabolite, a solvate, a pharmaceutically acceptable salt, a cocrystal, or a prodrug thereof.

A preferred embodiment of the present invention is a compound represented by general formula (I), wherein:
R¹ is selected from the group consisting of 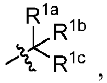 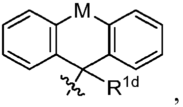 or 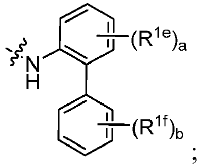
R^{1a} and R^{1b} are each independently selected from the group consisting of H, vinyl, ethynyl, propynyl, phenyl, cyclopropyl, cyclobutyl, cyclopentyl, cyclohexyl, cycloheptyl, benzyl, thienyl, furyl or pyridyl, preferably vinyl, ethynyl, phenyl, cyclobutyl, cyclopentyl, cyclohexyl, cycloheptyl, benzyl, or thienyl, wherein the vinyl, ethynyl, propynyl, phenyl, cyclopropyl, cyclobutyl, cyclopentyl, cyclohexyl, cycloheptyl, benzyl, thienyl, furyl or pyridyl is optionally further substituted with 0 to 4 substituents selected from the group consisting of F, Cl, Br, I, CF₃, OH, NH₂, carboxyl, cyano, nitro, a C₁₋₄ alkyl, a C₁₋₄ alkoxy, or a -O(=O)C₁₋₄ alkyl; with the proviso that R^{1a} and R^{1b} are not both H;
R^{1c} is selected from the group consisting of H, hydroxy, NH₂, methyl, ethyl, cyano, methylamino, dimethylamino, methoxy, ethoxy, -CH₂OH or -C(=O)NH₂, preferably H, hydroxy, NH₂, methyl, ethyl, methoxy or -CH₂OH;
M is selected from the group consisting of a bond, -CH₂-, -CH₂CH₂-, -O-, -S-, -OCH₂-, -SCH₂- or -CH=CH-; preferably a bond, -O- or -S-;
R^{1d} is selected from the group consisting of H, hydroxy, -CH₂OH, methyl or ethyl;
R^{1e} and R^{1f} are each independently selected from the group consisting of F, Cl, Br, I, CF₃, NH₂, OH, cyano, methyl, ethyl, methoxy, ethoxy, -NHCH₃ or -N(CH₃)₂;
R² is selected from the group consisting of methylene, ethylene, propylene, butylene or pentylene which is optionally further substituted with 0 to 5 substituents selected from the group consisting of F, Cl, Br, I, OH, cyano, methyl, ethyl, methoxy, or ethoxy;
W₁ is selected from the group consisting of a bond, -O-, -C(=O)NR^{w}-, -NR^{w}C(=O)-, -OC(=O)NR^{w}- or -NR^{w}C(=O)O-;
W₂ is selected from the group consisting of a bond or 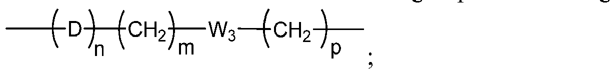
D is 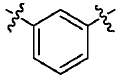 or 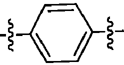 which is optionally further substituted with 0 to 4 substituents selected from the group consisting of F, Cl, Br, cyano, methyl, ethyl, propyl, isopropyl, ethynyl, CHF₂, CF₃, methoxy, ethoxy, -OCHF₂ or -OCF₃;
W₃ is selected from the group consisting of a bond, -O-, -C(=O)NR^{w}-, -NR^{w}C(=O)-, -OC(=O)NR^{w}- or -NR^{w}C(=O)O-;
R^{W} is selected from the group consisting of H, methyl, ethyl, or propyl;
X, Y and Z are each independently selected from the group consisting of -NH-, -N-, -CH₂-, -CH₂CH₂-, -CH-, -CH=CH-, -S-, -O- and -C(O)-;
R³ is each independently selected from the group consisting of F, Cl, Br, cyano, methyl, ethyl, propyl, isopropyl, ethynyl, CHF₂, CF₃, methoxy, ethoxy, -OCHF₂ or -OCF₃;
R⁴ is selected from the group consisting of methylene, ethylene, propylene, butylene, pentylene or 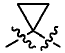 which is optionally further substituted with 0 to 5 substituents selected from the group consisting of F, Cl, Br, I, cyano, OH, methyl, ethyl, methoxy or ethoxy;
R⁵ and R⁶ are each independently selected from the group consisting of H, methyl or ethyl;
B is selected from the group consisting of 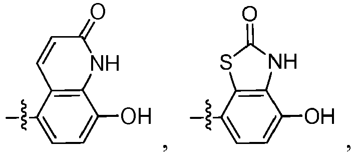 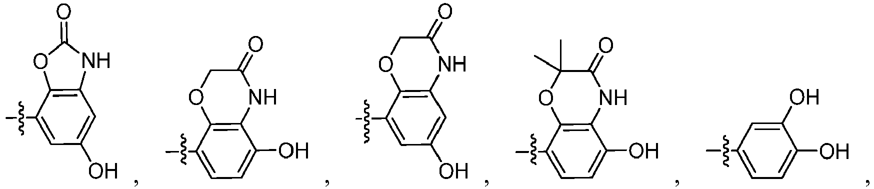 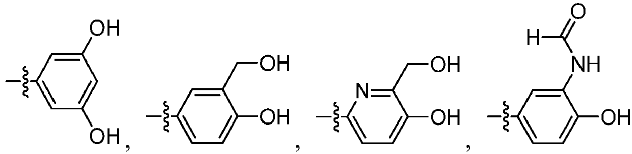 or 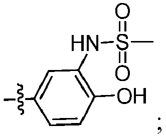
in formula (I), "------" is a single or double bond;
a is 0 or 1;
b is 0, 1, or 2;
c is 0, 1, or 2;
n is 0 or 1;
m is 0, 1, 2, 3, 4, or 5, with the proviso that when n is 0, m is 1, 2, 3, 4, or 5; and
p is 0, 1, 2, 3, or 4,
or a stereoisomer, a hydrate, a metabolite, a solvate, a pharmaceutically acceptable salt, a cocrystal, or a prodrug thereof.

A preferred embodiment of the present invention is a compound represented by general formula (I), wherein:
R¹ is selected from the group consisting of 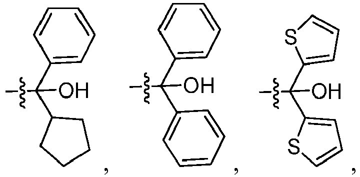 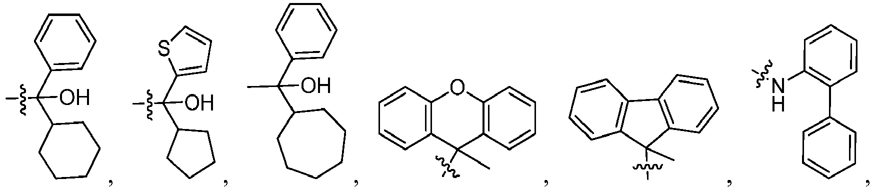 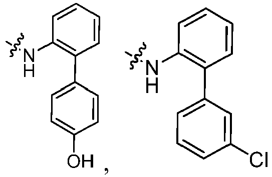 or 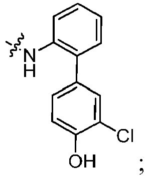
R² is selected from the group consisting of methylene, ethylene, propylene, -CH₂CH(CH₃)-, -CH(CH₃)CH₂-, butylene, -CH(CH₃)CH₂CH₂-, -CH₂CH(CH₃)CH₂-, -CH₂CH(CH₃)CH₂- or pentylene;
W₁ is selected from the group consisting of a bond, -O-, -C(=O)NR^{w}-, -NR^{w}C(=O)-, -OC(=O)NR^{w}- or -NR^{w}C(=O)O-;
W₂ is selected from the group consisting of a bond or 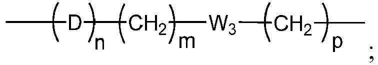
D is selected from the group consisting of 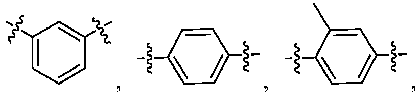 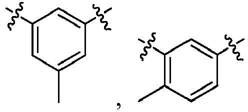 or 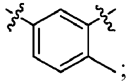
W₃ is selected from the group consisting of a bond, -O-, -C(=O)NR^{w}-, -NR^{w}C(=O)-, -OC(=O)NR^{w}- or -NR^{w}C(=O)O-;
R^{W} is selected from the group consisting of H, methyl, ethyl, or propyl;
X, Y and Z are each independently selected from the group consisting of -NH-, -N-, -CH₂-, -CH₂CH₂-, -CH-, -CH=CH-, -S-, -O- and -C(O)-;
R³ is each independently selected from the group consisting of F, Cl, Br, CHF₂, CF₃, cyano, methyl, ethyl, ethynyl, methoxy, ethoxy, -OCHF₂ or -OCF₃;
R⁴ is selected from the group consisting of methylene, ethylene, propylene, -CH₂CH(CH₃)-, -CH(CH₃)CH₂-, butylene, -CH(CH₃)CH₂CH₂-, -CH₂CH(CH₃)CH₂-, -CH₂CH(CH₃)CH₂-, pentylene or 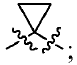
R⁵ and R⁶ are each independently selected from the group consisting of H, methyl or ethyl; preferably H;
B is selected from the group consisting of 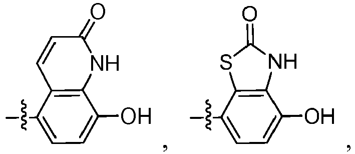 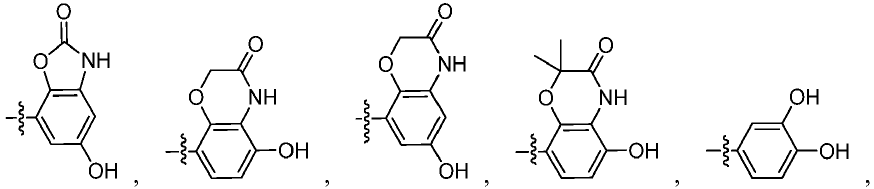 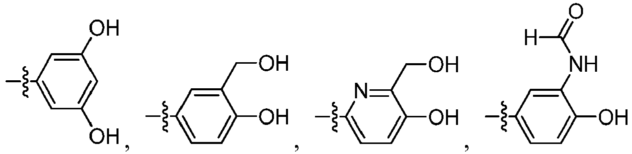 or 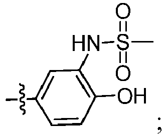
in formula (I), "------" is a single or double bond;
c is 0, 1, or 2;
n is 0 or 1;
m is 0, 1, 2, 3, 4, or 5, with the proviso that when n is 0, m is 1, 2, 3, 4, or 5; and
p is 0, 1, 2, 3, or 4,
or a stereoisomer, a hydrate, a metabolite, a solvate, a pharmaceutically acceptable salt, a cocrystal, or a prodrug thereof.

A preferred embodiment of the present invention relates to a compound represented by general formula (I) which includes, but is not limited to:

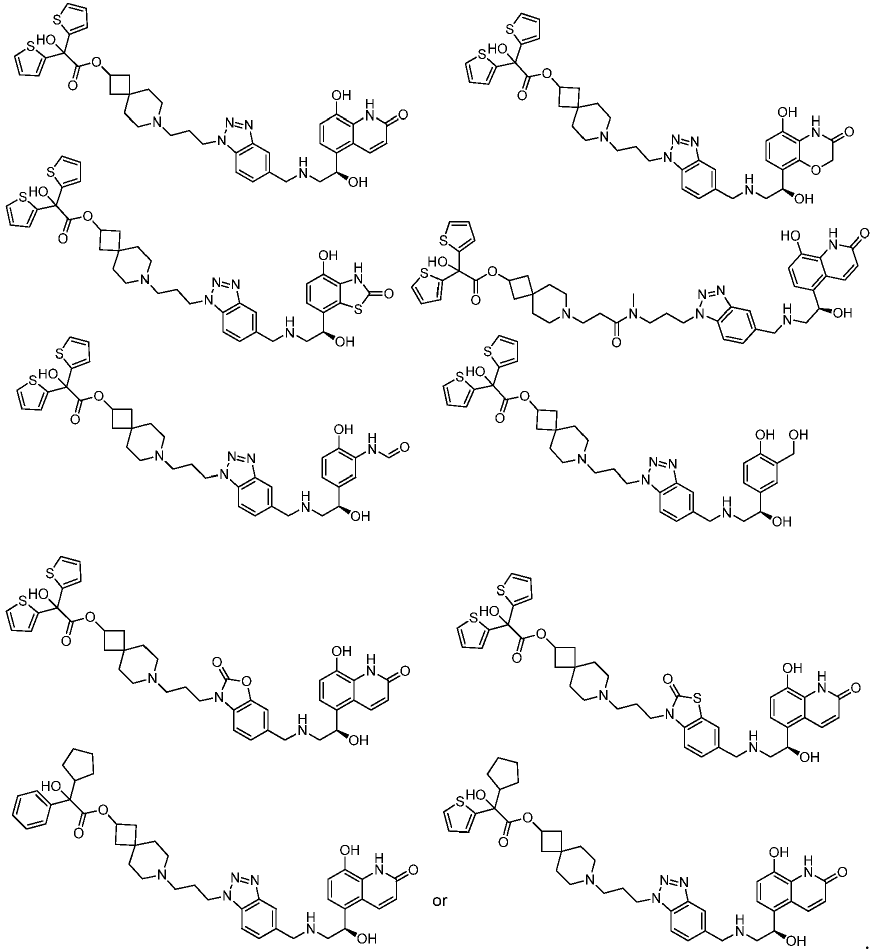

An embodiment of the present invention provides a pharmaceutical composition comprising: i) a therapeutically effective amount of any compound of general formula (I) or a stereoisomer, a hydrate, a metabolite, a solvate, a pharmaceutically acceptable salt, a cocrystal, or a prodrug thereof; and ii) a pharmaceutically acceptable carrier, diluent, adjuvant, vehicle, or excipient. The composition may further comprise one or more other therapeutic agents which are preferably selected from the group consisting of PDE4 inhibitors, muscarinic receptor antagonists, corticosteroids and β-adrenergic receptor agonists.

An embodiment of the present invention provides use of a compound of general formula (I), or a stereoisomer, a hydrate, a metabolite, a solvate, a pharmaceutically acceptable salt, a cocrystal, or a prodrug thereof, in the manufacture of a medicament for treatment of an airway obstructive disease, preferably asthma, chronic obstructive pulmonary disease (COPD) or bronchitis.

An embodiment of the present invention provides a method for treating an airway obstructive disease, comprising administrating a therapeutically effective amount of a compound of general formula (I), or a stereoisomer, a hydrate, a metabolite, a solvate, a pharmaceutically acceptable salt, a cocrystal, or a prodrug thereof as described above, or a pharmaceutical composition as described above.

An embodiment of the present invention provides a method for treating asthma, COPD or bronchitis, comprising administrating a therapeutically effective amount of a compound of general formula (I), or a stereoisomer, a hydrate, a metabolite, a solvate, a pharmaceutically acceptable salt, a cocrystal, or a prodrug thereof as described above, or a pharmaceutical composition as described above.

An embodiment of the present invention relates to an intermediate for preparing a compound of general formula (I) or a stereoisomer thereof, said intermediate being selected from compounds of general formula (II) or a stereoisomer thereof: 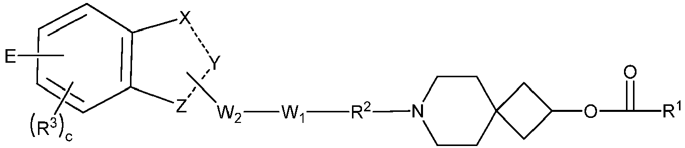 wherein:
R¹ is selected from the group consisting of 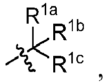 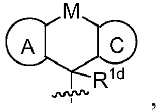 or 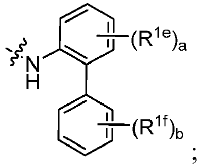
R^{1a} and R^{1b} are each independently selected from the group consisting of H, a C₂₋₆ alkenyl, a C₂₋₆ alkynyl, a C₃₋₁₀ carbocycle, a 3- to 8-membered heterocycle, a C₃₋₁₀ carbocyclyl-C₁₋₄ alkylene, or a 3- to 8-membered heterocyclyl-C₁₋₄ alkylene, wherein the alkenyl, alkynyl, alkylene, carbocycle or heterocycle is optionally further substituted with 0 to 4 substituents selected from the group consisting of F, Cl, Br, I, CH₂F, CHF₂, CF₃, OH, OCH₂F, OCHF₂, OCF₃, NH₂, carboxyl, cyano, nitro, a C₁₋₄ alkyl, a C₁₋₄ alkoxy, a -O(=O)C₁₋₄ alkyl, a -(=O)C₁₋₄ alkyl, a -OC₃₋₆ cycloalkyl or a C₁₋₄ alkylthio, wherein the heterocycle contains 1 to 3 hetero atoms selected from N, O or S; with the proviso that R^{1a} and R^{1b} are not both H;
R^{1c} is selected from the group consisting of H, hydroxy, cyano, NH₂, a C₁₋₆ alkyl, a C₁₋₆ alkoxy, a C₁₋₆ alkylthio or -C(=O)NH₂, wherein the NH₂, -C(=O)NH₂, or alkyl is optionally further substituted with 0 to 2 substituents selected from the group consisting of F, Cl, Br, I, hydroxy, cyano or a C₁₋₄ alkyl;
M is selected from the group consisting of a bond, -CH₂-, -CH₂CH₂-, -O-, -S-, -OCH₂-, -SCH₂- or -CH=CH-;
R^{1d} is selected from the group consisting of H, hydroxy, -CH₂OH or a C₁₋₄ alkyl;
ring A and ring C are each independently selected from a C₆₋₁₀ carbocycle or a 5- to 10-membered heterocycle which is optionally further substituted with 0 to 4 substituents selected from the group consisting of F, Cl, Br, I, a C₁₋₄ alkyl or a C₁₋₄ alkoxy, wherein the heterocycle contains 1 to 3 heteroatoms selected from N, O or S;
R^{1e} and R^{1f} are each independently selected from the group consisting of F, Cl, Br, I, CF₃, NH₂, OH, carboxyl, cyano, a C₁₋₄ alkyl, a C₁₋₄ alkoxy, a C₁₋₄ alkylthio, -NHC₁₋₄ alkyl, -N(C₁₋₄ alkyl)₂, -S(=O)-C₁₋₄ alkyl, -S(=O)₂-C₁₋₄ alkyl, or -C(=O)O-C₁₋₄ alkyl;
R² is selected from the group consisting of a C₁₋₆ alkylene, a C₂₋₆ alkenylene or a C₂₋₆ alkynylene, wherein the alkylene, alkenylene or alkynylene is optionally further substituted with 0 to 5 substituents selected from the group consisting of F, Cl, Br, I, OH, cyano, a C₁₋₄ alkyl, a C₁₋₄ alkoxy, phenyl or phenyl-C₁₋₄ alkylene;
W₁ is selected from the group consisting of a bond, -O-, -C(=O)O-, -OC(=O)-, -S-, -S(=O)-, -S(=O)₂-, -C(=O)NR^{w}-, -NR^{w}C(=O)-, -OC(=O)NR^{w}-, -NR^{w}C(=O)O-, -NR^{w}C(=O)NR^{w}-, -NR^{w}S(=O)₂-, -S(=O)₂NR^{w}-, -NR^{w}S(=O)₂NR^{w}-, or -NR^{w}-;
W₂ is selected from the group consisting of a bond or 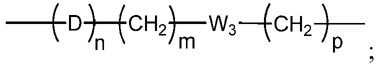
D is selected from the group consisting of a C₆₋₁₀ carbocyclene group or a 5- to 10-membered heterocyclene group, wherein the heterocyclene group contains 1 to 3 heteroatoms selected from N, O or S, and the carbocyclene or heterocyclene group is optionally further substituted with 0 to 4 substituents selected from the group consisting of F, Cl, Br, I, OH, NH₂, carboxyl, cyano, nitro, a C₁₋₄ alkyl, a C₂₋₄ alkynyl, a C₁₋₄ alkoxy, a -OC₃₋₆ cycloalkyl, a C₁₋₄ alkylthio, a -S(=O)-C₁₋₄ alkyl, a -S(=O)₂-C₁₋₄ alkyl, a -C(=O)-C₁₋₄ alkyl, a -C(=O)O-C₁₋₄ alkyl, a -OC(=O)-C₁₋₄ alkyl or a -C(=O)NH₂, where the alkyl, alkoxy, cycloalkyl, alkynyl, NH₂ and -C(=O)NH₂ are optionally further substituted with 0 to 4 substituents selected from the group consisting of F, Cl, Br, I, CF₃, a C₁₋₄ alkyl, a C₁₋₄ alkoxy or a -C(=O)-C₁₋₄ alkyl;
W₃ is selected from the group consisting of a bond, -O-, -C(=O)O-, -OC(=O)-, -S-, -S(=O)-, -S(=O)₂-, -C(=O)NR^{w}-, -NR^{w}C(=O)-, -OC(=O)NR^{w}-, -NR^{w}C(=O)O-, -NR^{w}C(=O)NR^{w}-, -NR^{w}S(=O)₂-, -S(=O)₂NR^{w}-, -NR^{w}S(=O)₂NR^{w}-, or -NR^{w}-;
R^{W} is selected from the group consisting of H or a C₁₋₄ alkyl;
X, Y and Z are each independently selected from the group consisting of -N-, -NR^{x1}-, -CR^{x1}R^{x2}-, -CR^{x1}R^{x2}CR^{x3}R^{x4}-, -CR^{x1}=CR^{x2}-, -S-, -O-, and -C(O)-;
R^{x1}, R^{x2}, R^{x3} or R^{x4} is each independently selected from the group consisting of H or a C₁₋₄ alkyl;
R³ is each independently selected from the group consisting of F, Cl, Br, I, OH, NH₂, carboxy, cyano, nitro, a C₁₋₄ alkyl, a C₂₋₄ alkenyl, a C₂₋₄ alkynyl, a C₁₋₄ alkoxy, a -OC₃₋₆ cycloalkyl, a C₁₋₄ alkylthio, a -S(=O)-C₁₋₄ alkyl, a -S(=O)₂-C₁₋₄ alkyl, a -C(=O)-C₁₋₄ alkyl, a -C(=O)O-C₁₋₄ alkyl, a -OC(=O)-C₁₋₄ alkyl or a -C(=O)NH₂, wherein the alkyl, alkoxy, cycloalkyl, alkenyl, alkynyl, NH₂ and -C(=O)NH₂ are optionally further substituted with 0 to 4 substituents selected from the group consisting of F, Cl, Br, I, CF₃, a C₁₋₄ alkyl, a C₁₋₄ alkoxy or a -C(=O)-C₁₋₄ alkyl;
E is selected from the group consisting of formyl, 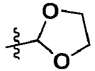 or 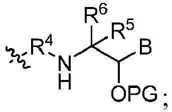
R⁴ is selected from a C₁₋₆ alkylene which is optionally further substituted with 0 to 5 substituents selected from R^{4a};
R^{4a} is selected from the group consisting of F, Cl, Br, I, cyano, OH, a C₁₋₄ alkyl, a C₁₋₄ alkoxy, phenyl or phenyl-C₁₋₄ alkylene;
alternatively, two R^{4a}s may form a 3- to 6-membered carbocycle together with the atoms to which they are attached, wherein the carbocycle is optionally further substituted with 0 to 5 substituents selected from the group consisting of F, Cl, Br, I, cyano, OH, a C₁₋₄ alkyl or a C₁₋₄ alkoxy;
R⁵ and R⁶ are each independently selected from the group consisting of H or a C₁₋₄ alkyl;
PG is a protective group for hydroxy; 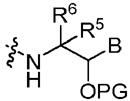 represents a β-adrenergic receptor binding group wherein the hydroxyl is protected by a TBS group;
in formula (II), "------" is a single or double bond;
a is 0, 1, 2, 3, 4 or 5;
b is 0, 1, 2, 3 or 4;
c is 0, 1, 2, 3 or 4;
n is 0 or 1;
m is 0, 1, 2, 3, 4, 5 or 6, with the proviso that when n is 0, m is 1, 2, 3, 4, 5 or 6; and
p is 0, 1, 2, 3, 4, 5 or 6.

An embodiment of the present invention relates to an intermediate for preparing a compound of general formula (I) or a stereoisomer thereof, said intermediate being selected from compounds of general formula (II) or a stereoisomer thereof, wherein:
R¹ is selected from the group consisting of 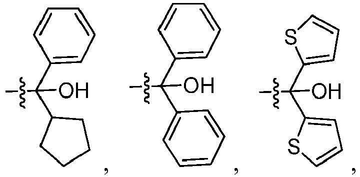 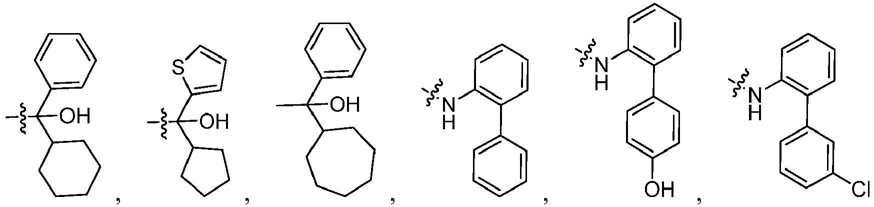 or 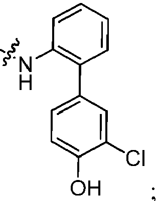
R² is selected from the group consisting of methylene, ethylene, propylene, -CH₂CH(CH₃)-, -CH(CH₃)CH₂-, butylene, -CH(CH₃)CH₂CH₂-, -CH₂CH(CH₃)CH₂-, -CH₂CH(CH₃)CH₂- or pentylene;
W₁ is selected from the group consisting of a bond, -O-, -C(=O)NR^{w}-, -NR^{w}C(=O)-, -OC(=O)NR^{w}- or -NR^{w}C(=O)O-;
W₂ is selected from the group consisting of a bond or 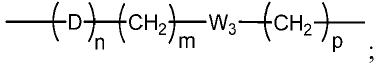
D is selected from the group consisting of 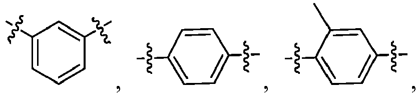 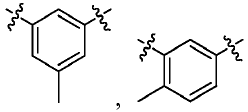 or 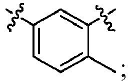
W₃ is selected from the group consisting of a bond, -O-, -C(=O)NR^{w}-, -NR^{w}C(=O)-, -OC(=O)NR^{w}- or -NR^{w}C(=O)O-;
R^{W} is selected from the group consisting of H, methyl, ethyl, or propyl;
X, Y and Z are each independently selected from the group consisting of -NH-, -N-, -CH₂-, -CH₂CH₂-, -CH-, -CH=CH-, -S-, -O- and -C(O)-;
R³ is selected from the group consisting of F, Cl, Br, CHF₂, CF₃, cyano, methyl, ethyl, ethynyl, methoxy, ethoxy, -OCHF₂ or -OCF₃;
E is selected from the group consisting of formyl, 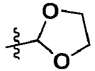 or 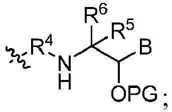
R⁴ is selected from the group consisting of methylene, ethylene, propylene, -CH₂CH(CH₃)-, -CH(CH₃)CH₂-, butylene, -CH(CH₃)CH₂CH₂-, -CH₂CH(CH₃)CH₂-, -CH₂CH(CH₃)CH₂-, 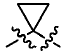 or pentylene;
R⁵ and R⁶ are each independently selected from the group consisting of H, methyl or ethyl;
B is selected from the group consisting of 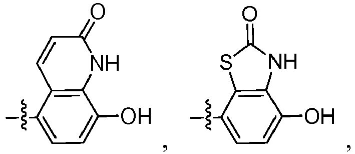 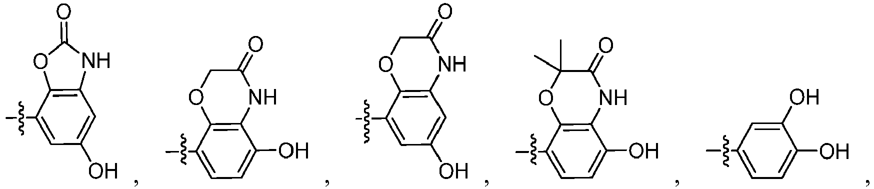 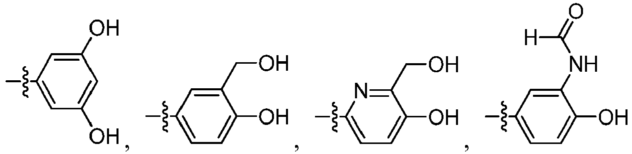 or 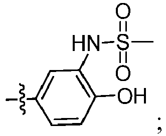
PG is TBS;
in formula (II), "------" is a single or double bond;
a is 0, 1, 2, 3, 4 or 5;
b is 0, 1, 2, 3 or 4;
c is 0, 1, 2, 3 or 4;
n is 0 or 1;
m is 0, 1, 2, 3, 4, 5 or 6, with the proviso that when n is 0, m is 1, 2, 3, 4, 5 or 6; and
p is 0, 1, 2, 3, 4, 5 or 6.

In a preferred embodiment of the present invention, compounds of general formula (II) are selected from, but not limited to:

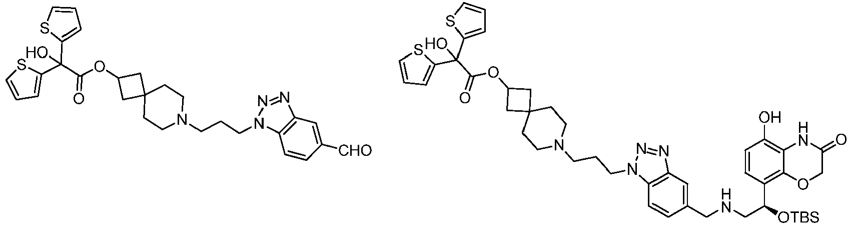

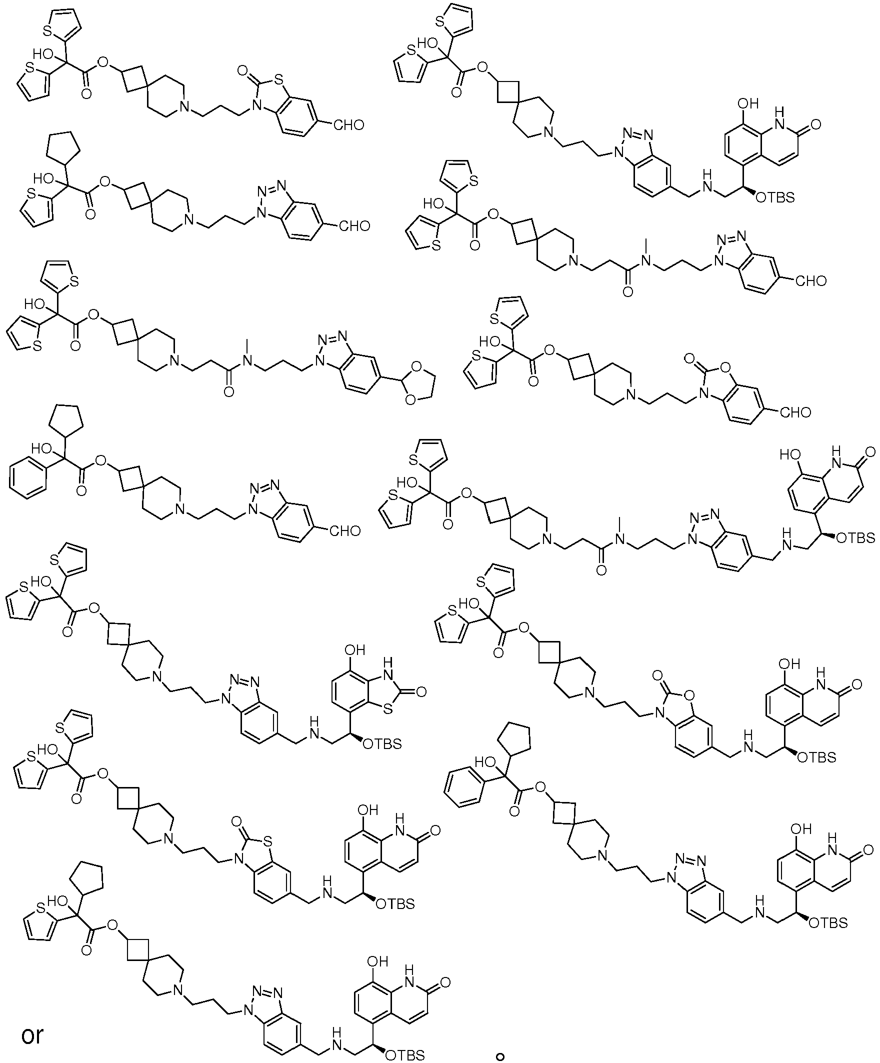

The ditrifluoroacetate of specific example compounds according to the present invention can be converted into their corresponding free-base form by dissolving it in a polar organic solvent (such as a mixed solvent of methanol and methylene chloride (v/v = 1/90)), adding an alkaline agent (such as a saturated sodium bicarbonate solution, a saturated sodium carbonate solution, or the like) to adjust the pH to alkaline, extracting the mixture with an organic solvent (such as dichloromethane, ethyl acetate, etc.) after stirring, and concentrating the organic phase under reduced pressure.

Unless otherwise indicated, the terms used throughout the specification and claims have the following meanings.

All of the carbon, hydrogen, oxygen, sulfur, nitrogen or halogen involved in the groups and compounds according to the present invention include their isotopes. All of the carbon, hydrogen, oxygen, sulfur, nitrogen or halogen involved in the groups and compounds according to the present invention are optionally further replaced by one or more of their corresponding isotopes, wherein the carbon isotopes include ¹²C, ¹³C and ¹⁴C, the hydrogen isotopes include protium (H), deuterium (D, also known as heavy hydrogen) and tritium (T, also known as superheavy hydrogen), the oxygen isotopes include ¹⁶O, ¹⁷O and ¹⁸O, the sulfur isotopes include ³²S, ³³S, ³⁴S and ³⁶S, the nitrogen isotopes include ¹⁴N and ¹⁵N, the fluorine isotopes include ¹⁹F, the chlorine isotopes include ³⁵Cl and ³⁷Cl, and the bromine isotopes include ⁷⁹Br and ⁸¹Br.

"Alkyl" means a linear or branched saturated monovalent hydrocarbon group, having in the main chain 1 to 10 carbon atoms, preferably 1 to 8 carbon atoms, more preferably 1 to 6 carbon atoms, even more preferably 1 to 4 carbon atoms, and most preferably 1 to 2 carbon atoms. Examples of alkyl include, but are not limited to, methyl, ethyl, n-propyl, isopropyl, n-butyl, isobutyl, sec-butyl, t-butyl, n-pentyl, 2-pentyl, 3-pentyl, 2-methyl-2-butyl, 3-methyl-2-butyl, n-hexyl, n-heptyl, n-octyl, n-nonyl, n-decyl, and the like. The alkyl may be optionally further substituted with 0 to 5 substituents selected from the group consisting of F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}, wherein R¹⁹ and R^{19a} are each independently selected from the group consisting of H, hydroxyl, amino, carboxy, C₁₋₈ alkyl, C₁₋₈ alkoxy, C₂₋₈ alkenyl, C₂₋₈ alkynyl, 3- to 10-membered carbocycle, 4- to 10-membered heterocyclyl, 3- to 10-membered carbocyclyloxy, or 4- to 10-membered heterocyclyloxy, k is selected from 0, 1, 2, 3, 4 or 5, and j is selected from 0, 1 or 2. Alkyl, k, j, or R¹⁹ and R^{19a} used throughout the specification are defined as above.

"Alkylene" refers to a linear and branched saturated divalent hydrocarbon group, including -(CH₂)ᵥ- (v is an integer from 1 to 10). Examples of alkylene include, but are not limited to, methylene, ethylene, propylene, butylene and the like. The alkylene may be optionally further substituted with 0 to 5 substituents selected from the group consisting of F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. When the number of substituents in the alkylene is 2 or more, the substituents may fuse together to form a cyclic structure. Alkylene used throughout the specification is defined as above.

"Alkoxy" means a monovalent group -O-alkyl, wherein the alkyl is as defined above. Examples of alkoxy include, but are not limited to, methoxy, ethoxy, 1-propoxy, 2-propoxy, 1-butoxy, 2-methyl-1-propoxy, 2-butoxy, 2-methyl-2-propoxy, 1-pentyloxy, 2-pentyloxy, 3-pentyloxy, 2-methyl-2-butoxy, 3-methyl-2-butoxy, 3-methyl-1-butoxy, 2-methyl-1-butoxy, and the like.

"Alkenyl" refers to a linear or branched unsaturated monovalent hydrocarbon group having at least one, generally one, two or three carbon-carbon double bonds, and having 2 to 10 carbon atoms, more preferably 2 to 6 carbon atoms, and even more preferably 2 to 4 carbon atoms in the main chain. Examples of alkenyl include, but are not limited to, vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadienyl, 1,3-pentadienyl, 1,4-pentadienyl, 1,4-hexadienyl, and the like. The alkenyl may be optionally further substituted with 0 to 5 substituents selected from the group consisting of F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. Alkenyl used throughout the specification is defined as above.

"Alkenylene" is a divalent group derived from alkenyl, wherein the alkenyl is defined as above.

"Alkynyl" refers to a linear or branched unsaturated monovalent hydrocarbon group having at least one, generally one, two or three carbon-carbon triple bonds, and having 2 to 10 carbon atoms, more preferably 2 to 6 carbon atoms, and even more preferably 2 to 4 carbon atoms in the main chain. Examples of alkynyl include, but are not limited to, ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, and the like. The alkynyl may be optionally further substituted with 0 to 5 substituents selected from the group consisting of F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. Alkynyl used throughout the specification is defined as above.

"Alkynylene" is a divalent group derived from alkynyl, wherein the alkynyl is defined as above.

"Cycloalkyl" refers to a saturated monovalent cyclic hydrocarbon group which generally has 3 to 10 carbon atoms. Non-limiting examples thereof include cyclopropyl, cyclobutyl, cyclopentyl, cyclohexyl, cycloheptyl, and the like. The cycloalkyl may be optionally further substituted with 0 to 5 substituents selected from the group consisting of F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. Cycloalkyl used throughout the specification is defined as above.

"Cycloalkylene" is a divalent group derived from cycloalkyl, wherein the cycloalkyl is defined as above.

"Carboncyclyl" refers to a saturated or unsaturated aromatic or non-aromatic cyclic group which may be a 3- to 10-membered monocyclic ring, a 4- to 12-membered bicyclic ring, or a 10- to 15-membered tricyclic ring system, and may be attached with a bridged or spiro ring. Non-limiting examples include cyclopropyl, cyclobutyl, cyclopentyl, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, cyclohexadienyl, cycloheptyl, cyclooctyl, cyclononyl, cyclodecyl, cycloundecyl, cyclododecyl, phenyl, naphthyl, 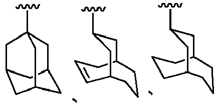 and 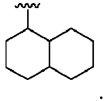 The carboncyclyl may be optionally further substituted with 0 to 5 substituents selected from the group consisting of F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. Carboncyclyl used throughout the specification is defined as above.

"Heterocyclyl" means a saturated or unsaturated, aromatic or non-aromatic ring having 1 to 4 heteroatoms selected from N, O or S, and the aromatic or non-aromatic ring may be a 3- to 10-membered monocyclic ring, a 4- to 12-membered bicyclic ring, or a 10- to 15-membered tricyclic ring system. A 3- to 8-membered heterocycle is preferred. The optional N and S substituted in the ring of a heterocyclyl may be oxidized to various oxidative states. A heterocyclyl may be attached to a heteroatom or a carbon atom, and may be attached with a bridged or spiro ring. Non-limiting examples include epoxyethyl, epoxypropyl, aziridinyl, oxetanyl, azetidinyl, thietanyl, 1,3-dioxolanyl, 1,4-dioxolanyl, 1,3-dioxetanyl, azepanyl, oxepanyl, thiepanyl, oxazepinyl, diazepinyl, thiazepinyl, pyridyl, piperidinyl, homopiperidyl, furyl, thienyl, pyranyl, N-alkylpyrrolyl, pyrimidinyl, pyrazinyl, pyridazinyl, piperazinyl, homopiperazinyl, imidazolyl, piperidinyl, morpholinyl, thiomorpholinyl, thioxanyl, 1,3-dithiyl, dihydrofuryl, dihydropyranyl, dithiolanyl, tetrahydrofuranyl, tetrahydrothienyl, tetrahydropyranyl, tetrahydrothiopyranyl, tetrahydropyrrolyl, tetrahydroimidazolyl, tetrahydrothiazolyl, tetrahydropyranyl, benzimidazolyl, benzopyridyl, pyrrolopyridyl, benzodihydrofuranyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydrothienyl, pyrazolidinyl, imidazolinyl, imidazolidinyl, 1,2,3,4-tetrahydroisoquinolinyl, 3-azabicyclo[3.1.0]hexyl, 3-azabicyclo[4.1.0]heptyl, azabicyclo[2.2.2]hexyl, 3H-indolylquinolizinyl, N-pyridylurea, 1,1-dioxothiomorpholinyl, azabicyclo[3.2.1]octanyl, azabicyclo[5.2.0]nonanyl, oxatricyclo[5.3.1.1]dodecanyl, azadamantyl, and oxaspiro[3.3]heptyl. The heterocyclyl may be optionally further substituted with 0 to 5 substituents selected from the group consisting of F, Cl, Br, I, =O, -CH₂F, -CHF₂, -CF₃, -OCH₂F, -OCHF₂, -OCF₃, hydroxy, -SR¹⁹, nitro, cyano, isocyanato, alkyl, hydroxyalkyl, alkoxy, carbocyclyl, heterocyclyl, C₂₋₈ alkenyl, C₂₋₈ alkynyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. Heterocyclyl used throughout the specification is defined as above.

A "β-adrenergic receptor binding group" refers to groups capable of binding a β-adrenergic receptor, see for example the review β-adrenergic receptors in Comprehensive Medicinal Chemistry, 1990, B.E.Main, p187 (Pergamon Press), and also see WO/2005092841, US/20050215542, WO/2005070872, WO/2006023460, WO/2006051373, WO/2006087315, and WO/2006032627. Non-limiting examples
include 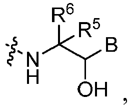 wherein R⁵ and R⁶ are each independently selected from the group consisting of H or a C₁₋₄ alkyl, and B is selected from the group consisting of 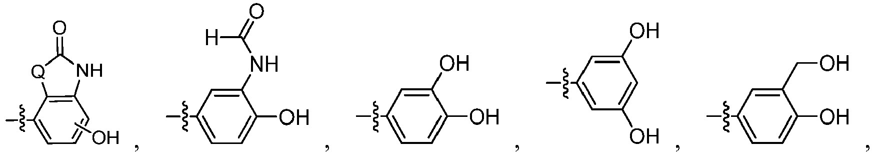 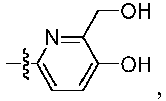 or 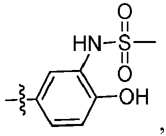 with Q selected from the group consisting of -CH=CH-, -CH₂CH₂-, -O-, -S-, -CH₂O-, -OCH₂-, -C(CH₃)₂O-, or -OC(CH₃)₂-.

A "hydroxyl protective group" refers to a protective group for hydroxyl, which is suitable for protecting a hydroxyl group from undergoing a chemical reaction but is easily removed after the desired chemical reaction is completed at other parts of the molecule. Non-limiting examples include, but are not limited to, trialkylsilyl (such as trimethylsilyl, triethylsilyl, triisopropylsilyl, t-butyldimethylsilyl or t-butyldiphenylsilyl), benzyl, a C₁₋₆ alkylacyl (e.g., formyl, acetyl, propionyl, etc.), or a C₆₋₁₀ arylacyl.

"Optional" or "optionally" means that the event or scenario described by them may, but does not have to, happen, and encompasses both cases where the event or scenario happens and does not happen. For example, "an alkyl optionally substituted with F" means that the alkyl may, but does not have to, be substituted with F, encompassing both the case where the alkyl is substituted with F and the case where the alkyl is not substituted with F.

A "pharmaceutically composition" refers to a mixture of one or more of the compounds according to the present disclosure or pharmaceutically/physiologically acceptable salts thereof with additional components, wherein the additional components include pharmaceutically/physiologically acceptable carriers or excipients.

"Carrier" means a vehicle or diluent that does not cause significant stimulation to an organism and does not eliminate the biological activity and characteristics of a given compound.

"Excipient" means an inert substance added into a pharmaceutical composition to further facilitate administration of a compound. Examples thereof include, but are not limited to, calcium carbonate, calcium phosphate, various sugars, different types of starch, cellulose derivatives (including microcrystalline cellulose), gelatin, vegetable oils, polyethylene glycols, diluent, a granulating agent, lubricant, binder, disintegrant, and so on.

A "prodrug" means a compound that can be converted under physiological conditions or under the action of solvent into the biologically active compound of the present invention. A prodrug of the present invention is prepared by modification of a functional group of the compound of the present invention. Such a modification can be removed *in vivo* or by conventional operations, so as to produce the parent compound.

A "stereoisomer" refers to an isomer due to a different spatial arrangement of atoms in a molecule, including cis-trans isomers, enantiomers, and conformational isomers.

An "effective amount" means an amount that causes a physiological or medical response in a tissue, system or subject and is a desirable amount, including the amount of a compound that is, after administered to a subject to be treated, sufficient to prevent occurrence of one or more symptoms of the disease or disorder to be treated or to reduce the symptom(s) to a certain degree.

A "solvate" refers to the compound of the present invention or a salt thereof that further contains a stoichiometric or non-stoichiometric amount of solvent bound via a non-covalent intermolecular force. When the solvent is water, the solvate is hydrate.

"IC₅₀" means half maximal inhibitory concentration, the concentration that achieves half of the maximum inhibitory effect.

### DETAILED DESCRIPTION OF INVENTION

The technical solutions of the present invention are described in detail hereinafter in connection with the figures and Examples, but the scope of protection of the present invention is not limited thereto.

The structures of compounds were determined by nuclear magnetic resonance (NMR) and/or mass spectroscopy (MS). NMR shifts (δ) are presented in 10⁻⁶ ppm. NMR measurements were performed with a Bruker ADVANCE III 400 NMR device and a Brucker ADVANCE 300 NMR device, wherein the measurement solvents were hexadeuterodimethyl sulfoxide (DMSO-*d₆*), deuterochloroform (CDCl₃), and deuteromethanol (CD₃OD), and the internal reference was tetramethylsilane (TMS).

MS measurements were performed with Agilent 6120B (ESI) and Agilent 6120B (APCI).

HPLC measurements were performed with Agilent 1260DAD High-pressure Liquid Chromatograph (Zorba x SB-C18 100 x 4.6 mm).

Thin-layer chromatography silica gel plate: HSGF254 silica gel plate (Huanghai, Yantai) or GF254 silica gel plate (Qingdao). The specification of the silica gel plate used for thin-layer chromatography (TLC) was 0.15 mm to 0,20 mm, and that for product isolation and purification by TLC was 0.4 mm to 0.5 mm.

The chromatography column generally used the silica gel (Huanghai, Yantai) of 200 to 300 mesh as a carrier.

Known starting materials in connection with the present invention can be synthesized following or using methods known in the art, or can be purchased from companies such as Titansci, Energy Chemical, Demochem (Shanghai), Kelong Chemical (Chengdu), Accela ChemBio, and J&K Scientific.

A N₂ atmosphere means that the reaction vessel is connected to a N₂ balloon of about 1 L in volume.

A H₂ atmosphere means that the reaction vessel is connected to a H₂ balloon of about 1 L in volume.

Hydrogenation reactions generally involve a vacuuming and H₂-charging operation repeated 3 times.

In the Example, unless particularly specified, reactions were carried out under a N₂ atmosphere.

In the Example, unless particularly specified, solutions refer to aqueous solutions.

In the Example, unless particularly specified, reaction temperatures are room temperature, and most suitable room temperature as a reaction temperature is 20°C to 30°C.

In the Example, unless particularly specified, M refers to mole/litre.

TBS means t-butyldimethylsilyl.

Boc means t-butoxycarbonyl.

TFA means trifluoroacetic acid.

### Intermediate 1: 7-azaspiro[3.5]nonan-2-yl 2-hydroxy-2,2-bis(2-thienyl)acetate (Intermediate 1)

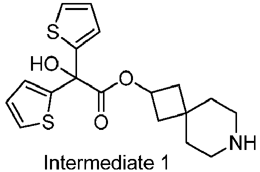

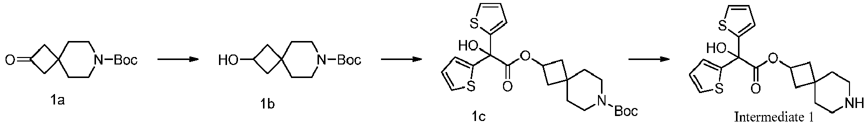

### Step 1: tert-butyl 2-hydroxy-7-azaspiro[3.5]nonane-7-carboxylate (1b)

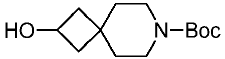

Ethyl tert-butyl-2-carbonyl-7-azaspiro[3.5]nonane-7-carboxylate (7 g, 29.3 mmol) in methanol (100 ml) was cooled to 0°C, and sodium borohydride (1.1 g, 29.3 mmol) was added thereto, followed by reaction at room temperature for 2 hours. Water (100 ml) was added, followed by extraction with methylene chloride (100 mL×3). The organic phases were combined, dried over anhydrous sodium sulfate, and concentrated under reduced pressure, to produce the title compound tert-butyl 2-hydroxy-7-azaspiro[3.5]nonane-7-carboxylate (**1b**) as a white solid (7 g, yield: 99.2%).
¹H NMR (400 MHz, CDCl₃) δ 4.39 - 4.23 (m, 1H), 3.38 - 3.22 (m, 4H), 2.32 - 2.22 (m, 2H), 2.04 (d, 1H), 1.74 - 1.62 (m, 2H), 1.50 (m, 4H), 1.45 - 1.41 (m, 9H). LCMS m/z =264.1[M+23]⁺.

### Step 2: tert-butyl 2-[2-hydroxy-2,2-bis(2-thienyl)acetyl]oxy-7-azaspiro[3.5]nonane-7-carboxylate (1c)

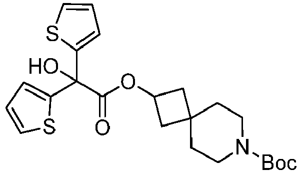

Tert-butyl 2-hydroxy-7-azaspiro[3.5]nonane-7-carboxylate (1c) (0.482 g, 2 mmol) was dissolved in toluene (10 ml), to which sodium hydride (0.024 g, 1 mmol) was added, followed by stirring for 5 min. 2-hydroxy-2,2-bis(2-thienyl)acetate (0.508 g, 2 mmol) was added thereto, and the temperature was elevated to 155°C to allow a reaction to proceed for 1 h. The reaction was quenched by addition of water, extracted with ethyl acetate (20 mL×2), washed with a saturated sodium chloride solution (20 mL×1), dried over anhydrous sodium sulfate, and filtered, and the filtrate was concentrated under reduced pressure. The residue was purified by silica gel column chromatography (eluent: ethyl acetate/petroleum ether (v/v) = 1/100 to 1/20), to obtain the title compound tert-butyl 2-[2-hydroxy-2,2-bis(2-thienyl)acetyl]oxy-7-azaspiro[3.5]nonane-7-carboxylate (1c) as a light yellow solid (0.23 g, yield: 25%).
¹H NMR (400 MHz, CDCl₃) δ 7.29 (dd, 2H), 7.17 (dd, 2H), 6.98 (dd, 2H), 5.25 - 5.05 (m, 1H), 4.66 (s, 1H), 3.32 (dd, 2H), 3.29 - 3.23 (m, 2H), 2.41 - 2.30 (m, 2H), 1.88 (dd, 2H), 1.54 - 1.50 (m, 2H), 1.47 (d, 2H), 1.44 (s, 9H).
LCMS m/z =486.3[M+23]⁺.

### Step 3: 7-azaspiro[3.5]nonan-2-yl 2-hydroxy-2,2-bis(2-thienyl)acetate (Intermediate 1)

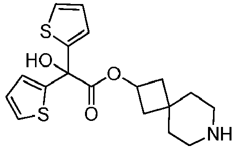

Tert-butyl 2-[2-hydroxy-2,2-bis(2-thienyl)acetyl]oxy-7-azaspiro[3.5]nonane-7-carboxylate (1c) (0.5 g, 1.08 mmol) was dissolved in 1,4-dioxane (10 ml), into which an excess amount of HCl gas was blown, followed by reaction at room temperature for 2 hours. After addition of water (20 ml), the pH was adjusted to 8-9 with solid potassium carbonate at 0°C. The resultant was extracted with ethyl acetate (20 mL×2), washed with a saturated sodium chloride solution (20 mL×1), dried over anhydrous sodium sulfate, and filtered, and the filtrate was concentrated under reduced pressure, to obtain the title compound 7-azaspiro[3.5]nonan-2-yl 2-hydroxy-2,2-bis(2-thienyl)acetate (Intermediate 1) as a white solid (0.24 g, yield: 61%).
¹H NMR (400 MHz, CDCl₃) δ 7.30 (dd, 2H), 7.16 (dd, 2H), 6.98 (dd, 2H), 5.15 (t, 1H), 4.61 (s, 1H), 3.08 (s, 2H), 3.01 (s, 2H), 2.45 - 2.33 (m, 2H), 1.99 - 1.87 (m, 4H), 1.87 - 1.79 (m, 2H).
LCMS m/z =364.0[M+1]⁺.

### Intermediate2: 7-azaspiro[3.5]nonan-2-yl 2-cyclopentyl-2-hydroxy-2-phenyl-acetate (Intermediate 2)

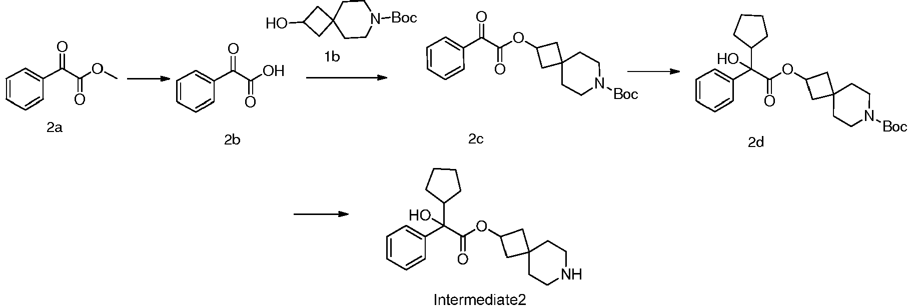

### Step 1: 2-oxo-2-phenyl-acetic acid (2b)

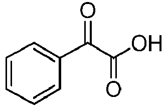

Methyl 2-oxo-2-phenyl-acetate (**2a**) (10 g, 60.92 mmol) was dissolved in water (50 ml), to which sodium hydroxide (4.9 g, 121.8 mmol) was added, followed by reaction at room temperature for 2 hours. The reaction solution was extracted with methylene chloride (50 mL×2), and the aqueous phase was adjusted to pH=3 with an aqueous solution of 4M HCl and extracted with methylene chloride (100 mL×3). The organic phases were combined and concentrated under reduced pressure, to obtain the title compound 2-oxo-2-phenyl-acetic acid (2b) as a white solid (9 g, yield: 98.41%).
¹H NMR (400 MHz, DMSO-d6) δ 7.95-7.88 (m, 2H), 7.78 - 7.71 (m, 1H), 7.64 - 7.55 (m, 2H).
LCMS m/z =149.1 [M-1].

### Step 2: tert-butyl 2-(2-oxo-2-phenyl-acetyl)oxy-7-azaspiro[3.5]nonane-7-carboxylate (2c)

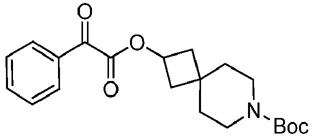

2-oxo-2-phenyl-acetic acid (2b) (4.0 g, 27 mmol) was dissolved in methylene chloride (100 ml), to which oxalyl chloride (6.8 g, 53 mmol) was added and one drop of N,N-dimethylformamide was added, followed by reaction at room temperature for 1 h. The reaction solution was concentrated under reduced pressure into **Reaction Solution 1.** Tert-butyl 2-hydroxy-7-azaspiro[3.5]nonane-7-carboxylate (1b) (4.8 g, 20 mmol) was dissolved in methylene chloride (100 ml), triethylamine (11 g, 110 mmol) was added thereto, and **Reaction Solution 1** was added while on an ice bath, followed by reaction at room temperature for 1 h. The reaction solution was extracted after addition of methylene chloride (50 ml) and water (50 ml). The aqueous phase was extracted with methylene chloride (50 mL×1). The organic phases were combined, dried over anhydrous sodium sulfate, filtered, and the filtrate was concentrated under reduced pressure. The residue was purified by silica gel column chromatography (ethyl acetate/petroleum ether (v/v) = 0:1 to 1:9) to give the yellow oily title compound tert-butyl 2-(2-oxo-2-phenyl-acetyl)oxy-7-azaspiro[3.5]nonane-7-carboxylate (2c) (6.0 g, yield: 60%).
¹H NMR (400 MHz, CDCl₃) δ 8.00 (d, 2H), 7.70-7.62 (m, 1H), 7.52 (t, 2H), 5.35 - 5.28 (m, 1H), 3.42 - 3.34 (m, 2H), 3.34 - 3.26 (m, 2H), 2.54 - 2.41 (m, 2H), 2.07 - 1.98 (m, 2H), 1.64 - 1.53 (m, 4H), 1.50 - 1.37 (m, 9H).
LCMS m/z =396.2[M+23].

### Step 3: tert-butyl 2-(2-cyclopentyl-2-hydroxy-2-phenyl-acetyl)oxy-7-azaspiro[3.5]nonane-7-carboxylate (2d)

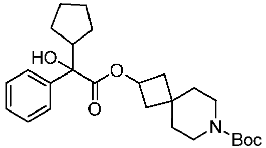

Tert-butyl 2-(2-oxo-2-phenyl-acetyl)oxy-7-azaspiro[3.5]nonane-7-carboxylate (2c) (1.0 g, 11 mmol) was dissolved in tetrahydrofuran (100 ml), a solution of 2.0 M cyclopentylmagnesium bromide in tetrahydrofuran (8 mL, 16 mmol) was added thereto at -40°C, and the temperature was gradually elevated to room temperature to allow a reaction to proceed for 2 hours. The reaction solution was extracted after addition of a saturated ammonium chloride solution (60 ml) and ethyl acetate (50 ml). The aqueous phase was extracted with ethyl acetate (50 mL×1). The organic phases were combined, dried over anhydrous sodium sulfate, filtered, and the filtrate was concentrated under reduced pressure. The residue was purified by silica gel column chromatography (ethyl acetate/petroleum ether (v/v) = 0:1 to 1:19) to give the colorless oily title compound tert-butyl 2-(2-cyclopentyl-2-hydroxy-2-phenyl-acetyl)oxy-7-azaspiro[3.5]nonane-7-carboxylate (2d) (2.4g, yield: 51%).
¹H NMR (400 MHz, CDCl₃) δ 7.68 - 7.42 (m, 2H), 7.38 - 7.29 (m, 2H), 7.29 - 7.23 (m, 1H), 5.08 - 4.95 (m, 1H), 3.39 - 3.17 (m, 4H), 2.97-2.82 (m, 1H), 2.44-2.20(m, 2H), 1.90-1.80 (m, 1H), 1.79 - 1.54 (m, 7H), 1.53-1.47 (m, 4H), 1.45 - 1.43 (m, 9H), 1.38-1.30 (m, 2H).
LCMS m/z =466.3[M+23].

### Step 4: 7-azaspiro[3.5]nonan-2-yl 2-cyclopentyl-2-hydroxy-2-phenyl-acetate (Intermediate 2)

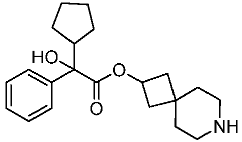

Tert-butyl 2-(2-cyclopentyl-2-hydroxy-2-phenyl-acetyl)oxy-7-azaspiro[3.5]nonane-7-carboxylate (2d) (2.4 g, 5.4 mmol) was dissolved in methylene chloride (20 ml), to which trifluoroacetic acid (6.2 g, 54 mmol) was added, followed by reaction at room temperature for 3 hours. The reaction solution was adjusted to pH=10 with aqueous ammonia, and extracted after addition of methylene chloride (50 ml) and water (50 ml). The aqueous phase was extracted with methylene chloride (20 mL×1). The organic phases were combined, dried over anhydrous sodium sulfate, filtered, and the filtrate was concentrated under reduced pressure, to obtain the yellow oily title compound 7-azaspiro[3.5]nonan-2-yl 2-cyclopentyl-2-hydroxy-2-phenyl-acetate (Intermediate 2) (1.7 g, yield: 91%).
¹H NMR (400 MHz, CDCl₃) δ 7.66 - 7.40 (m, 2H), 7.38-7.29 (m, 2H), 7.29 - 7.24 (m, 1H), 5.07 - 4.95 (m, 1H), 3.03 - 2.84 (m, 4H), 2.51 - 2.18 (m, 2H), 2.00 - 1.27 (m, 15H).
LCMS m/z =344.2[M+1].

### Isomer 1 of Intermediate 2 and Isomer 2 of Intermediate 2:

Tert-butyl 2-(2-cyclopentyl-2-hydroxy-2-phenyl-acetyl)oxy-7-azaspiro[3.5]nonane-7-carboxylate (2d) (13 g, 29.31 mmol) was dissolved in methylene chloride (50 ml), to which trifluoroacetic acid (33.41 g, 293.1 mmol) was added, followed by reaction at room temperature for 3 hours. The reaction solution was adjusted to pH=10 with aqueous ammonia, and extracted after addition of methylene chloride (50 ml) and water (50 ml). The aqueous phase was extracted with methylene chloride (50 mL×1). The organic phases were combined. The organic phases were dried over anhydrous sodium sulfate, filtered, and the filtrate was concentrated. The residue was purified by silica gel column chromatography, wherein the chromatography conditions were: Shimadzu LC-20AP Prep HPLC (PrepL-GB); the chromatographic column: ChiralPak AY, 300×50mm, 10u; Mobile phase: A heptane (0.1% NH3H2O) and B ethanol; Gradient: B 25%; Flow rate: 80 ml/min; Column temperature: R.T.; to obtain the title compounds: yellow oily **Isomer 1** of 7-azaspiro[3.5]nonan-2-yl 2-cyclopentyl-2-hydroxy-2-phenyl-acetate (Intermediate 2) (2.7 g, yield: 27%) and yellow oily **Isomer 2** of 7-azaspiro[3.5]nonan-2-yl 2-cyclopentyl-2-hydroxy-2-phenyl-acetate (Intermediate 2) (2.5 g, yield: 25%) . The time for Isomer 1 of Intermediate 2 to reach peak is shorter than that of Isomer 2 of Intermediate 2.

Isomer 1 of Intermediate 2: ¹H NMR (400 MHz, CDCl₃) δ 7.65-7.40 (m, 2H), 7.37 - 7.29 (m, 2H), 7.29 - 7.23 (m, 1H), 5.05-4.95 (m, 1H), 2.96-2.88 (m, 1H), 2.88 - 2.72 (m, 4H), 2.46 - 2.20 (m, 2H), 1.91 - 1.79 (m, 1H), 1.79 - 1.40 (m, 12H), 1.37-1.30 (m, 1H).
LCMS m/z =344.3[M+1].

Isomer 2 of Intermediate 2: ¹H NMR (400 MHz, CDCl₃) δ 9.21 (s, 1H), 7.62 (dd, 2H), 7.36 - 7.30 (m, 2H), 7.29 - 7.24 (m, 1H), 5.08 - 4.96 (m, 1H), 3.12-2.94 (m, 4H), 2.94-2.85 (m 1H), 2.48 - 2.37 (m, 1H), 2.37 - 2.27 (m, 1H), 1.93 (dd, 1H), 1.86-1.74 (m, 5H), 1.73 - 1.40 (m, 6H), 1.40-1.30 (m, 2H).
LCMS m/z =344.3[M+1].

### Intermediate 3: 7-azaspiro[3.5]nonan-2-yl 2-cyclopentyl-2-hydroxy-2-(2-thienyl)acetate (Intermediate 3)

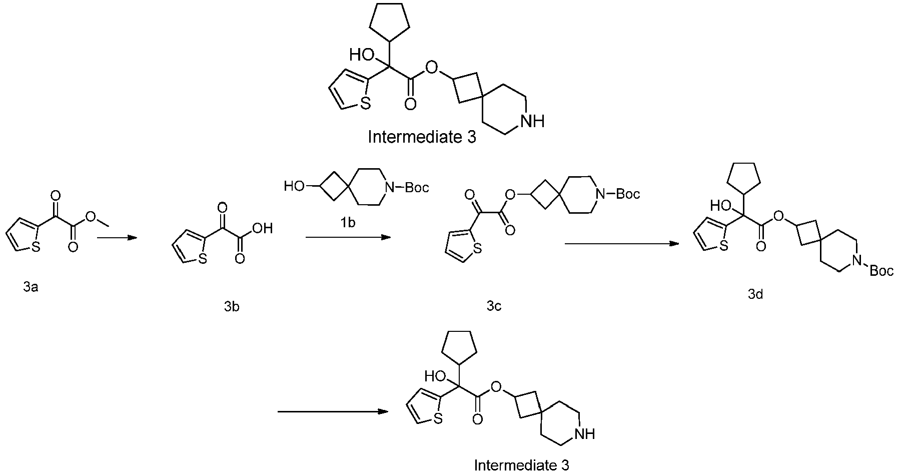

### Step 2: 2-oxo-2-(2-thienyl)acetic acid (3b)

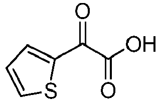

Methyl 2-oxo-2-(2-thienyl)acetate (**3a**) (20 g, 108.6 mmol) was dissolved in water (100 ml), to which sodium hydroxide (8.69 g, 217.1 mmol) was added, followed by reaction at room temperature for 2 hours. The reaction solution was extracted with methylene chloride (100 mL×2), and the aqueous phase was adjusted to pH=3 with an aqueous solution of 4M HCl and extracted with methylene chloride (100 mL×2). The organic phases were concentrated to obtain the title compound 2-oxo-2-(2-thienyl)acetic acid (3b) as a white solid (16.5 g, yield: 7.3%).
¹H NMR (400 MHz, CDCl₃) δ 8.53 (dd, 1H), 7.96 (dd, 1H), 7.26 (dd, 1H).
LCMS m/z =155.1 [M-1].

### Step 2: tert-butyl 2-[2-oxo-2-(2-thienyl)acetyl]oxy-7-azaspiro[3.5]nonane-7-carboxylate (3c)

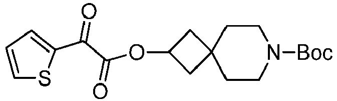

2-oxo-2-(2-thienyl)acetic acid (3b) (16.5 g, 106 mmol) was dissolved in methylene chloride (200 ml), to which oxalyl chloride (26.8 g, 211 mmol) was added and one drop of N,N-dimethylformamide was added, followed by reaction at room temperature for 1 h. The reaction solution was concentrated under reduced pressure into **Reaction Solution 1.** Tert-butyl 2-hydroxy-7-azaspiro[3.5]nonane-7-carboxylate (1b) (21.7 g, 89.8 mmol) was dissolved in methylene chloride (200 ml), triethylamine (42.8 g, 423 mmol) was added thereto, and Reaction Solution 1 was added while on an ice bath, followed by reaction at room temperature for 1 h. The reaction solution was extracted after addition of methylene chloride (50 ml) and water (200 ml). The aqueous phase was extracted with methylene chloride (100 mL×1). The organic phases were combined. The organic phase was dried over anhydrous sodium sulfate, filtered, and the filtrate was concentrated under reduced pressure. The residue was purified by silica gel column chromatography (ethyl acetate/petroleum ether (v/v) = 0:1 to 1:9) to give the yellow oily title compound tert-butyl 2-[2-oxo-2-(2-thienyl)acetyl]oxy-7-azaspiro[3.5]nonane-7-carboxylate (3c) (26.0 g, yield: 64.8%).
¹H NMR (400 MHz, CDCl₃) δ 8.18-8.06 (m, 1H), 7.89 - 7.76 (m, 1H), 7.22 - 7.18 (m, 1H), 5.29-5.21 (m, 1H), 3.42 - 3.26 (m, 4H), 2.52 - 2.37 (m, 2H), 2.11 - 1.96 (m, 2H), 1.63 - 1.53 (m, 4H), 1.45 (s, 9H).
LCMS m/z =402.1[M+23].

### Step 3: tert-butyl 2-[2-cyclopentyl-2-hydroxy-2-(2-thienyl)acetyl]oxy-7-azaspiro[3.5]nonane-7-carboxy late (3d)

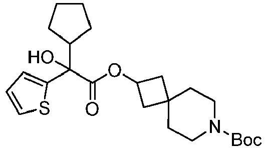

Tert-butyl 2-[2-oxo-2-(2-thienyl)acetyl]oxy-7-azaspiro[3.5]nonane-7-carboxylate (3c) (26 g, 68.51 mmol) was dissolved in tetrahydrofuran (200 ml), a solution of 2.0 M cyclopentylmagnesium bromide in tetrahydrofuran (56 mL, 102 mmol) was added thereto at -40°C, and the temperature was gradually elevated to room temperature to allow a reaction to proceed for 2 hours. The reaction solution was extracted after addition of a saturated ammonium chloride solution (200 ml) and ethyl acetate (200 ml). The aqueous phase was extracted with ethyl acetate (100 mL×1). The organic phases were combined. The organic phase was dried over anhydrous sodium sulfate, filtered, and the filtrate was concentrated under reduced pressure. The residue was purified by silica gel column chromatography (ethyl acetate/petroleum ether (v/v) = 0:1 to 1:19) to give the yellow oily title compound tert-butyl 2-[2-cyclopentyl-2-hydroxy-2-(2-thienyl)acetyl]oxy-7-azaspiro[3.5]nonane-7-carboxy late (3d) (15 g, yield: 48.70%).
¹H NMR (400 MHz, CDCl₃) δ 7.23 - 7.19 (m, 1H), 7.12 - 7.06 (m, 1H), 6.99 - 6.93 (m, 1H), 5.13 - 5.02 (m, 1H), 3.39 - 3.16 (m, 4H), 2.86 - 2.71 (m, 1H), 2.46 - 2.21 (m, 2H), 1.93-1.86 (m, 1H), 1.85-1.78 (m, 1H), 1.70 - 1.47 (m, 12H), 1.44 (s,9H). LCMS m/z =472.3[M+23].

### Step 4: 7-azaspiro[3.5]nonan-2-yl 2-cyclopentyl-2-hydroxy-2-(2-thienyl)acetate (Intermediate 3)

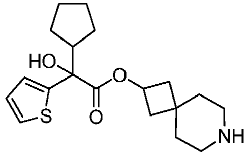

Tert-butyl 2-[2-cyclopentyl-2-hydroxy-2-(2-thienyl)acetyl]oxy-7-azaspiro[3.5]nonane-7-carboxy late (3d) (15 g, 33.36 mmol) was dissolved in dioxane (100 ml), into which HCl gas was blown, followed by reaction at room temperature for 4 hours. The reaction solution was concentrated at reduced pressure, adjusted to pH=10 with aqueous ammonia, and extracted after addition of methylene chloride (100 ml) and water (30 ml). The aqueous phase was extracted with methylene chloride (50 mL×1). The organic phases were combined. The organic phase was dried over anhydrous sodium sulfate, filtered, and the filtrate was concentrated under reduced pressure, to obtain 7-azaspiro[3.5]nonan-2-yl 2-cyclopentyl-2-hydroxy-2-(2-thienyl)acetate (Intermediate 3).

### Preparation of Isomer 1 and Isomer 2 of Intermediate 3:

7-azaspiro[3.5]nonan-2-yl 2-cyclopentyl-2-hydroxy-2-(2-thienyl)acetate (Intermediate 3) was separated by preparative chromatography under the following conditions: Chromatographic column: ChiralPak IC, 250×30mm I.D., 5µm; Mobile phase: A CO₂ and B methanol (0.1% NH3H2O); Gradient: B 40%; Flow rate: 80 ml/min; Backpressure: 100 bar; Column temperature: 38°C; Wavelength: 220 nm; Period: ∼3 min. Obtained were the title compounds yellow oily Isomer 1 of Intermediate 3 (1.93 g, yield: 16.6%) and yellow oily Isomer 2 of Intermediate 3 (2.57 g, yield: 22%).

### Isomer 1 of Intermediate 3:

¹H NMR (400 MHz, DMSO-d6) δ 7.38 (dd, 1H), 7.08 (dd, 1H), 6.96 (dd, 1H), 5.93 (s, 1H), 4.96 - 4.92 (m, 1H), 2.75 - 2.71(m, 1H), 2.58 - 2.53 (m, 4H), 2.29 - 2.18 (m, 2H), 1.75-1.65 (m, 1H), 1.56 - 1.38 (m, 12H).
LCMS m/z =350.3[M+1].

### Isomer 2 of Intermediate 3:

Isomer 2 of Intermediate 3: YH1Z00001960-P2
¹H NMR (400 MHz, DMSO-d6) δ 7.38 (dd, 1H), 7.08 (dd, 1H), 6.96 (dd, 1H), 5.93 (s, 1H), 4.96 - 4.92 (m, 1H), 2.75 - 2.71(m, 1H), 2.58 - 2.53 (m, 4H), 2.29 - 2.18 (m, 2H), 1.75-1.65 (m, 1H), 1.56 - 1.38 (m, 12H).
LCMS m/z =350.2[M+1].

### Step 4: 3-[2-[2-hydroxy-2,2-bis(2-thienyl)acetyl]oxy-7-azaspiro[3.5]nonan-7-yl]propanoic acid (Intermediate 4)

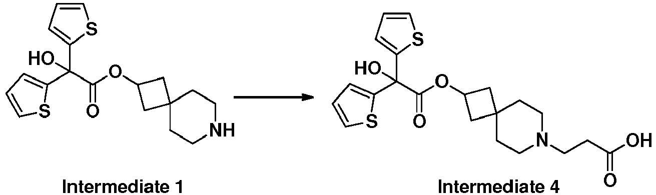

7-azaspiro[3.5]nonan-2-yl 2-hydroxy-2,2-bis(2-thienyl)acetate (Intermediate 1) (0.8 g, 2 mmol) and acrylic acid (2.0 g, 20 mmol) were dissolved in 2-methyltetrahydrofuran (10 ml), placed in a microwave reactor to raise the temperature to 100°C at which a reaction was allowed to proceed for 1 h, then cooled to room temperature, and concentrated at reduced pressure. The residue was purified by silica gel column chromatography (methanol/methylene chloride (v/v) = 0:1 to 1:9) to give the title compound
3-[2-[2-hydroxy-2,2-bis(2-thienyl)acetyl]oxy-7-azaspiro[3.5]nonan-7-yl]propanoic acid (Intermediate 4) as a light yellow solid (0.8 g, yield: 80%).
¹H NMR (400 MHz, DMSO-d6) δ7.50 (dd, 2H), 7.10 (dd, 1H), 7.00 (dd, 2H), 5.07-5.03 (m, 1H), 2.77-2.73 (m, 2H), 2.51-2.44 (m, 6H), 2.32-2.27(m, 2H), 1.80-1.75 (m, 2H), 1.64-1.55 (m, 4H).
LCMS m/z =436.0[M+1].

### Intermediate 5: 7-[(1R)-2-amino-1-[tert-butyl(dimethyl)silyl]oxy-ethyl]-4-[tert-butyl(dimethyl)silyl]o xy-3H-1,3-benzothiazol-2-one (Intermediate 5)

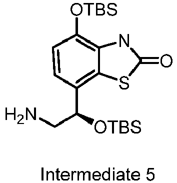

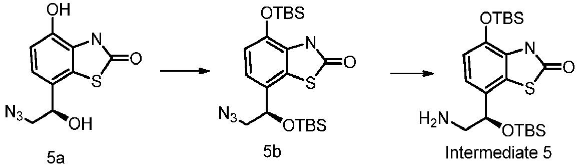

### Step 1: 7-[(1R)-2-azido-1-[tert-butyl(dimethyl)silyl]oxy-ethyl]-4-[tert-butyl(dimethyl)silyl]ox y-3H-1,3-benzothiazol-2-one (5b)

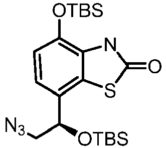

7-[(1R)-2-azido-1-hydroxy-ethyl]-4-hydroxy-3H-1,3-benzothiazol-2-one (5a) (prepared with reference to WO2009098448A1) (0.56 g, 2.2 mmol) was dissolved in N,N-dimethylformamide (20 ml), imidazole (0.6 g, 8.9 mmol) was added thereto, tert-butyldimethylsilyl chloride (1.3 g, 8.9 mmol) was added in batches, then a catalytic amount of 4-dimethylaminopyridine was added, and the temperature was raised to 40°C, followed by stirring for 7 hours. The reaction solution was poured into water (100 ml) and extracted with ethyl acetate (100 mL×1). The organic phase was washed with a saturated sodium chloride solution (100 mL×2), dried over anhydrous sodium sulfate, and filtered, and the filtrate was concentrated under reduced pressure. The residue was purified by silica gel column chromatography (eluent: ethyl acetate/petroleum ether (v/v) = 0:1 to 5:59) to give the title compound 7-[(1R)-2-azido-1-[tert-butyl(dimethyl)silyl]oxy-ethyl]-4-[tert-butyl(dimethyl)silyl]ox y-3H-1,3-benzothiazol-2-one (5b) as a white solid (0.85 g, yield 80%).
¹H NMR (400 MHz, CDCl₃) δ 8.25 (s, 1H), 6.92 (d, 1H), 6.71 (d, 1H), 4.78 (dd, 1H), 3.41 (dd, 1H), 3.25 (dd, 1H), 1.05-0.98 (m, 9H), 0.92-0.88 (m, 9H), 0.28 (m, 6H), 0.12 (d, 3H), -0.04 (d, 3H).

### Step 2: 7-[(1R)-2-amino-1-[tert-butyl(dimethyl)silyl]oxy-ethyl]-4-[tert-butyl(dimethyl)silyl]o xy-3H-1,3-benzothiazol-2-one (Intermediate 5)

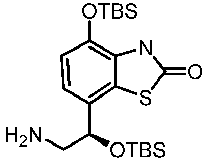

7-[(1R)-2-azido-1-[tert-butyl(dimethyl)silyl]oxy-ethyl]-4-[tert-butyl(dimethyl)silyl]ox y-3H-1,3-benzothiazol-2-one (5b) (0.85 g, 1.8 mmol) was dissolved in ethyl acetate (20 ml), and 10% (w/w) Pd/C (0.085 g) was added, followed by stirring overnight at normal pressure from a H₂ balloon. The resultant was filtered through diatomite , and concentrated to obtain the title compound 7-[(1R)-2-amino-1-[tert-butyl(dimethyl)silyl]oxy-ethyl]-4-[tert-butyl(dimethyl)silyl]o xy-3H-1,3-benzothiazol-2-one (Intermediate 5) as a light black solid (0.7 g, yield 90%).
¹H NMR (400 MHz, CDCl₃) δ 6.89 (d, 1H), 6.68 (d, 1H), 4.64 (dd, 1H), 2.94-2.82 (m, 2H), 1.04-0.96 (s, 9H), 0.95-0.87 (s, 9H), 0.33-0.23 (m, 6H), 0.12-0.06 (m, 3H), -0.04--0.11 (m, 3H).

### Example 1:

### [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-hydroxy-2,2-bis(2-thienyl)acetate

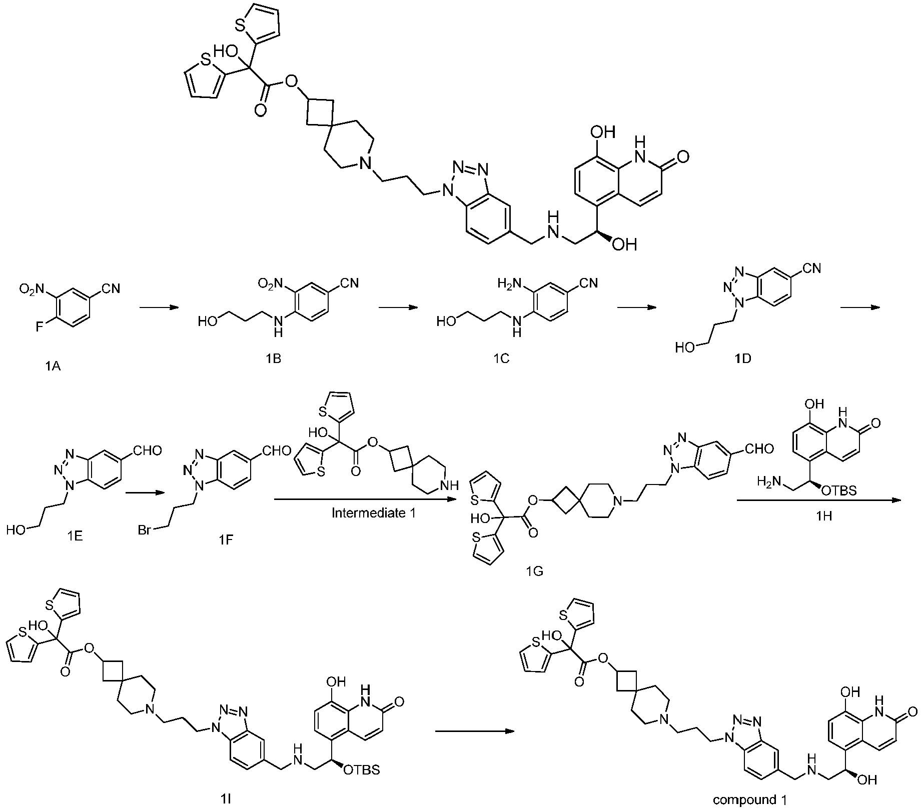

### Step 1: 4-(3-hydroxypropylamino)-3-nitro-benzonitrile (1B)

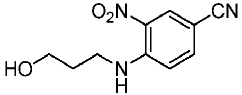

4-fluoro-3-nitro-benzonitrile (**1A**) (5.0 g, 30 mmol) was dissolved in tetrahydrofuran (50 ml), to which 3-amino-1-propanol (2.5 g, 33 mmol) and N,N-diisopropylethylamine (4.3 g, 33 mmol) were added, followed by reaction at room temperature for 4 hours. Ethyl acetate (60 ml) was added to the reaction solution, which was washed with a saturated sodium bicarbonate solution (60 mL×2) and with a saturated sodium chloride solution (60 mL×1), dried over anhydrous sodium sulfate, and filtered. The filtrate was concentrated under reduced pressure, to obtain the title compound 4-(3-hydroxypropylamino)-3-nitro-benzonitrile (1B) as a yellow solid (6 g, yield: 90%).
¹H NMR (400 MHz, CDCl₃) δ 8.67 (s, 1H), 8.50 (d, 1H), 7.59 (d, 1H), 6.97 (d, 1H), 3.86 (t, 2H), 3.56 - 3.51 (m, 2H), 2.02 - 1.97 (m, 2H), 1.72 (s, 1H).
LCMS m/z =244[M+23]⁺.

### Step 2: 3-amino-4-(3-hydroxypropylamino)benzonitrile (1C)

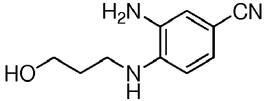

4-(3-hydroxypropylamino)-3-nitro-benzonitrile (1B) (6 g, 27.12 mmol) was dissolved in ethanol (100 ml), and 10% Pd/C (0.9 g) was added thereto, followed by reaction at room temperature under a H₂ atmosphere for 8 hours. After the reaction was complete, the reaction solution was filtered, and the filtrate was concentrated to obtain the title compound 3-amino-4-(3-hydroxypropylamino)benzonitrile (1C) (4.5 g, yield 87%).
¹H NMR (400 MHz, DMSO-d6) δ 6.92 (dd, 1H), 6.77 (d, 1H), 6.46 (d, 1H), 5.38 (br, 1H), 4.97 (br, 2H), 4.51 (br, 1H), 3.52 (m, 2H), 3.19- 3.14 (m, 2H), 1.81 - 1.68 (m, 2H).
LCMS m/z=192.1[M+1]⁺.

### Step 3: 1-(3-hydroxypropyl)benzotriazole-5-carbonitrile (1D)

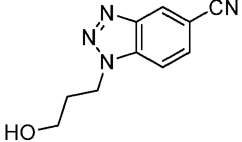

3-amino-4-(3-hydroxypropylamino)benzonitrile (1C) (0.5 g, 2.61 mmol) was dissolved in an aqueous solution of 6 M HCl (10 ml), and an aqueous solution (2 ml) of sodium nitrite (0.271 g, 3.92 mmol) was added dropwise at -5°C, followed by reaction at room temperature for 3 hours. The reaction solution was extracted with methylene chloride (30 mL×2), dried over anhydrous sodium sulfate, filtered, and the filtrate was concentrated under reduced pressure, to obtain the title compound 1-(3-hydroxypropyl)benzotriazole-5-carbonitrile (1D) (0.25 g, yield 66%).
¹H NMR (400 MHz, DMSO-d6) δ 8.81 - 8.75 (m, 1H), 8.11 (dd, 1H), 7.97 - 7.89 (m, 1H), 4.83 (t, 2H), 4.77 - 4.54 (m, 1H), 3.41 (t, 2H), 2.13 - 2.03 (m, 2H).
LCMS m/z=203.1[M+1]⁺.

### Step 4: 1-(3-hydroxypropyl)benzotriazole-5-carbaldehyde (1E)

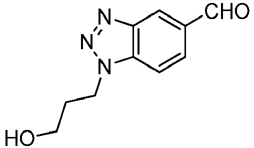

1-(3-hydroxypropyl)benzotriazole-5-carbonitrile (1D) (3.0 g, 14.8 mmol) was dissolved in an aqueous solution of 75% formic acid (33.3 ml), aluminum-nickel alloy (3.3 g) was added thereto, followed by reaction at 90°C for 4 hours. The reaction solution was filtered through diatomite , the filtrate was concentrated, the residue was dissolved by addition of ethanol (30 ml), and the pH was adjusted to about 10 by addition of a 2 M sodium hydroxide solution, followed by stirring at room temperature for 1 h. The pH was adjusted to about 7 with a 2 M dilute HCl. The resultant was extracted with ethyl acetate (50 mL×3), and washed with a saturated sodium chloride solution (30 mL×1). The organic phase was dried over anhydrous sodium sulfate, and filtered, and the filtrate was concentrated under reduced pressure. The residue was purified by silica gel column chromatography (eluent: ethyl acetate/petroleum ether (v/v) = 1/100 to 1/10), to obtain the title compound 1-(3-hydroxypropyl)benzotriazole-5-carbaldehyde (1E) as an off-white solid (1.8 g, yield 59%).
¹H NMR (400 MHz, CDCl₃) δ 10.11 (s, 1H), 8.52 (s, 1H), 8.04 (dd, 1H), 7.72 (d, 1H), 4.85 (t, 2H), 3.67 (t, 2H), 2.34 - 2.23 (m, 2H).
LCMS m/z=206.1[M+1]⁺.

### Step 5: 1-(3-bromopropyl)benzotriazole-5-carbaldehyde (1F)

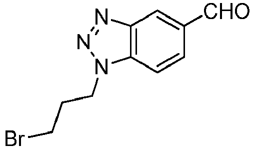

Under N₂ protection, 1-(3-hydroxypropyl)benzotriazole-5-carbaldehyde (1E) (0.2 g, 1 mmol) was dissolved in methylene chloride (10.5 ml), carbon tetrabromide (0.4 g, 1.2 mmol) was added thereto while on an ice bath, triphenylphosphine (0.3 g, 1.2 mmol) was added slowly, followed by stirring at room temperature for 2 hours. Then carbon tetrabromide (0.4 g, 1.2 mmol) was further added, and then triphenylphosphine (0.3 g, 1.2 mmol) was added, followed by reaction at room temperature for 1 h. After the reaction was complete, concentration under reduced pressure was directly performed and the residue was purified by silica gel column chromatography (eluent: ethyl acetate/petroleum ether (v/v) = 1/100 to 1/10), to obtain the title compound 1-(3-bromopropyl)benzotriazole-5-carbaldehyde (1F) as a white solid (0.1 g, yield 40%).
¹H NMR (400 MHz, CDCl₃) δ 10.15 (s, 1H), 8.58 (s, 1H), 8.11 (dd, 1H), 7.74 (d, 1H), 4.87 (t, 2H), 3.41 (t, 2H), 2.68 - 2.55 (m, 2H).
LCMS m/z=268[M+1]⁺.

### Step 6: [7-[3-(5-formylbenzotriazol-1-yl)propyl]-7-azaspiro[3.5]nonan-2-yl] 2-hydroxy-2,2-bis(2-thienyl)acetate (1G)

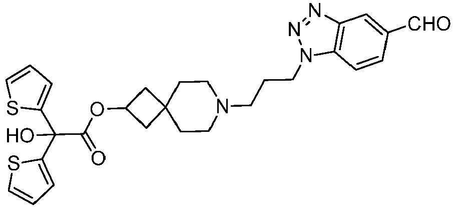

7-azaspiro[3.5]nonan-2-yl 2-hydroxy-2,2-bis(2-thienyl)acetate (Intermediate 1) (1.1 g, 3.026 mmol) was dissolved in acetonitrile (20 ml), and under N₂ protection, 1-(3-bromopropyl)benzotriazole-5-carbaldehyde (1F) (0.811 g, 3.026 mmol) and N,N-diisopropylethylamine (0.54 g, 4.237 mmol) were added, followed by reaction at 85°C for 4 hours. The reaction solution was directly concentrated under reduced pressure and the residue was purified by silica gel column chromatography (eluent: methylene chloride/methanol (v/v) = 100:1 to 100:3), to obtain the title compound [7-[3-(5-formylbenzotriazol-1-yl)propyl]-7-azaspiro[3.5]nonan-2-yl] 2-hydroxy-2,2-bis(2-thienyl)acetate (1G) as a brown solid (1.2 g, yield: 79%).
¹H NMR (400 MHz, CDCl₃) δ 10.13 (s, 1H), 8.54 (s, 1H), 8.05 (dd, 1H), 7.69 (d, 1H), 7.31 - 7.27 (m, 2H), 7.17 (dd, 2H), 6.97 (dd, 2H), 5.14 - 5.11 (m, 1H), 4.77 - 4.72 (m, 2H), 2.26 - 2.17 (m, 10H), 1.89 - 1.77 (m, 2H), 1.60- 1.48 (m, 4H).
LCMS m/z=551.0[M+1]⁺.

### Step 7: [7-[3-[5-[[[(2R)-2-[tert-butyl(dimethyl)silyl]oxy-2-(8-hydroxy-2-oxo-1H-quinolin-5-y 1)ethyl]amino]methyl]benzotriazol-1-yl]propyl]-7-azaspiro[3.5]nonan-2-yl] 2-hydroxy-2,2-bis(2-thienyl)acetate (1I)

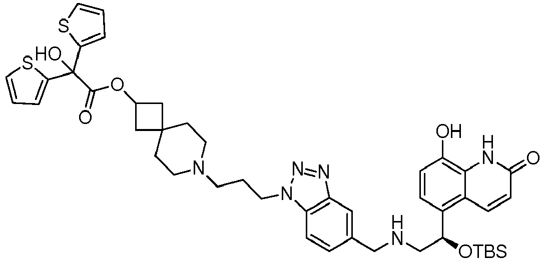

[7-[3-(5-formylbenzotriazol-1-yl)propyl]-7-azaspiro[3.5]nonan-2-yl]2-hydroxy-2,2-bis(2-thienyl)acetate (1G) (0.8 g, 1.45 mmol) and 5-[(1R)-2-amino-1-[t-butyl(dimethyl)silyl]oxymethyl]-8-hydroxy-1H-quinolin-2-one (1H) (0.535 g, 1.6 mmol) were dissolved in a mixed solvent of isopropanol (10 ml) and tetrahydrofuran (5 ml), to which N,N-diisopropylethylamine (0.222 g, 1.71 mmol) and sodium triacetoxyborohydride (0.453 g, 2.14 mmol) were added, followed by reaction at room temperature overnight. Sodium triacetoxyborohydride (1.35 g, 6.39 mmol) was then added, and after a 3-h reaction, sodium triacetoxyborohydride (0.453 g, 2.14 mmol) was further added, followed by reaction for 2 hours. The pH was adjusted to about 9 with a saturated aqueous solution of sodium bicarbonate. The resultant was extracted with methylene chloride (30 mL×2), and washed with a saturated sodium chloride solution (30 mL×1). The organic phase was dried over anhydrous sodium sulfate, and filtered, and the filtrate was concentrated under reduced pressure. The residue was purified by silica gel column chromatography (eluent: methylene chloride/methanol (v/v) = 100/1 to 20/1), to produce the title compound [7-[3-[5-[[[(2R)-2-[tert-butyl(dimethyl)silyl]oxy-2-(8-hydroxy-2-oxo-1H-quinolin-5-y 1)ethyl]amino]methyl]benzotriazol-1-yl]propyl]-7-azaspiro[3.5]nonan-2-yl] 2-hydroxy-2,2-bis(2-thienyl) acetate (**1I**) as a brown solid (0.4 g, yield: 32%).
LCMS m/z =869.2[M+1]⁺.

### Step 8: [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-hydroxy-2,2-bis(2-thienyl)acetate

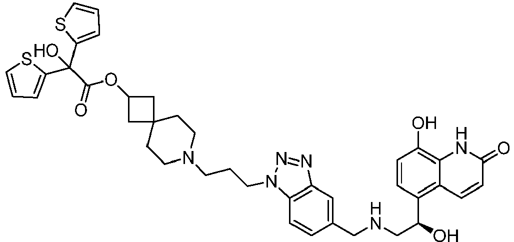

[7-[3-[5-[[[(2R)-2-[tert-butyl(dimethyl)silyl]oxy-2-(8-hydroxy-2-oxo-1H-quinolin-5-y 1)ethyl]amino]methyl]benzotriazol-1-yl]propyl]-7-azaspiro[3.5]nonan-2-yl] 2-hydroxy-2,2-bis(2-thienyl) acetate (1I) (0.4 g, 0.46 mmol) was dissolved in tetrahydrofuran (2 ml), and triethylamine trihydrofluoride (0.3 g, 1.8 mmol) was added thereto, followed by stirring at room temperature for 8 hours. The pH was adjusted to about 9 with a saturated aqueous solution of sodium bicarbonate. The resultant was extracted with methylene chloride (50 mL×2), and washed with a saturated sodium chloride solution. The organic phase was dried over anhydrous sodium sulfate, and filtered, and the filtrate was concentrated under reduced pressure. The residue was purified by silica gel column chromatography (eluent: methylene chloride/methanol (v/v) = 100/1 to 10/1), to produce the title compound [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-hydroxy-2,2-bis(2-thienyl)acetate (Compound 1) as a yellow solid (0.24 g, 69%).
¹H NMR (400 MHz, DMSO-d6) δ 8.12 (d, 1H), 7.92 (s, 1H), 7.77 (d, 1H), 7.51 (dd, 1H), 7.47 (dd, 2H), 7.11 - 7.04 (m, 3H), 6.99 (dd, 2H), 6.91 (d, 1H), 6.42 (d, 1H), 5.12 - 5.06 (m, 1H), 5.06 - 4.98 (m, 1H), 4.68 (t, 2H), 3.91 (s, 2H), 2.76 - 2.65 (m, 2H), 2.28 - 2.08 (m, 8H), 2.07 4 2.02 (m, 2H), 1.71 (dd, 2H), 1.50 - 1.39 (m, 4H).
LCMS m/z =755.2[M+1]⁺.

### Example 2:

### [7-[3-[5-[[[(2R)-2-hydroxy-2-(5-hydroxy-3-oxo-4H-1,4-benzoxazin-8-yl)ethyl]amino ]methyl]benzotriazol-1-yl]propyl]-7-azaspiro[3.5]nonan-2-yl] 2-hydroxy-2,2-bis(2-thienyl)acetate; ditrifluoroacetic acid (Compound 2)

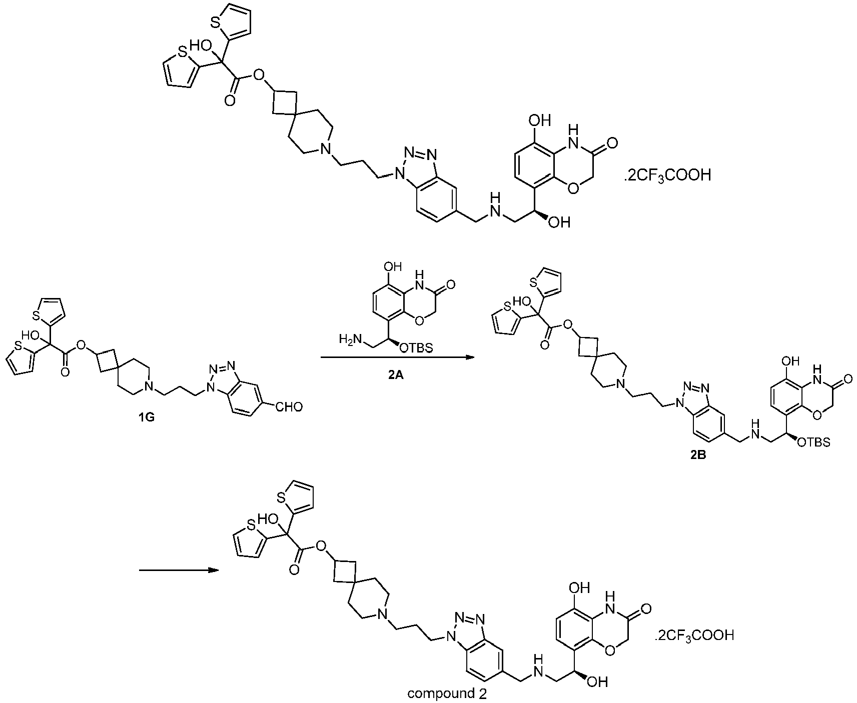

### Step 1: [7-[3-[5-[[[(2R)-2-[tert-butyl(dimethyl)silyl]oxy-2-(5-hydroxy-3-oxo-4H-1,4-benzoxa zin-8-yl)ethyl]amino]methyl]benzotriazol-1-yl]propyl]-7-azaspiro[3.5]nonan-2-yl] 2-hydroxy-2,2-bis(2-thienyl)acetate (2B)

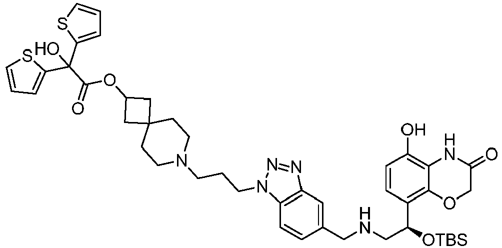

[7-[3-(5-formylbenzotriazol-1-yl)propyl]-7-azaspiro[3.5]nonan-2-yl] 2-hydroxy-2,2-bis(2-thienyl)acetate (**1G**) (1.0 g, 1.82 mmol) and 8-[(1R)-2-amino-1-[t-butyl(dimethyl)silyl]oxyethyl]-5-hydroxy-4H-1,4-benzoxazin-3-one (**2A**) (prepared according to the method for synthesis of Intermediate 65 in WO2008149110A1) (0.738 g, 2.18 mmol) were dissolved in a mixed solvent of isopropanol (10 ml) and tetrahydrofuran (5 ml), to which N,N-diisopropylethylamine (0.296 g, 2.29 mmol) and sodium triacetoxyborohydride (0.566 g, 2.67 mmol) were added, followed by reaction at room temperature overnight. Sodium triacetoxyborohydride (1.69 g, 7.99 mmol) was then added, and after a 3-h reaction, sodium triacetoxyborohydride (0.566 g, 2.67 mmol) was further added, followed by reaction for 2 hours. The pH was adjusted to about 9 with a saturated aqueous solution of sodium bicarbonate. The resultant was extracted with methylene chloride (30 mL×2), washed with a saturated sodium chloride solution (30 mL×1), dried over anhydrous sodium sulfate, and filtered, and the filtrate was concentrated under reduced pressure. The residue was purified by silica gel column chromatography (eluent: methylene chloride/methanol (v/v) = 100/1 to 20/1), to produce the title compound [7-[3-[5-[[[(2R)-2-[tert-butyl(dimethyl)silyl]oxy-2-(5-hydroxy-3-oxo-4H-1,4-benzoxa zin-8-yl)ethyl]amino]methyl]benzotriazol-1-yl]propyl]-7-azaspiro[3.5]nonan-2-yl] 2-hydroxy-2,2-bis(2-thienyl)acetate (2B) as a gray solid (0.43 g, yield: 27%).
LCMS m/z =873.3[M+1]⁺.

### Step 2: [7-[3-[5-[[[(2R)-2-hydroxy-2-(5-hydroxy-3-oxo-4H-1,4-benzoxazin-8-yl)ethyl]amino ]methyl]benzotriazol-1-yl]propyl]-7-azaspiro[3.5]nonan-2-yl] 2-hydroxy-2,2-bis(2-thienyl)acetate; ditrifluoroacetic acid (Compound 2)

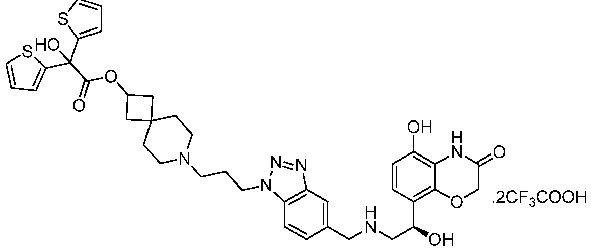

[7-[3-[5-[[[(2R)-2-[tert-butyl(dimethyl)silyl]oxy-2-(5-hydroxy-3-oxo-4H-1,4-benzoxa zin-8-yl)ethyl]amino]methyl]benzotriazol-1-yl]propyl]-7-azaspiro[3.5]nonan-2-yl] 2-hydroxy-2,2-bis(2-thienyl)acetate (2B) (0.42 g, 0.48 mmol) was dissolved in tetrahydrofuran (2 ml), and triethylamine trihydrofluoride (0.31 g, 1.9 mmol) was added thereto, followed by stirring at room temperature for 8 hours. The pH was adjusted to about 9 with a saturated aqueous solution of sodium bicarbonate. The resultant was extracted with methylene chloride (50 mL×2), washed with a saturated sodium chloride solution, dried over anhydrous sodium sulfate, and concentrated under reduced pressure. The residue was purified by preparative liquid column chromatography (liquid preparation condition: C18 preparative column; Eluent: acetonitrile (A) and 0.05% TFA in deionized water (B); Gradient elution: A:B=20%-25%; Elution time: 32 min) to obtain the title compound [7-[3-[5-[[[(2R)-2-hydroxy-2-(5-hydroxy-3-oxo-4H-1,4-benzoxazin-8-yl)ethyl]amino ]methyl]benzotriazol-1-yl]propyl]-7-azaspiro[3.5]nonan-2-yl] 2-hydroxy-2,2-bis(2-thienyl)acetate; ditrifluoroacetic acid (Compound 2) as a white solid (0.17 g, yield 36%).
¹H NMR (400 MHz, CD₃OD) δ 8.23 (s, 1H), 7.94 (d, 1H), 7.73 (dd, 1H), 7.39 (dd, 2H), 7.15 (br, 2H), 7.03 (d, 1H), 7.00 (dd, 2H), 6.57 (d, 1H), 5.21 (dd, 1H), 5.17 - 5.14 (m, 1H), 4.88 - 4.86 (m, 2H), 4.50 (s, 2H), 4.39 (dd, 2H), 3.54 - 3.39 (m, 2H), 3.29 - 3.24 (m, 2H), 3.21 (d, 1H), 3.09 (dd, 1H), 2.99 - 2.94 (m, 2H), 2.57 - 2.46 (m, 3H), 2.40 - 2.35 (m, 1H), 2.02 - 1.96 (m, 3H), 1.87 - 1.80 (m, 3H).
LCMS m/z=759.2[M+1]⁺.

### Example 3:

### [7-[3-[5-[[[(2R)-2-(3-formamido-4-hydroxy-phenyl)-2-hydroxy-ethyl]amino]methyl]b enzotriazol-1-yl]propyl]-7-azaspiro[3.5]nonan-2-yl] 2-hydroxy-2,2-bis(2-thienyl)acetate; ditrifluoroacetic acid (Compound 3)

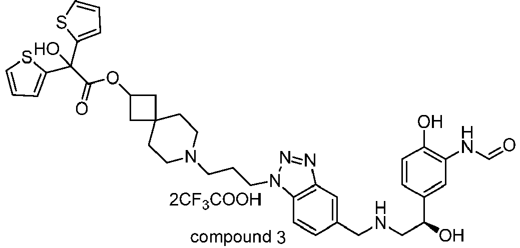

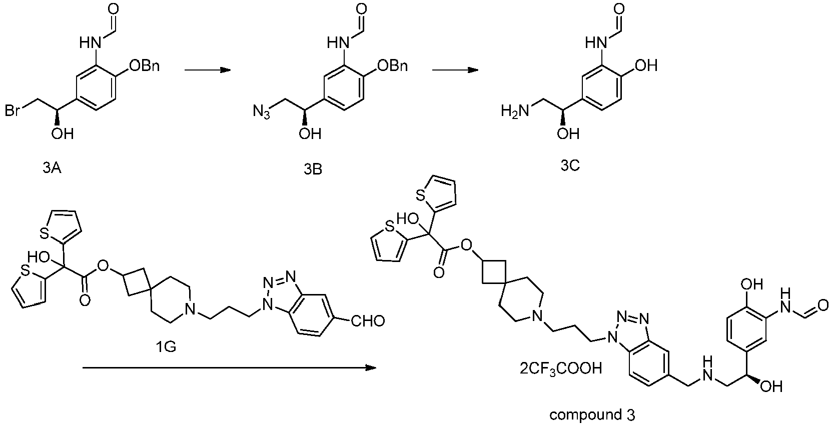

### Step 1: N-[5-[(1R)-2-azido-1-hydroxy-ethyl]-2-benzyloxy-phenyl]formamide (3B)

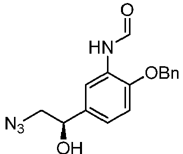

N-[2-benzyloxy-5-[(1R)-2-bromo-1-hydroxy-ethyl]phenyl]formamide (**3A**) (which can be prepared according to Bioorganic & Medicinal Chemistry Letters, 22(4), 1523-1526; 2012) (7 g, 19.99 mmol) was dissolved in N,N-dimethylformamide (100 ml), and sodium azide (1.95 g, 29.98 mmol) was added thereto, followed by reaction at 75°C for 6 hours. The reaction solution was extracted after addition of ethyl acetate (200 ml) and water (200 ml). The organic phase was washed with water (200 mL×2) and with saturated brine (200 mL×1), dried over anhydrous sodium sulfate, filtered, and the filtrate was concentrated under reduced pressure, to obtain the title compound N-[5-[(1R)-2-azido-1-hydroxy-ethyl]-2-benzyloxy-phenyl]formamide (3B) as a yellowish white solid (5.3 g, 85%).
¹H NMR (400 MHz, DMSO-d6) δ 9.61 - 9.46 (m, 1H), 8.36 (d, 1H), 8.21 (d, 1H), 7. 53-7.51 (m, 2H), 7.45 - 7.27 (m, 3H), 7.19 - 6.97 (m, 2H), 5.77-5.69 (m, 1H), 5.21 (s, 2H), 4.75-4.65 (m, 1H), 3.38 - 3.18 (m, 2H).
LCMS m/z=335.2[M+23].

### Step 2: N-[5-[(lR)-2-amino-1-hydroxy-ethyl]-2-hydroxy-phenyl]formamide (3C)

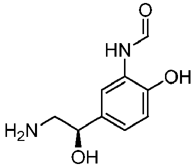

N-[5-[(1R)-2-azido-1-hydroxy-ethyl]-2-benzyloxy-phenyl]formamide (3B) (3.0 g, 9.6 mmol) was dissolved in methanol (80 ml) and methylene chloride (20 ml), to which 10% (w/w) Pd/C (3 g) was added, and a hydrogenation reaction was carried out at 45°C for 48 hours. The reaction solution was filtered by suction through diatomite and rinsed 4 times with methanol (100 ml). The filtrate was concentrated to obtain the title compound N-[5-[(1R)-2-amino-1-hydroxy-ethyl]-2-hydroxy-phenyl]formamide (3C) as a yellow solid (1.8 g, yield: 96%).
¹H NMR (400 MHz, CD₃OD) δ 8.32 (s, 1H), 8.05 (d, 1H), 7.07 - 6.98 (m, 1H), 6.92 - 6.82 (m, 1H), 4.63 (dd, 1H), 2.94 - 2.80 (m, 2H).
LCMS m/z=197.2[M+1].

### Step 3: [7-[3-[5-[[[(2R)-2-(3-formamido-4-hydroxy-phenyl)-2-hydroxy-ethyl]amino]methyl]b enzotriazol-1-yl]propyl]-7-azaspiro[3.5]nonan-2-yl] 2-hydroxy-2,2-bis(2-thienyl)acetate; ditrifluoroacetic acid (Compound 3)

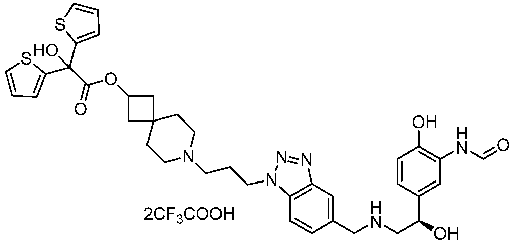

[7-[3-(5-formylbenzotriazol-1-yl)propyl]-7-azaspiro[3.5]nonan-2-yl] 2-hydroxy-2,2-bis(2-thienyl)acetate (1G) (0.400 g, 0.726 mmol) was dissolved in methanol (10 ml), to which N-[5-[(1R)-2-amino-1-hydroxy-ethyl]-2-hydroxy-phenyl]formamide (3C) (0.171 g, 0.872 mmol) and anhydrous zinc chloride (0.396 g, 2.91 mmol) were added, followed by reaction at 55°C for 1 h. Sodium cyanoborohydride (0.137 g, 2.18 mmol) was added, followed by reaction at 55°C for 2 h. The reaction solution was extracted after addition of methylene chloride (50 ml) and a saturated solution of sodium bicarbonate (20 ml). The aqueous phase was extracted with methylene chloride (30 mL×2). The organic phases were combined, dried over anhydrous sodium sulfate, filtered, and the filtrate was concentrated under reduced pressure. The residue was purified by preparative liquid column chromatography (preparation condition: C18 reverse preparative column; Mobile phase: 0.05% TFA in deionized water (A) and 0.05% TFA in acetonitrile (B); Gradient elution with A:B=5%-100% for 10 min, followed by elution with 100% B for 5 min; Flow rate: 1.0 ml/min; Column temperature: 40°C) to obtain the title compound [7-[3-[5-[[[(2R)-2-(3-formamido-4-hydroxy-phenyl)-2-hydroxy-ethyl]amino]methyl]b enzotriazol-1-yl]propyl]-7-azaspiro[3.5]nonan-2-yl] 2-hydroxy-2,2-bis(2-thienyl)acetate; ditrifluoroacetic acid (Compound 3) as a yellow solid (0.100 g, yield 14.4%).
¹H NMR (400 MHz, CD₃OD) δ 8.31 (s, 1H), 8.23 (s, 1H), 8.11 (d, 1H), 7.94 (d, 1H), 7.75 (dd, 1H), 7.39 (dd, 2H), 7.15 (s, 2H), 7.05 (dd, 1H), 6.99 (dd, 2H), 6.88 (d, 1H), 5.19 - 5.09 (m, 1H), 4.94-4.89 (m, 1H), 4.89-4.86 (m, 2H), 4.50 (s, 2H), 3.45 (dd, 2H), 3.28 - 3.11 (m, 4H), 3.01 - 2.84 (m, 2H), 2.57 - 2.44 (m, 3H), 2.37 (s, 1H), 2.02-1.90(dd, 3H), 1.89 - 1.73 (m, 3H).
¹⁹F NMR (376 MHz, CD₃OD) δ -75.39.
LCMS m/z=366.2[(M+2)/2].

### Example 4:

### [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-cyclopentyl-2-hydroxy-2-phenyl-acetate; ditrifluoroacetic acid (Compound 4)

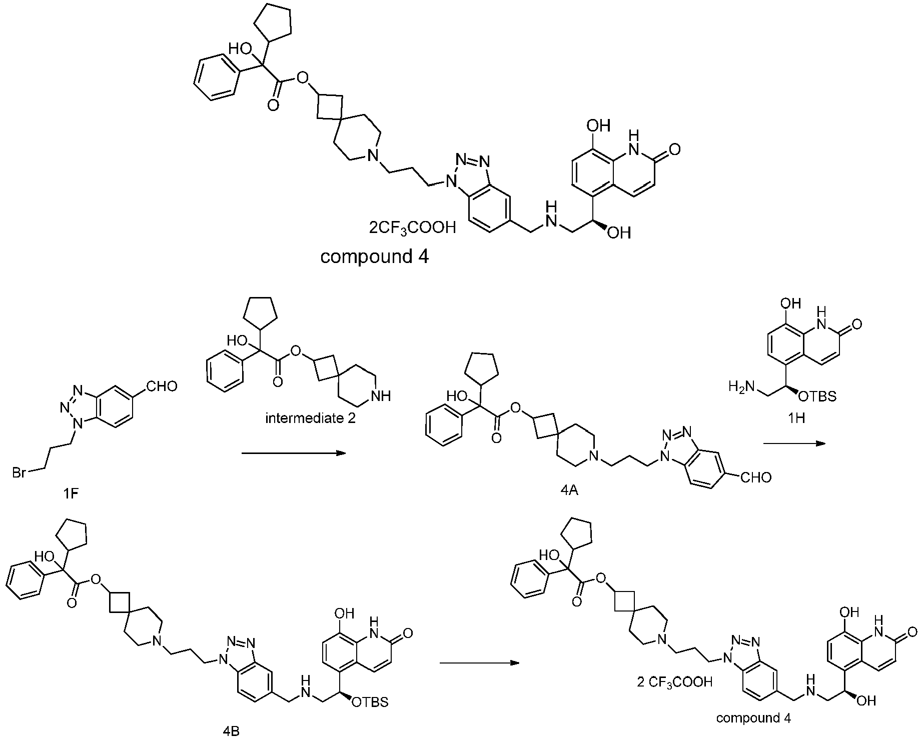

### Step 1: [7-[3-(5-formylbenzotriazol-1-yl)propyl]-7-azaspiro[3.5]nonan-2-yl] 2-cyclopentyl-2-hydroxy-2-phenyl-acetate (4A)

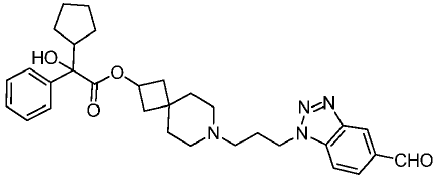

7-azaspiro[3.5]nonan-2-yl 2-cyclopentyl-2-hydroxy-2-phenyl-acetate (Intermediate 2) (0.500 g, 1.46 mmol) was dissolved in acetonitrile (20 ml), and 1-(3-bromopropyl)benzotriazole-5-carbaldehyde (1F) (0.429 g, 1.60 mmol) and triethylamine (0.263 g, 2.04 mmol) were added, followed by reaction at 85°C for 4 hours. The reaction solution was concentrated under reduced pressure and the residue was purified by silica gel column chromatography (ethyl acetate/petroleum ether (v/v) = 1:1 to 1:0), to obtain the title compound [7-[3-(5-formylbenzotriazol-1-yl)propyl]-7-azaspiro[3.5]nonan-2-yl] 2-cyclopentyl-2-hydroxy-2-phenyl-acetate (4A) as a yellow solid (0.650 g, yield: 84.1%).
LCMS m/z=531.3[M+1].

### Step 2: [7-[3-[5-[[[(2R)-2-[tert-butyl(dimethyl)silyl]oxy-2-(8-hydroxy-2-oxo-1H-quinolin-5-y 1)ethyl]amino]methyl]benzotriazol-1-yl]propyl]-7-azaspiro[3.5]nonan-2-yl] 2-cyclopentyl-2-hydroxy-2-phenyl-acetate (4B)

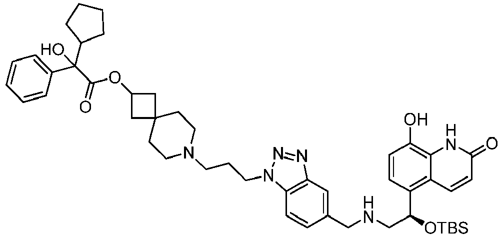

[7-[3-(5-formylbenzotriazol-1-yl)propyl]-7-azaspiro[3.5]nonan-2-yl] 2-cyclopentyl-2-hydroxy-2-phenyl-acetate (**4A**) (0.600 g, 1.13 mmol) was dissolved in methanol (10 ml), to which 5-[(1R)-2-amino-1-[t-butyl(dimethyl)silyl]oxymethyl]-8-hydroxy-1H-quinolin-2-one (1H) (0.378 g, 1.13 mmol) and anhydrous zinc chloride (0.616 g, 4.52 mmol) were added, followed by reaction at 55°C for 1 h. Sodium cyanoborohydride (0.213 g, 3.39 mmol) was added, followed by reaction at 55°C for 2 h. The reaction solution was extracted after addition of methylene chloride (50 ml) and a saturated solution of sodium bicarbonate (20 ml). The aqueous phase was extracted with methylene chloride (30 mL×2). The organic phases were combined, dried over anhydrous sodium sulfate, filtered, and the filtrate was concentrated under reduced pressure, to obtain the title compound [7-[3-[5-[[[(2R)-2-[tert-butyl(dimethyl)silyl]oxy-2-(8-hydroxy-2-oxo-1H-quinolin-5-y l)ethyl]amino]methyl]benzotriazol-1-yl]propyl]-7-azaspiro[3.5]nonan-2-yl] 2-cyclopentyl-2-hydroxy-2-phenyl-acetate (4B) as a yellow solid (0.45 g, yield 46.9%).
LCMS m/z=425.4[(M+2)/2].

### Step 3: [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-cyclopentyl-2-hydroxy-2-phenyl-acetate; ditrifluoroacetic acid (Compound 4)

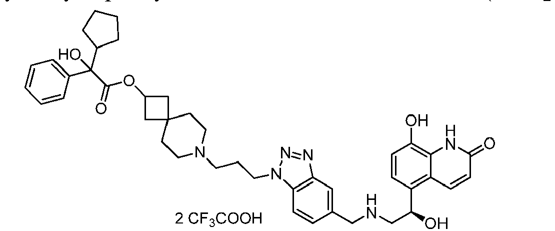

[7-[3-[5-[[[(2R)-2-[tert-butyl(dimethyl)silyl]oxy-2-(8-hydroxy-2-oxo-1H-quinolin-5-y 1)ethyl]amino]methyl]benzotriazol-1-yl]propyl]-7-azaspiro[3.5]nonan-2-yl] 2-cyclopentyl-2-hydroxy-2-phenyl-acetate (4B) (0.450 g, 0.530 mmol) was dissolved in tetrahydrofuran (5 ml), and triethylamine trihydrofluoride (0.854 g, 5.30 mmol) was added thereto, followed by reaction at room temperature for 24 hours. A 10% (v/v) methanol/methylene chloride solution (50 ml) was added, followed by addition of a saturated solution of sodium bicarbonate to adjust the pH to about 8, and then the solution was partitioned. The aqueous phase was extracted with a 10% (v/v) methanol/methylene chloride solution (50 mL×2). The organic phases were combined. The organic phase was washed with saturated brine (20 mL×1), dried over anhydrous sodium sulfate, filtered, and the filtrate was concentrated under reduced pressure. The residue was purified by preparative liquid column chromatography (preparation condition: C18 reverse preparative column; Mobile phase: 0.05% TFA in deionized water (A) and 0.05% TFA in acetonitrile (B); Gradient elution with A:B=5%-100% for 10 min, followed by elution with 100% B for 5 min; Flow rate: 1.0 ml/min; Column temperature: 40°C) to obtain the title compound [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-cyclopentyl-2-hydroxy-2-phenyl-acetate; ditrifluoroacetic acid (Compound 4) as a white solid (0.100g, yield 19.6%).
¹H NMR (400 MHz, CD₃OD) δ 8.28 - 8.19 (m, 2H), 7.91 (d, 1H), 7.73 (d, 1H), 7.67 - 7.59 (m, 2H), 7.40 - 7.23 (m, 4H), 7.04 (d, 1H), 6.58 (d, 1H), 5.49-5.40 (m, 1H), 5.08 - 4.97 (m, 1H), 4.89-4.85 (m, 2H), 4.57 - 4.47 (m, 2H), 3.55-3.35 (m, 2H), 3.31 - 3.21 (m, 4H), 3.06 - 2.79 (m, 3H), 2.58-2.20 (m, 4H), 2.00-1.75(m, 6H), 1.70 - 1.31 (m, 8H).
¹⁹F NMR (376 MHz, CD₃OD) δ -75.33.
LCMS m/z=368.3[(M+2)/2].

### Example 5:

### Isomer 1 of [7-[3-[5-[[[(2R)-2-hydroxy-2-(8-hydroxy-2-oxo-1H-quinolin-5-yl)ethyl] amino]methyl]benzotriazol-1-yl]propyl]-7-azaspiro[3.5]nonan-2-yl] 2-cyclopentyl-2-hydroxy-2-phenyl- acetate; ditrifluoroacetic acid (Isomer 1 of Compound 4)

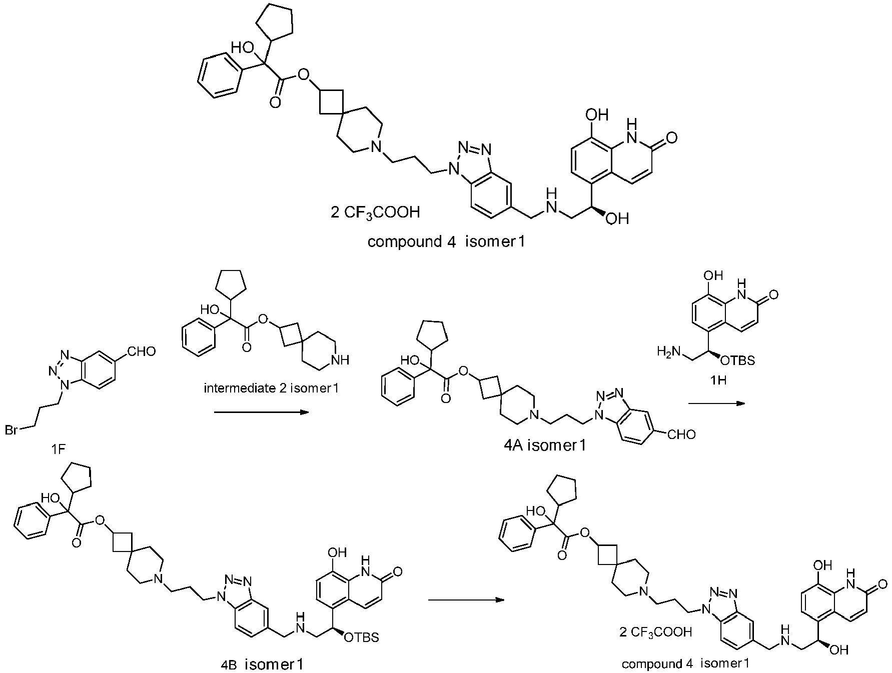

### Step 1: Isomer 1 of [7-[3-(5-formylbenzotriazol-1-yl)propyl]-7-azaspiro[3.5]nonan-2-yl] 2-cyclopentyl-2-hydroxy-2-phenyl-acetate (Isomer 1 of 4A)

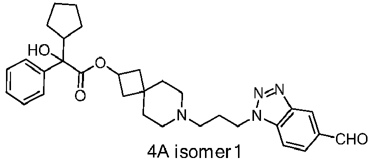

Isomer 1 of 7-azaspiro[3.5]nonan-2-yl 2-cyclopentyl-2-hydroxy-2-phenyl-acetate (Isomer 1 of Intermediate 2) (0.600 g, 1.75 mmol) was dissolved in acetonitrile (20 ml), and 1-(3-bromopropyl)benzotriazole-5-carbaldehyde (1F) (0.515 g, 1.92 mmol) and diisopropylethylamine (0.316 g, 2.45 mmol) were added, followed by reaction at 85°C for 4 hours. The reaction solution was concentrated and the residue was purified by silica gel column chromatography (ethyl acetate/petroleum ether (v/v) = 1:1 to 1:0), to obtain the title compound Isomer 1 of [7-[3-(5-formylbenzotriazol-1-yl)propyl]-7-azaspiro[3.5]nonan-2-yl] 2-cyclopentyl-2-hydroxy-2-phenyl-acetate (Isomer 1 of 4A) as a yellow solid (0.550 g, yield: 59.3%).
¹H NMR (400 MHz, CDCl₃) δ 10.14 (s, 1H), 8.56 (d, 1H), 8.11 - 8.00 (m, 1H), 7.74 - 7.67 (m, 1H), 7.66 - 7.41 (m, 2H), 7.37-7.30 (m, 2H), 7.28 - 7.23 (m, 1H), 5.07 - 4.91 (m, 1H), 4.75 (t, 2H), 2.96-2.84 (m, 1H), 2.40-2.30 (m, 4H), 2.28-2.18 (m, 4H), 1.88 -1.28 (m, 16H).
LCMS m/z =531.3[M+1].

### Step 2: Isomer 1 of [7-[3-[5-[[[(2R)-2-[tert-butyl(dimethyl)silyl]oxy-2-(8-hydroxy-2-oxo-1H-quinolin-5-y l)ethyl]amino]methyl]benzotriazol-1-yl]propyl]-7-azaspiro[3.5]nonan-2-yl] 2-cyclopentyl-2-hydroxy-2-phenyl-acetate (Isomer 1 of 4B)

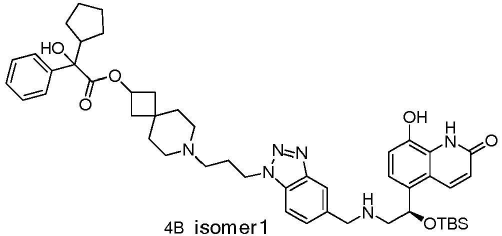

Isomer 1 of [7-[3-(5-formylbenzotriazol-1-yl)propyl]-7-azaspiro[3.5]nonan-2-yl] 2-cyclopentyl-2-hydroxy-2-phenyl-acetate (Isomer 1 of 4A) (0.550 g, 1.04 mmol) was dissolved in methanol (10 ml), to which 5-[(1R)-2-amino-1-[t-butyl(dimethyl)silyl]oxymethyl]-8-hydroxy-1H-quinolin-2-one (1H) (0.347 g, 1.04 mmol) and anhydrous zinc chloride (0.565 g, 4.15 mmol) were added, followed by reaction at 55°C for 1 h. Sodium cyanoborohydride (0.195 g, 3.11 mmol) was added, followed by reaction at 55°C for 2 h. The reaction solution was extracted after addition of methylene chloride (50 ml) and a saturated solution of sodium bicarbonate (20 ml). The aqueous phase was extracted with methylene chloride (30 mL×2). The organic phases were combined, dried over anhydrous sodium sulfate, filtered, and the filtrate was concentrated under reduced pressure, to obtain the title compound Isomer 1 of [7-[3-[5-[[[(2R)-2-[tert-butyl(dimethyl)silyl]oxy-2-(8-hydroxy-2-oxo-1H-quinolin-5-y 1)ethyl]amino]methyl]benzotriazol-1-yl]propyl]-7-azaspiro[3.5]nonan-2-yl] 2-cyclopentyl-2-hydroxy-2-phenyl-acetate (Isomer 1 of 4B) as a yellow solid (0.80g, yield 90%).
LCMS m/z =425.4[(M+2)/2].

### Step 3: Isomer 1 of [7-[3-[5-[[[(2R)-2-hydroxy-2-(8-hydroxy-2-oxo-1H-quinolin-5-yl)ethyl] amino] methyl]benzotriazol-1-yl]propyl]-7-azaspiro[3.5]nonan-2-yl] 2-cyclopentyl-2-hydroxy-2-phenyl- acetate; ditrifluoroacetic acid (Isomer 1 of Compound 4)

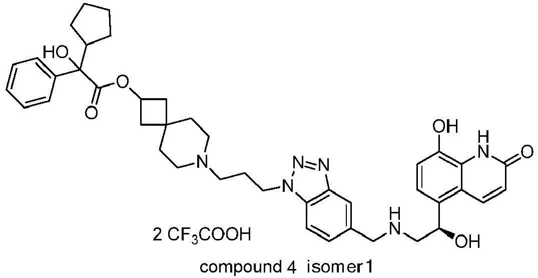

Isomer 1 of [7-[3-[5-[[[(2R)-2-[tert-butyl(dimethyl)silyl]oxy-2-(8-hydroxy-2-oxo-1H-quinolin-5-y 1)ethyl]amino]methyl]benzotriazol-1-yl]propyl]-7-azaspiro[3.5]nonan-2-yl] 2-cyclopentyl-2-hydroxy-2-phenyl-acetate (Isomer 1 of 4B) (0.800 g, 0.942 mmol) was dissolved in tetrahydrofuran (5 ml), and triethylamine trihydrofluoride (1.52 g, 9.42 mmol) was added thereto, followed by reaction at room temperature for 24 hours. A 10% (v/v) methanol/methylene chloride solution (50 ml) was added, followed by addition of a saturated solution of sodium bicarbonate to adjust the pH to about 8, and then the solution was partitioned. The aqueous phase was extracted with a 10% (v/v) methanol/methylene chloride solution (50 mL×2). The organic phases were combined. The organic phase was washed with saturated brine (20 mL×1), dried over anhydrous sodium sulfate, filtered, and the filtrate was concentrated under reduced pressure. The residue was purified by preparative liquid column chromatography (preparation condition: C18 reverse preparative column; Mobile phase: 0.05% TFA in deionized water (A) and 0.05% TFA in acetonitrile (B); Gradient elution with A:B=5%-100% for 10 min, followed by elution with 100% B for 5 min; Flow rate: 1.0 ml/min; Column temperature: 40°C) to obtain the title compound Isomer 1 of [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-cyclopentyl-2-hydroxy-2-phenyl-acetate; ditrifluoroacetic acid (Isomer 1 of Compound 4) as a white solid (0.150 g, yield 19.6%).
¹H NMR (400 MHz, CD₃OD) δ 8.26 - 8.16 (m, 2H), 7.89 (d, 1H), 7.70 (d, 1H), 7.65 - 7.57 (m, 2H), 7.36 - 7.19 (m, 4H), 7.01 (d, 1H), 6.55 (d, 1H), 5.48-5.37 (m, 1H), 5.05 - 4.93 (m, 1H), 4.87-4.83 (m, 2H), 4.50 (s, 2H), 3.53-3.35 (m, 2H), 3.28-3.16 (m, 4H), 3.04-2.78 (m, 3H), 2.56-2.20 (m, 4H), 1.98-1.74 (m, 6H), 1.70-1.42 (m, 6H), 1.39 - 1.27 (m, 2H).
¹⁹F NMR (376 MHz, CD₃OD) δ -75.32.
LCMS m/z=368.4[(M+2)/2].

### Example 6:

### Isomer 2 of [7-[3-[5-[[[(2R)-2-hydroxy-2-(8-hydroxy-2-oxo-1H-quinolin-5-yl)ethyl] amino]methyl]benzotriazol-1-yl]propyl]-7-azaspiro[3.5]nonan-2-yl] 2-cyclopentyl-2-hydroxy-2-phenyl- acetate; ditrifluoroacetic acid (Isomer 2 of Compound 4)

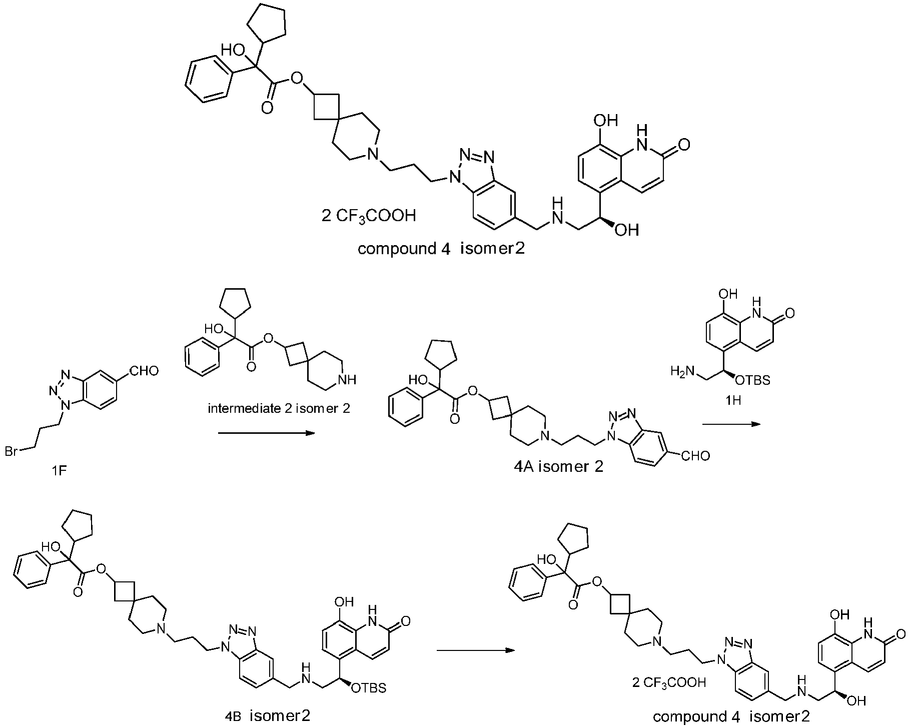

### Step 1: Isomer 2 of [7-[3-(5-formylbenzotriazol-1-yl)propyl]-7-azaspiro[3.5]nonan-2-yl] 2-cyclopentyl-2-hydroxy-2-phenyl-acetate (Isomer 2 of 4A)

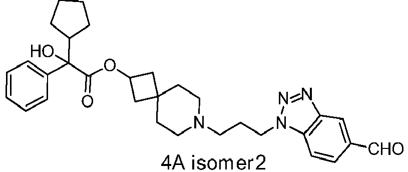

Isomer 2 of 7-azaspiro[3.5]nonan-2-yl 2-cyclopentyl-2-hydroxy-2-phenyl-acetate (Isomer 2 of Intermediate 2) (0.600 g, 1.75 mmol) was dissolved in acetonitrile (20 ml), and 1-(3-bromopropyl)benzotriazole-5-carbaldehyde (0.515 g, 1.92 mmol) and diisopropylethylamine (0.316 g, 2.45 mmol) were added, followed by reaction at 85°C for 4 hours. The reaction solution was concentrated under reduced pressure and the residue was purified by silica gel column chromatography (ethyl acetate/petroleum ether (v/v) = 1:1 to 1:0), to obtain the title compound Isomer 2 of [7-[3-(5-formylbenzotriazol-1-yl)propyl]-7-azaspiro[3.5]nonan-2-yl] 2-cyclopentyl-2-hydroxy-2-phenyl-acetate (Isomer 2 of 4A) as a yellow solid (0.640g, yield: 68.9%).
¹H NMR (400 MHz, CDCl₃) δ 10.14 (s, 1H), 8.55 (s, 1H), 8.07 (d, 1H), 7.74-7.67 (m, 1H), 7.65-7.57 (m, 2H), 7.33 (t, 2H), 7.29 - 7.23 (m, 1H), 5.05-4.93 (m, 1H), 4.76 (t, 2H), 2.96 - 2.82 (m, 1H), 2.54 (s, 4H), 2.40-2.22 (m, 4H), 1.93 - 1.22 (m, 16H). LCMS m/z =531.3[M+1].

### Step 2: Isomer 2 of [7-[3-[5-[[[(2R)-2-[tert-butyl(dimethyl)silyl]oxy-2-(8-hydroxy-2-oxo-1H-quinolin-5-y l)ethyl]amino]methyl]benzotriazol-1-yl]propyl]-7-azaspiro[3.5]nonan-2-yl] 2-cyclopentyl-2-hydroxy-2-phenyl-acetate (Isomer 2 of 4B)

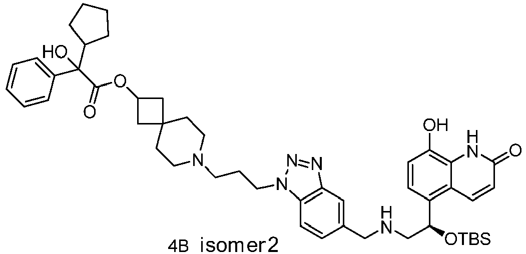

Isomer 2 of [7-[3-(5-formylbenzotriazol-1-yl)propyl]-7-azaspiro[3.5]nonan-2-yl] 2-cyclopentyl-2-hydroxy-2-phenyl-acetate (Isomer 2 of 4A) (0.640 g, 1.21 mmol) was dissolved in methanol (10 ml), to which 5-[(1R)-2-amino-1-[t-butyl(dimethyl)silyl]oxymethyl]-8-hydroxy-1H-quinolin-2-one (1H) (0.403 g, 1.21 mmol) and anhydrous zinc chloride (0.658 g, 4.82 mmol) were added, followed by reaction at 55°C for 1 h. Sodium cyanoborohydride (0.227 g, 3.62 mmol) was added, followed by reaction at 55°C for 2 h. The reaction solution was extracted after addition of methylene chloride (50 ml) and a saturated solution of sodium bicarbonate (20 ml). The aqueous phase was extracted with methylene chloride (30 mL×2). The organic phases were combined, dried over anhydrous sodium sulfate, filtered, and the filtrate was concentrated under reduced pressure, to obtain the title compound Isomer 2 of [7-[3-[5-[[[(2R)-2-[tert-butyl(dimethyl)silyl]oxy-2-(8-hydroxy-2-oxo-1H-quinolin-5-y 1)ethyl]amino]methyl]benzotriazol-1-yl]propyl]-7-azaspiro[3.5]nonan-2-yl] 2-cyclopentyl-2-hydroxy-2-phenyl-acetate (Isomer 2 of 4B) as a yellow solid (0.80g, yield 77.9%).
LCMS m/z=425.4[(M+2)/2].

### Step 3: Isomer 2 of [7-[3-[5-[[[(2R)-2-hydroxy-2-(8-hydroxy-2-oxo-1H-quinolin-5-yl)ethyl]amino] methyl]benzotriazol-1-yl]propyl]-7-azaspiro[3.5]nonan-2-yl] 2-cyclopentyl-2-hydroxy-2-phenyl- acetate; ditrifluoroacetic acid (Isomer 2 of Compound 4)

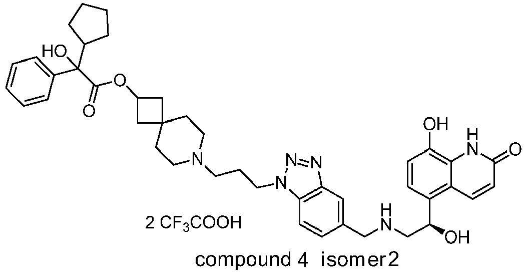

Isomer 2 of [7-[3-[5-[[[(2R)-2-[tert-butyl(dimethyl)silyl]oxy-2-(8-hydroxy-2-oxo-1H-quinolin-5-y l)ethyl]amino]methyl]benzotriazol-1-yl]propyl]-7-azaspiro[3.5]nonan-2-yl] 2-cyclopentyl-2-hydroxy-2-phenyl-acetate (Isomer 2 of 4B) (0.800 g, 0.942 mmol) was dissolved in tetrahydrofuran (5 ml), and triethylamine trihydrofluoride (1.52 g, 9.42 mmol) was added thereto, followed by reaction at room temperature for 24 hours. A 10% (v/v) methanol/methylene chloride solution (50 ml) was added, followed by addition of a saturated solution of sodium bicarbonate to adjust the pH to about 8, and then the solution was partitioned. The aqueous phase was extracted with a 10% (v/v) methanol/methylene chloride solution (50 mL×2). The organic phases were combined. The organic phase was washed with saturated brine (20 mL×1), dried over anhydrous sodium sulfate, filtered, and the filtrate was concentrated under reduced pressure. The residue was purified by preparative liquid column chromatography (preparation condition: C18 reverse preparative column; Mobile phase: 0.05% TFA in deionized water (A) and 0.05% TFA in acetonitrile (B); Gradient elution with A:B=5%-100% for 10 min, followed by elution with 100% B for 5 min; Flow rate: 1.0 ml/min; Column temperature: 40°C) to obtain the title compound Isomer 2 of [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-cyclopentyl-2-hydroxy-2-phenyl-acetate; ditrifluoroacetic acid (Isomer 2 of Compound 4) as a white solid (0.250g, yield 27.6%).
¹H NMR (400 MHz, CD₃OD) δ 8.27 - 8.17 (m, 2H), 7.89 (d, 1H), 7.71 (d, 1H), 7.61 (d, 2H), 7.35 - 7.19 (m, 4H), 7.01 (d, 1H), 6.56 (d, 1H), 5.48-5.30 (m, 1H), 5.04 - 4.96 (m, 1H), 4.85 (s, 2H), 4.51 (s, 2H), 3.52-3.34 (m, 2H), 3.29 - 3.18 (m, 4H), 3.03-2.77 (m, 3H), 2.55 - 2.21 (m, 4H), 1.98-1.73 (m, 6H), 1.72 - 1.52 (m, 5H), 1.51-1.26 (m, 3H).
¹⁹F NMR (376 MHz, CD₃OD) δ -75.33.
LCMS m/z=368.4[(M+2)/2].

### Example 7:

### [7-[3-[6-[[[(2R)-2-hydroxy-2-(8-hydroxy-2-oxo-1H-quinolin-5-yl)ethyl]amino]methyl ]-2-oxo-1,3-benzothiazol-3-yl]propyl]-7-azaspiro[3.5]nonan-2-yl] 2-hydroxy-2,2-bis(2-thienyl)acetate; ditrifluoroacetic acid (Compound 7)

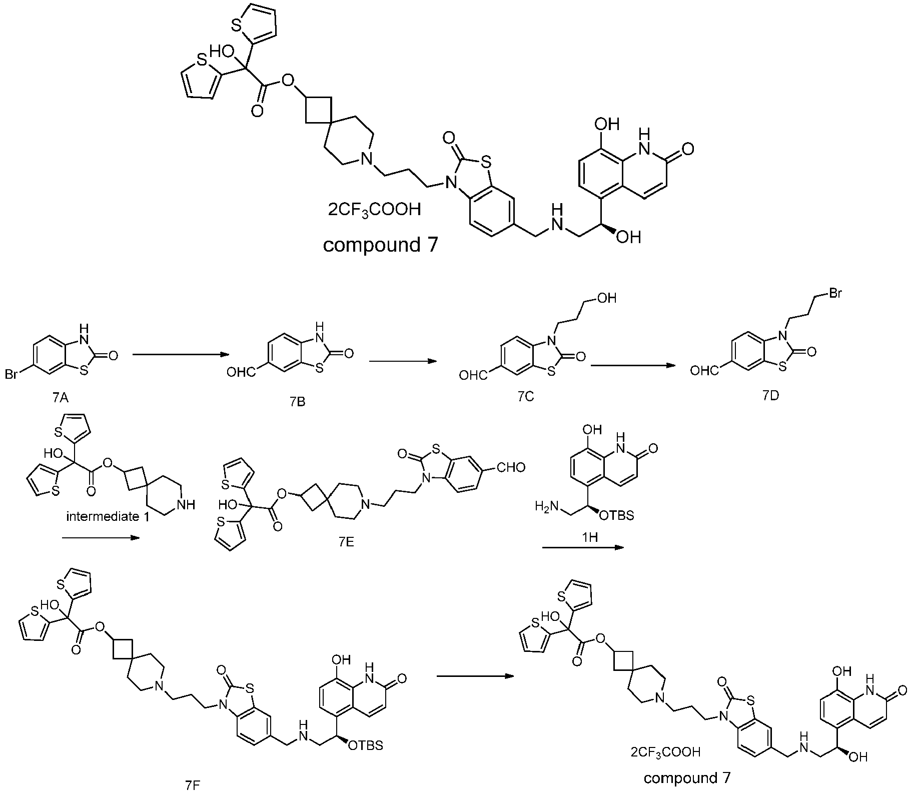

### Step 1: 2-oxo-3H-1,3-benzothiazole-6-carbaldehyde (7B)

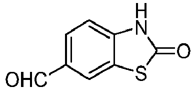

6-bromo-3H-1,3-benzothiazol-2-one (7A) (3.00 g, 13.0 mmol) was dissolved in tetrahydrofuran (15 ml), and under N₂ protection, a solution of 2M isopropyl magnesium chloride in tetrahydrofuran (7 mL, 14 mmol) were added at -10°C. The temperature was raised to 0°C, followed by reaction for 1 hour. A solution of n-butyllithium in tetrahydrofuran (21 ml, 52 mmol) was added at -25°C, followed by reaction at -10°C for 30 min. N,N-dimethylformamide (9.5 g, 130 mmol) was added at -10°C, and the temperature was gradually raised to room temperature, followed by reaction for 0.5 hours. The reaction solution was poured into water (50 ml) containing 5 g citric acid, and was extracted by addition of ethyl acetate (50 ml). The aqueous phase was extracted with ethyl acetate (50 mL×1). The organic phases were combined. The organic phase was dried over anhydrous sodium sulfate and filtered. The filtrate was concentrated under reduced pressure, made into a slurry with 50% (v/v) ethyl acetate/petroleum ether, and purified by silica gel column chromatography (ethyl acetate/petroleum ether (v/v) = 0:1 to 1:4) to obtain the title compound 2-oxo-3H-1,3-benzothiazole-6-carbaldehyde (7B) as a white solid (1.60 g, yield 68.7%).
¹H NMR (400 MHz, DMSO-d6) δ 12.37 (s, 1H), 9.90 (s, 1H), 8.16 (d, 1H), 7.84 (dd, 1H), 7.29 (d, 1H).
LCMS m/z=180.1[M+1].

### Step 2: 3-(3-hydroxypropyl)-2-oxo-1,3-benzothiazole-6-carbaldehyde (7C)

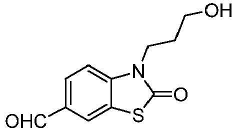

2-oxo-3H-1,3-benzothiazole-6-carbaldehyde (7B) (1.10 g, 6.14 mmol) was dissolved in tetrahydrofuran (20 ml), to which bromopropanol (1.02 g, 7.37 mmol), triphenylphosphine (1.93 g, 7.37 mmol) and DIAD (Diisopropyl azodiformate, CAS: 2446-83-5) (1.49 g, 7.37 mmol) were added, followed by reaction at room temperature for 3 hours. The reaction solution was concentrated, and the residue was purified by silica gel column chromatography (ethyl acetate : petroleum ether (v/v) = 0:1 to 3:7) to give the title compound 3-(3-hydroxypropyl)-2-oxo-1,3-benzothiazole-6-carbaldehyde (7C) as a white solid (1.46 g, yield: 100%).

### Step 3: 3-(3-bromopropyl)-2-oxo-1,3-benzothiazole-6-carbaldehyde (7D)

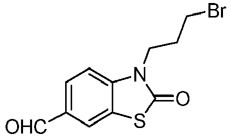

3-(3-hydroxypropyl)-2-oxo-1,3-benzothiazole-6-carbaldehyde (7C) (1.46 g, 6.15 mmol) was dissolved in methylene chloride (30 ml), to which carbon tetrabromide (4.90 g, 14.8 mmol) and triphenylphosphine (3.87 g, 14.8 mmol) were added, followed by reaction at room temperature for 2 hours. The reaction solution was concentrated under reduced pressure, and the residue was purified by silica gel column chromatography (ethyl acetate : petroleum ether (v/v) = 0:1 to 1:4) to give the title compound 3-(3-bromopropyl)-2-oxo-1,3-benzothiazole-6-carbaldehyde (7D) as a white solid (0.550 g, yield: 29.8%).
¹H NMR (400 MHz, CDCl₃) δ 7.70 (dd, 1H), 7.53 (dd, 1H), 7.17-7.11 (m, 1H), 6.67 (s, 1H), 4.15 - 4.06 (m, 2H), 3.51-3.39 (m, 2H), 2.40 - 2.23 (m, 2H).

### Step 4: [7-[3-(6-formyl-2-oxo-1,3-benzothiazol-3-yl)propyl]-7-azaspiro[3.5]nonan-2-yl] 2-hydroxy-2,2-bis(2-thienyl)acetate (7E)

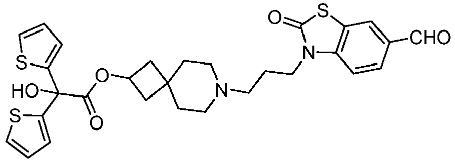

7-azaspiro[3.5]nonan-2-yl 2-hydroxy-2,2-bis(2-thienyl)acetate (Intermediate 1) (0.666 g, 1.83 mmol) was dissolved in acetonitrile (20 ml), and 3-(3-bromopropyl)-2-oxo-1,3-benzothiazole-6-carbaldehyde (7D) (0.550 g, 1.83 mmol) and diisopropylethylamine (0.331 g, 2.56 mmol) were added thereto, followed by reaction at 85°C for 4 hours. The reaction solution was cooled to room temperature and concentrated under reduced pressure, and the residue was purified by silica gel column chromatography (ethyl acetate/petroleum ether (v/v) = 1:1 to 1:0), to obtain the title compound [7-[3-(6-formyl-2-oxo-1,3-benzothiazol-3-yl)propyl]-7-azaspiro[3.5]nonan-2-yl] 2-hydroxy-2,2-bis(2-thienyl)acetate (7E) as a yellow solid (0.480g, yield: 45.0%).
¹H NMR (400 MHz, CDCl₃) δ 9.95 (s, 1H), 7.97 (d, 1H), 7.84 (dd, 1H), 7.34 (d, 1H), 7.29 (m, 2H), 7.17 (m, 2H), 6.98 (m, 2H), 5.18 - 5.09 (m, 1H), 4.07 (t, 2H), 2.41 (s, 2H), 2.37 - 2.22 (m, 4H), 1.99 (s, 2H), 1.91-1.78 (m, 2H), 1.62 (s, 6H).
LCMS m/z=583.2[M+1].

### Step 5: [7-[3-[6-[[[(2R)-2-[tert-butyl(dimethyl)silyl]oxy-2-(8-hydroxy-2-oxo-1H-quinolin-5-y l)ethyl]amino]methyl]-2-oxo-1,3-benzothiazol-3-yl]propyl]-7-azaspiro[3.5]nonan-2-yl] 2-hydroxy-2,2-bis(2-thienyl)acetate (7F)

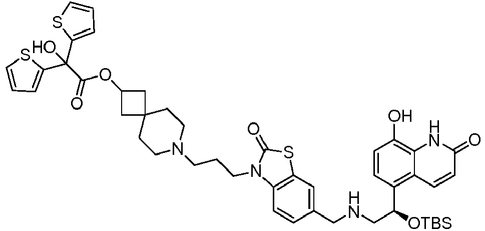

[7-[3-(6-formyl-2-oxo-1 ,3 -benzothiazol-3 -yl)propyl] -7-azaspiro [3.5]nonan-2-yl] 2-hydroxy-2,2-bis(2-thienyl)acetate (7E) (0.480 g, 0.824 mmol) was dissolved in methanol (10 ml), to which 5-[(1R)-2-amino-1-[t-butyl(dimethyl)silyl]oxymethyl]-8-hydroxy-1H-quinolin-2-one (1H) (0.331 g, 0.988 mmol) and anhydrous zinc chloride (0.449 g, 3.29 mmol) were added, followed by reaction at 55°C for 1 h. Sodium cyanoborohydride (0.155 g, 2.47 mmol) was added, followed by reaction at 55°C for 2 h. The reaction solution was extracted after addition of methylene chloride (50 ml) and a saturated solution of sodium bicarbonate (20 ml). The aqueous phase was extracted with methylene chloride (30 mL×2). The organic phases were combined, dried over anhydrous sodium sulfate, filtered, and the filtrate was concentrated under reduced pressure, to obtain the title compound [7-[3-[6-[[[(2R)-2-[tert-butyl(dimethyl)silyl]oxy-2-(8-hydroxy-2-oxo-1H-quinolin-5-y l)ethyl]ammo]methyl]-2-oxo-1,3-benzothiazol-3-yl]propyl]-7-azaspiro[3.5]nonan-2-yl] 2-hydroxy-2,2-bis(2-thienyl)acetate (7F) as a yellow solid (0.742 g, yield: 100%).
LCMS m/z=451.3[(M+2)/2].

### Step 6: [7-[3-[6-[[[(2R)-2-hydroxy-2-(8-hydroxy-2-oxo-1H-quinolin-5-yl)ethyl]amino]methyl ]-2-oxo-1,3-benzothiazol-3-yl]propyl]-7-azaspiro[3.5]nonan-2-yl] 2-hydroxy-2,2-bis(2-thienyl)acetate; ditrifluoroacetic acid (Compound 7)

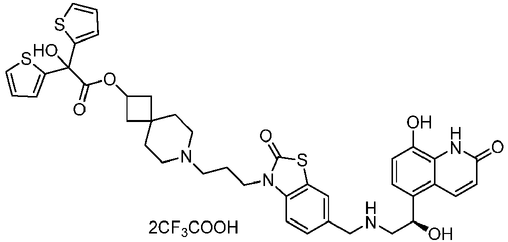

[7-[3-[6-[[[(2R)-2-[tert-butyl(dimethyl)silyl]oxy-2-(8-hydroxy-2-oxo-1H-quinolin-5-y l)ethyl]amino]methyl]-2-oxo-1,3-benzothiazol-3-yl]propyl]-7-azaspiro[3.5]nonan-2-yl] 2-hydroxy-2,2-bis(2-thienyl)acetate (7F) (0.742 g, 0.823 mmol) was dissolved in tetrahydrofuran (5 ml), and triethylamine trihydrofluoride (1.33 g, 8.23 mmol) was added thereto, followed by reaction at room temperature for 24 hours. A 10% (v/v) methanol/methylene chloride solution (50 ml) was added, followed by addition of a saturated solution of sodium bicarbonate to adjust the pH to about 8, and then the solution was partitioned. The aqueous phase was extracted with a 10% (v/v) methanol/methylene chloride solution (50 mL×2). The organic phases were combined. The organic phase was washed with saturated brine (20 mL×1), dried over anhydrous sodium sulfate, filtered, and the filtrate was concentrated under reduced pressure. The residue was purified by preparative liquid column chromatography (preparation condition: C18 reverse preparative column; Mobile phase: 0.05% TFA in deionized water (A) and 0.05% TFA in acetonitrile (B); Gradient elution with A:B=5%-100% for 10 min, followed by elution with 100% B for 5 min; Flow rate: 1.0 ml/min; Column temperature: 40°C) to obtain the title compound [7-[3-[6-[[[(2R)-2-hydroxy-2-(8-hydroxy-2-oxo-1H-quinolin-5-yl)ethyl]amino]methyl ]-2-oxo-1,3-benzothiazol-3-yl]propyl]-7-azaspiro[3.5]nonan-2-yl] 2-hydroxy-2,2-bis(2-thienyl)acetate; ditrifluoroacetic acid (Compound 7) as a white solid (0.200 g, yield 23.9%).
¹H NMR (400 MHz, CD₃OD) δ 8.23 (d, 1H), 7.69 (d, 1H), 7.51 (m, 1H), 7.40 - 7.32 (m, 3H), 7.27 (d, 1H), 7.13 (s, 2H), 7.04 - 6.92 (m, 3H), 6.60 (d, 1H), 5.42 (m, 1H), 5.22 - 5.04 (m, 1H), 4.33 (s, 2H), 4.11 (t, 2H), 3.43 (m, 2H), 3.26 - 3.11 (m, 4H), 3.01-2.81(m, 2H), 2.57-2.41 (m, 1H), 2.41-2.27 (m, 1H), 2.25 - 2.09 (m, 2H), 2.03 - 1.88 (m, 3H), 1.88 - 1.71 (m, 3H).
¹⁹F NMR (376 MHz, CD₃OD) δ -75.43.
LCMS m/z=394.1[(M+2)/2].

### Example 8:

### [7-[3-[6-[[[(2R)-2-hydroxy-2-(8-hydroxy-2-oxo-1H-qumolm-5-yl)ethyl]amino]methyl ]-2-oxo-1,3-benzoxazol-3-yl]propyl]-7-azaspiro[3.5]nonan-2-yl] 2-hydroxy-2,2-bis(2-thienyl)acetate; ditrifluoroacetic acid (Compound 8)

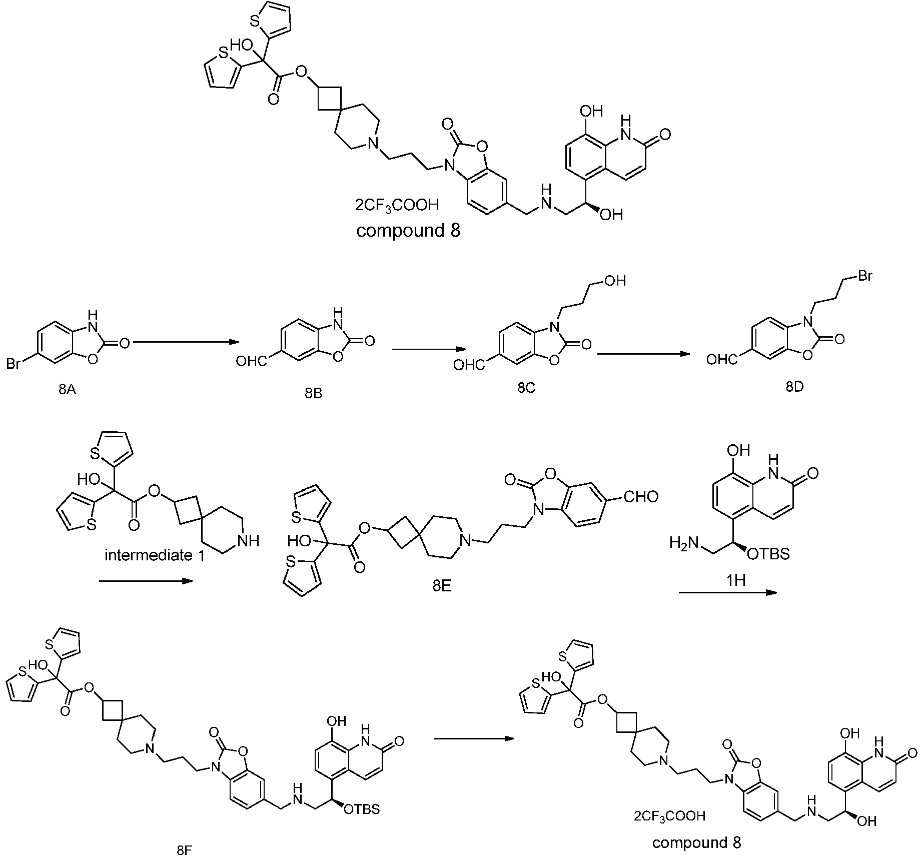

### Step 1: 2-oxo-3H-1,3-benzoxazole-6-carbaldehyde (8B)

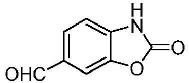

6-bromo-3H-1,3-benzoxazol-2-one (**8A**) (1.78g, 13 mmol) was dissolved in tetrahydrofuran (15 ml), and under N₂ protection, a solution of 2M isopropyl magnesium chloride in tetrahydrofuran (7 mL, 14 mmol) were added at -10°C. The temperature was raised to 0°C, followed by reaction for 1 hour. A solution of n-butyllithium in tetrahydrofuran (21 ml, 52 mmol) was added at -25°C, followed by reaction at -10°C for 30 min. N,N-dimethylformamide (9.5 g, 130 mmol) was added at -10°C, and the temperature was gradually raised to room temperature, followed by reaction for 0.5 hours. The reaction solution was poured into water (50 ml) containing 5 g citric acid, and was extracted by addition of ethyl acetate (50 ml). The aqueous phase was extracted with ethyl acetate (50 mL×1). The organic phases were combined. The organic phase was dried over anhydrous sodium sulfate and filtered.The filtrate was concentrated under reduced pressure, made into a slurry with 50% (v/v) ethyl acetate/petroleum ether, and purified by silica gel column chromatography (ethyl acetate/petroleum ether (v/v) = 0:1 to 1:4) to obtain the title compound 2-oxo-3H-1,3-benzoxazole-6-carbaldehyde (8B) as a white solid (1.60 g, yield 75.4%).
¹H NMR (400 MHz, DMSO-d6) δ 12.16 (s, 1H), 9.92 (s, 1H), 7.78 (dd, 1H), 7.74 (d, 1H), 7.29 (d, 1H).
LCMS m/z=164.1[M+1].

### Step 2: 3-(3-hydroxypropyl)-2-oxo-1,3-benzoxazole-6-carbaldehyde (8C)

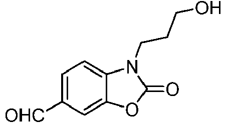

2-oxo-3H-1,3-benzoxazole-6-carbaldehyde (8B) (1.00 g, 6.14 mmol) was dissolved in tetrahydrofuran (20 ml), to which bromopropanol (1.33 g, 9.56 mmol), triphenylphosphine (2.51g, 9.56 mmol) and DIAD (Diisopropyl azodiformate) (1.93g, 9.56 mmol) were added, followed by reaction at room temperature for 3 hours. The reaction solution was concentrated, and the residue was purified by silica gel column chromatography (ethyl acetate : petroleum ether (v/v) = 0:1 to 3:7) to give the title compound 3-(3-hydroxypropyl)-2-oxo-1,3-benzoxazole-6-carbaldehyde (8C) as a white solid (1.30g, yield: 96.0%).

### Step 3: 3-(3-bromopropyl)-2-oxo-1,3-benzoxazole-6-carbaldehyde (8D)

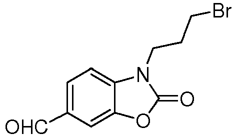

3-(3-hydroxypropyl)-2-oxo-1,3-benzoxazole-6-carbaldehyde (8C) (1.7 g, 7.7 mmol) was dissolved in methylene chloride (30 ml), to which carbon tetrabromide (6.10 g, 18 mmol) and triphenylphosphine (4.8 g, 18 mmol) were added, followed by reaction at room temperature for 2 hours. The reaction solution was concentrated under reduced pressure, and the residue was purified by silica gel column chromatography (ethyl acetate : petroleum ether (v/v) = 0:1 to 1:4) to give the title compound 3-(3-bromopropyl)-2-oxo-1,3-benzoxazole-6-carbaldehyde (8D) as a white solid (0.850 g, yield: 38.9%).
¹H NMR (400 MHz, CDCl₃) δ 7.54 (d, 1H), 7.37 (m, 1H), 7.05 (d, 1H), 6.68 (s, 1H), 4.01 (t, 2H), 3.45 (t, 2H), 2.41 - 2.30 (m, 2H).

### Step 4: [7-[3-(6-formyl-2-oxo-1,3-benzoxazol-3-yl)propyl]-7-azaspiro[3.5]nonan-2-yl] 2-hydroxy-2,2-bis(2-thienyl)acetate (8E)

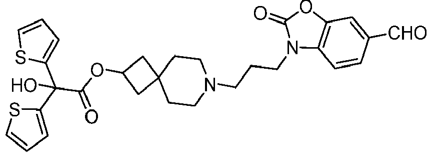

7-azaspiro[3.5]nonan-2-yl 2-hydroxy-2,2-bis(2-thienyl)acetate (Intermediate 1) (1.09 g, 2.99 mmol) was dissolved in acetonitrile (20 ml), and 3-(3-bromopropyl)-2-oxo-1,3-benzoxazole-6-carbaldehyde (8D) (0.850 g, 2.99 mmol) and diisopropylethylamine (0.541 g, 4.49 mmol) were added thereto, followed by reaction at 85°C for 4 hours. The reaction solution was concentrated under reduced pressure, and the residue was purified by silica gel column chromatography (ethyl acetate/petroleum ether (v/v) = 1:1 to 1:0), to obtain the title compound [7-[3-(6-formyl-2-oxo-1,3-benzoxazol-3-yl)propyl]-7-azaspiro[3.5]nonan-2-yl] 2-hydroxy-2,2-bis(2-thienyl)acetate (8E) as a yellow solid (0.630 g, yield: 37.2%).
¹H NMR (400 MHz, CDCl₃) δ 9.94 (s, 1H), 7.70 (m, 1H), 7.58 - 7.51 (m, 1H), 7.50 - 7.42 (m, 1H), 7.31 - 7.27 (m, 2H), 7.18-7.13 (m, 2H), 7.02 - 6.92 (m, 2H), 5.17 - 5.08 (m, 1H), 4.00 - 3.83 (m, 2H), 2.44 - 2.18 (m, 6H), 1.99 (s, 2H), 1.90 - 1.77 (m, 2H), 1.70-1.45 (m, 6H).
LCMS m/z=567.2[M+1].

### Step 5: [7-[3-[6-[[[(2R)-2-[tert-butyl(dimethyl)silyl]oxy-2-(8-hydroxy-2-oxo-1H-quinolin-5-y l)ethyl]amino]methyl]-2-oxo-1,3-benzoxazol-3-yl]propyl]-7-azaspiro[3.5]nonan-2-yl] 2-hydroxy-2,2-bis(2-thienyl)acetate (8F)

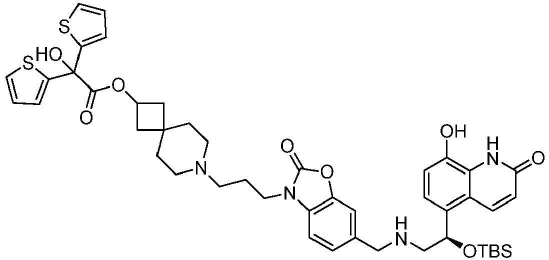

[7-[3-(6-formyl-2-oxo-1,3-benzoxazol-3-yl)propyl]-7-azaspiro[3.5]nonan-2-yl] 2-hydroxy-2,2-bis(2-thienyl)acetate (8E) (0.630 g, 1.11 mmol) was dissolved in methanol (10 ml), to which 5-[(1R)-2-amino-1-[t-butyl(dimethyl)silyl]oxymethyl]-8-hydroxy-1H-quinolin-2-one (1H) (0.446 g, 1.33 mmol) and anhydrous zinc chloride (0.606 g, 4.45 mmol) were added, followed by reaction at 55°C for 1 h. Sodium cyanoborohydride (0.210 g, 3.34 mmol) was added, followed by reaction at 55°C for 2 h. The reaction solution was extracted after addition of methylene chloride (50 ml) and a saturated solution of sodium bicarbonate (20 ml). The aqueous phase was extracted with methylene chloride (30 mL×2). The organic phases were combined, dried over anhydrous sodium sulfate, filtered, and the filtrate was concentrated under reduced pressure, to obtain the title compound [7-[3-[6-[[[(2R)-2-[tert-butyl(dimethyl)silyl]oxy-2-(8-hydroxy-2-oxo-1H-quinolin-5-y l)ethyl]amino]methyl]-2-oxo-1,3-benzoxazol-3-yl]propyl]-7-azaspiro[3.5]nonan-2-yl] 2-hydroxy-2,2-bis(2-thienyl)acetate (8F) as a yellow solid (0.984 g, yield: 100%).
LCMS m/z=443.2[(M+2)/2].

### Step 6: [7-[3-[6-[[[(2R)-2-hydroxy-2-(8-hydroxy-2-oxo-1H-quinolin-5-yl)ethyl]amino]methyl ]-2-oxo-1,3-benzoxazol-3-yl]propyl]-7-azaspiro[3.5]nonan-2-yl] 2-hydroxy-2,2-bis(2-thienyl)acetate; ditrifluoroacetic acid (Compound 8)

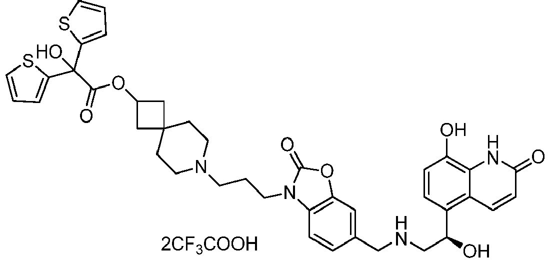

[7-[3-[6-[[[(2R)-2-[tert-butyl(dimethyl)silyl]oxy-2-(8-hydroxy-2-oxo-1H-quinolin-5-y l)ethyl]amino]methyl]-2-oxo-1,3-benzoxazol-3-yl]propyl]-7-azaspiro[3.5]nonan-2-yl] 2-hydroxy-2,2-bis(2-thienyl)acetate (8F) (0.984 g, 1.11 mmol) was dissolved in tetrahydrofuran (5 ml), and triethylamine trihydrofluoride (1.79 g, 11.1 mmol) was added thereto, followed by reaction at room temperature for 24 hours. A 10% (v/v) methanol/methylene chloride solution (50 ml) was added, followed by addition of a saturated solution of sodium bicarbonate to adjust the pH to about 8, and then the solution was partitioned . The aqueous phase was extracted with a 10% (v/v) methanol/methylene chloride solution (50 mL×2). The organic phases were combined. The organic phase was washed with saturated brine (20 mL×1), dried over anhydrous sodium sulfate, filtered, and the filtrate was concentrated under reduced pressure. The residue was purified by preparative liquid column chromatography (preparation condition: C18 reverse preparative column; Mobile phase: 0.05% TFA in deionized water (A) and 0.05% TFA in acetonitrile (B); Gradient elution with A:B=5%-100% for 10 min, followed by elution with 100% B for 5 min; Flow rate: 1.0 ml/min; Column temperature: 40°C) to obtain the title compound [7-[3-[6-[[[(2R)-2-hydroxy-2-(8-hydroxy-2-oxo-1H-quinolin-5-yl)ethyl]amino]methyl ]-2-oxo-1,3-benzoxazol-3-yl]propyl]-7-azaspiro[3.5]nonan-2-yl] 2-hydroxy-2,2-bis(2-thienyl)acetate; ditrifluoroacetic acid (Compound 8) as a white solid (0.120 g, yield 10.8%).
¹H NMR (400 MHz, CD₃OD) δ 8.24 (d, 1H), 7.43 (s, 1H), 7.40-7.33 (m, 3H), 7.27 (m, 2H), 7.13 (s, 2H), 7.02 (d, 1H), 6.97 (m, 2H), 6.61 (d, 1H), 5.41 (m, 1H), 5.20 - 5.06 (m, 1H), 4.33 (s, 2H), 3.98 (t, 2H), 3.44 (m, 2H), 3.27 - 3.14 (m, 4H), 3.02 - 2.80 (m, 2H), 2.57-2.42 (m, 1H), 2.42-2.28 (m, 1H), 2.27 - 2.14 (m, 2H), 2.02 - 1.87 (m, 3H), 1.88 - 1.71 (m, 3H).
¹⁹F NMR (376 MHz, CD₃OD) δ -75.40.
LCMS m/z=386.1[(M+2)/2].

### Example 9: Isomer 1 of Compound 9

### [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-cyclopentyl-2-hydroxy-2-(2-thienyl)acetate; ditrifluoroacetic acid (Compound 9)

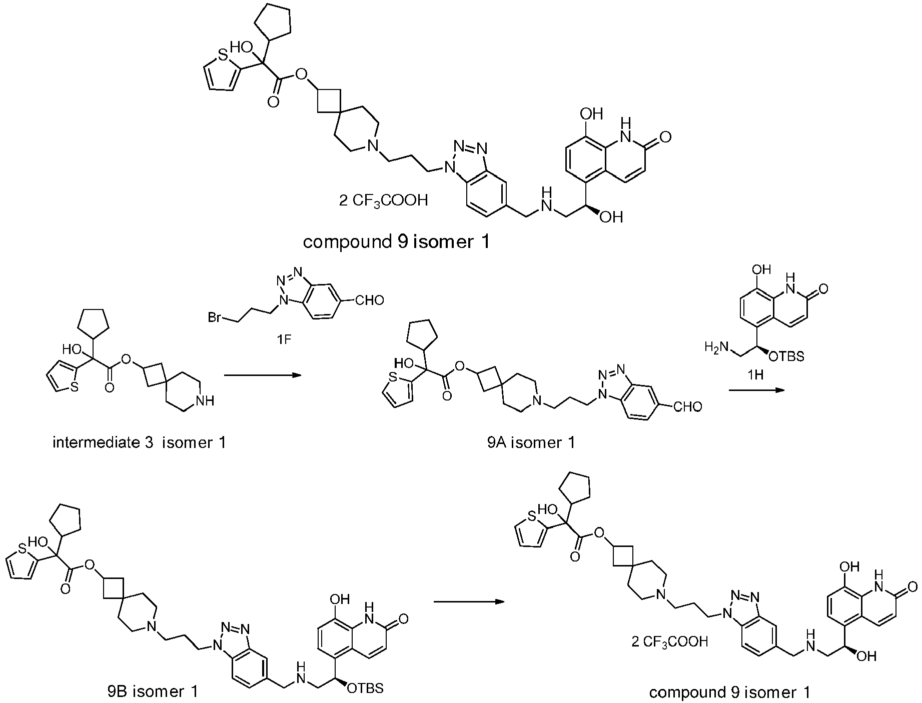

### Step 1: Isomer 1 of 9A [7-[3-(5-formylbenzotriazol-1-yl)propyl]-7-azaspiro[3.5]nonan-2-yl] 2-cyclopentyl-2-hydroxy-2-(2-thienyl)acetate (9A)

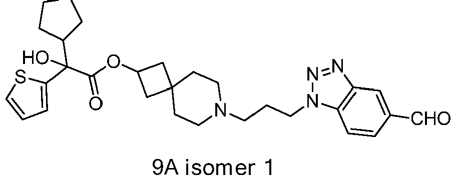

Isomer 1 of 7-azaspiro[3.5]nonan-2-yl 2-cyclopentyl-2-hydroxy-2-(2-thienyl)acetate (Intermediate 3) (0.600 g, 1.72 mmol) was dissolved in acetonitrile (20 ml), and 1-(3-bromopropyl)benzotriazole-5-carbaldehyde (1F) (0.506 g, 1.89 mmol) and diisopropylethylamine (0.311g, 2.40 mmol) were added, followed by reaction at 85°C for 4 hours. The reaction solution was concentrated under reduced pressure and the residue was purified by silica gel column chromatography (ethyl acetate/petroleum ether (v/v) = 1:1 to 1:0), to obtain the title compound Isomer 1 of [7-[3-(5-formylbenzotriazol-1-yl)propyl]-7-azaspiro[3.5]nonan-2-yl] 2-cyclopentyl-2-hydroxy-2-(2-thienyl)acetate (9A) as a yellow solid (0.800 g, yield: 86.8%).
¹H NMR (400 MHz, CDCl₃) δ 10.14 (s, 1H), 8.55 (s, 1H), 8.08 (m, 1H), 7.77 (d, 1H), 7.21 (m, 1H), 7.10 (m, 1H), 6.96 (m, 1H), 5.11 - 4.94 (m, 1H), 4.81 (t, 2H), 3.77 - 3.63 (m, 1H), 2.77 (m, 1H), 2.64 (s, 4H), 2.47 (s, 2H), 2.43 - 2.35 (m, 1H), 2.35 - 2.26 (m, 1H), 1.91 (m, 1H), 1.87-1.74 (m, 4H), 1.57 - 1.41 (m, 10H).
LCMS m/z=537.3[M+1].

### Step 2: Isomer 1 of 9B [7-[3-[5-[[[(2R)-2-[tert-butyl(dimethyl)silyl]oxy-2-(8-hydroxy-2-oxo-1H-quinolin-5-y l)ethyl]amino]methyl]benzotriazol-1-yl]propyl]-7-azaspiro[3.5]nonan-2-yl] 2-cyclopentyl-2-hydroxy-2-(2-thienyl)acetate (9B)

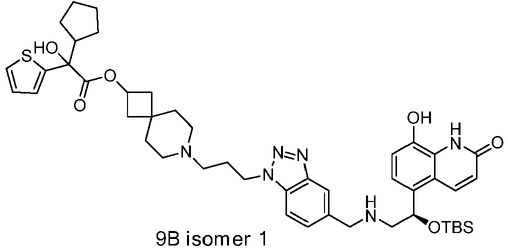

Isomer 1 of [7-[3-(5-formylbenzotriazol-1-yl)propyl]-7-azaspiro[3.5]nonan-2-yl] 2-cyclopentyl-2-hydroxy-2-(2-thienyl)acetate (9A) (0.800 g, 1.49 mmol) was dissolved in methanol (10 ml), to which 5-[(1R)-2-amino-1-[t-butyl(dimethyl)silyl]oxymethyl]-8-hydroxy-1H-quinolin-2-one (1H) (0.499 g, 1.49 mmol) and anhydrous zinc chloride (0.813 g, 5.96 mmol) were added, followed by reaction at 55°C for 1 h. Sodium cyanoborohydride (0.281 g, 4.47 mmol) was added, followed by reaction at 55°C for 2 h. The reaction solution was extracted after addition of methylene chloride (50 ml) and a saturated solution of sodium bicarbonate (20 ml). The aqueous phase was extracted with methylene chloride (30 mL×2). The organic phases were combined, dried over anhydrous sodium sulfate, filtered, and the filtrate was concentrated under reduced pressure, to obtain the title compound Isomer 1 of [7-[3-[5-[[[(2R)-2-[tert-butyl(dimethyl)silyl]oxy-2-(8-hydroxy-2-oxo-1H-quinolin-5-y l)ethyl]amino]methyl]benzotriazol-1-yl]propyl]-7-azaspiro[3.5]nonan-2-yl] 2-cyclopentyl-2-hydroxy-2-(2-thienyl)acetate (9B) as a yellow solid (1.00 g, yield 78.5%).
LCMS m/z=428.3[(M+2)/2].

### Step 3: Isomer 1 of Compound 9 [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-cyclopentyl-2-hydroxy-2-(2-thienyl)acetate; ditrifluoroacetic acid (Compound 9)

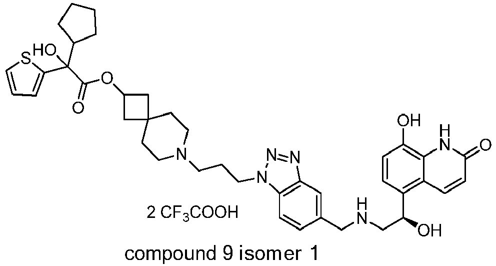

Isomer 1 of [7-[3-[5-[[[(2R)-2-[tert-butyl(dimethyl)silyl]oxy-2-(8-hydroxy-2-oxo-1H-quinolin-5-y l)ethyl]amino]methyl]benzotriazol-1-yl]propyl]-7-azaspiro[3.5]nonan-2-yl] 2-cyclopentyl-2-hydroxy-2-(2-thienyl)acetate (9B) (1.00 g, 1.17 mmol) was dissolved in tetrahydrofuran (5 ml), and triethylamine trihydrofluoride (1.9 g, 12 mmol) was added thereto, followed by reaction at room temperature for 24 hours. A 10% (v/v) methanol/methylene chloride solution (50 ml) was added, followed by addition of a saturated solution of sodium bicarbonate to adjust the pH to about 8, and then the reaction solution was partitioned. The aqueous phase was extracted with a 10% (v/v) methanol/methylene chloride solution (50 mL×2). The organic phases were combined. The organic phase was washed with saturated brine (20 mL×1), dried over anhydrous sodium sulfate, filtered, and the filtrate was concentrated under reduced pressure. The residue was purified by preparative liquid column chromatography (preparation condition: C18 reverse preparative column; Mobile phase: 0.05% TFA in deionized water (A) and 0.05% TFA in acetonitrile (B); Gradient elution with A:B=5%-100% for 10 min, followed by elution with 100% B for 5 min; Flow rate: 1.0 ml/min; Column temperature: 40°C) to obtain the title compound Isomer 1 of [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-cyclopentyl-2-hydroxy-2-(2-thienyl)acetate; ditrifluoroacetic acid (Compound 9) as a white solid (0.450 g, yield 39.7%).
¹H NMR (400 MHz, CD₃OD) δ 8.21 (d, 2H), 7.89 (d, 1H), 7.70 (m, 1H), 7.33 - 7.22 (m, 2H), 7.12 (d, 1H), 7.01 (d, 1H), 6.95 (dd, 1H), 6.55 (d, 1H), 5.43 (m, 1H), 5.10 - 4.98 (m, 1H), 4.87-4.83 (m, 2H), 4.57 - 4.42 (m, 2H), 3.53-3.39(m, 2H), 3.29 - 3.21 (m, 4H), 3.02 - 2.77 (m, 3H), 2.57 - 2.27 (m, 4H), 2.05 - 1.74 (m, 6H), 1.68 - 1.43 (m, 8H).
¹⁹F NMR (376 MHz, CD₃OD) δ -74.98.
LCMS m/z=371.3[(M+2)/2].

### Example 10: Isomer 2 of Compound 9

### [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-cyclopentyl-2-hydroxy-2-(2-thienyl)acetate; ditrifluoroacetic acid (Compound 9)

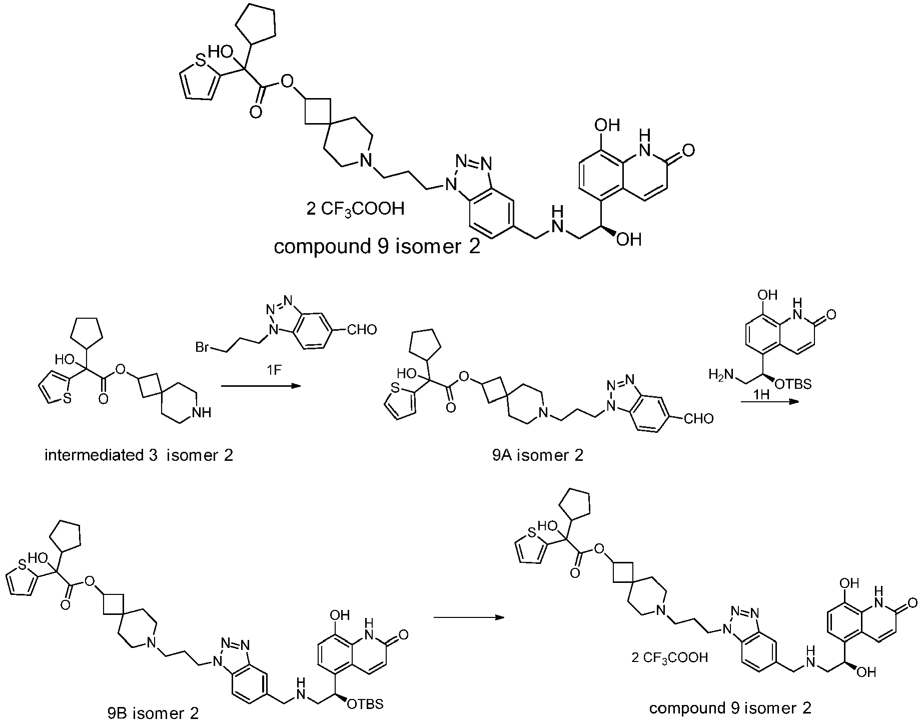

### Step 1: Isomer 2 of 9A [7-[3-(5-formylbenzotriazol-1-yl)propyl]-7-azaspiro[3,5]nonan-2-yl] 2-cyclopentyl-2-hydroxy-2-(2-thienyl)acetate (9A)

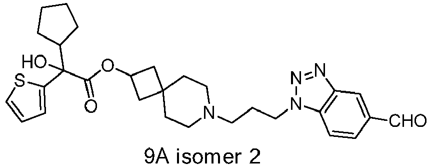

Isomer 2 of 7-azaspiro[3.5]nonan-2-yl 2-cyclopentyl-2-hydroxy-2-(2-thienyl)acetate (Intermediate 3) (0.600 g, 1.72 mmol) was dissolved in acetonitrile (20 ml), and 1-(3-bromopropyl)benzotriazole-5-carbaldehyde (0.506 g, 1.89 mmol) and diisopropylethylamine (0.311g, 2.40 mmol) were added, followed by reaction at 85°C for 4 hours. The reaction solution was concentrated and the residue was purified by silica gel column chromatography (ethyl acetate/petroleum ether (v/v) = 1:1 to 1:0), to obtain the title compound Isomer 2 of [7-[3-(5-formylbenzotriazol-1-yl)propyl]-7-azaspiro[3.5]nonan-2-yl] 2-cyclopentyl-2-hydroxy-2-(2-thienyl)acetate (9A) as a yellow solid (0.800 g, yield: 86.7%).
¹H NMR (400 MHz, CDCl₃) δ 10.14 (s, 1H), 8.55 (s, 1H), 8.08 (m, 1H), 7.79 (d, 1H), 7.21 (m, 1H), 7.10 (m, 1H), 6.96 (m, 1H), 5.11-4.98 (m, 1H), 4.81 (t, 2H), 3.78-3.66 (m, 1H), 2.83 - 2.72 (m, 1H), 2.66 (s, 4H), 2.53 - 2.43 (m, 2H), 2.43 - 2.34 (m, 1H), 2.34 - 2.24 (m, 1H), 1.91 (m, 1H), 1.84-1.73 (m, 4H), 1.56 - 1.44 (m, 10H).
LCMS m/z=537.3[M+1].

### Step 2: Isomer 2 of 9B [7-[3-[5-[[[(2R)-2-[tert-butyl(dimethyl)silyl]oxy-2-(8-hydroxy-2-oxo-1H-quinolin-5-y l)ethyl]amino]methyl]benzotriazol-1-yl]propyl]-7-azaspiro[3.5]nonan-2-yl] 2-cyclopentyl-2-hydroxy-2-(2-thienyl)acetate (9B)

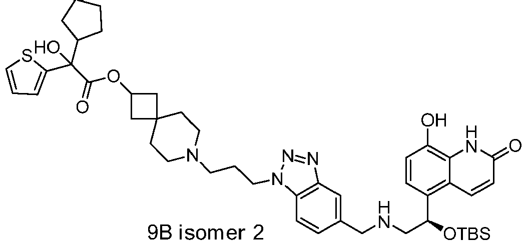

Isomer 2 of [7-[3-(5-formylbenzotriazol-1-yl)propyl]-7-azaspiro[3.5]nonan-2-yl] 2-cyclopentyl-2-hydroxy-2-(2-thienyl)acetate (9A) (0.800 g, 1.49 mmol) was dissolved in methanol (10 ml), to which 5-[(1R)-2-amino-1-[t-butyl(dimethyl)silyl]oxymethyl]-8-hydroxy-1H-quinolin-2-one (1H) (0.499 g, 1.49 mmol) and anhydrous zinc chloride (0.813 g, 5.96 mmol) were added, followed by reaction at 55°C for 1 h. Sodium cyanoborohydride (0.281 g, 4.47 mmol) was added, followed by reaction at 55°C for 2 h. The reaction solution was extracted after addition of methylene chloride (50 ml) and a saturated solution of sodium bicarbonate (20 ml). The aqueous phase was extracted with methylene chloride (30 mL×2). The organic phases were combined, dried over anhydrous sodium sulfate, filtered, and the filtrate was concentrated under reduced pressure, to obtain the title compound Isomer 2 of [7-[3-[5-[[[(2R)-2-[tert-butyl(dimethyl)silyl]oxy-2-(8-hydroxy-2-oxo-1H-quinolin-5-y 1)ethyl]amino]methyl]benzotriazol-1-yl]propyl]-7-azaspiro[3.5]nonan-2-yl] 2-cyclopentyl-2-hydroxy-2-(2-thienyl)acetate (9B) as a yellow solid (1.00g, yield 78.5%).
LCMS m/z=428.4[(M+2)/2].

### Step 3: Isomer 2 of Compound 9 [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-cyclopentyl-2-hydroxy-2-(2-thienyl)acetate; ditrifluoroacetic acid (Compound 9)

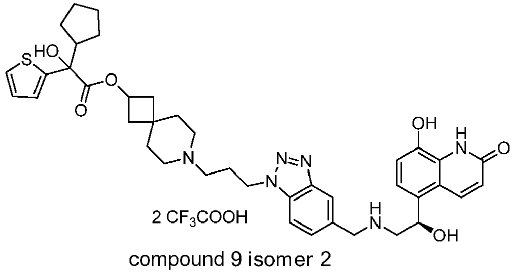

Isomer 2 of [7-[3-[5-[[[(2R)-2-[tert-butyl(dimethyl)silyl]oxy-2-(8-hydroxy-2-oxo-1H-quinolin-5-y l)ethyl]amino]methyl]benzotriazol-1-yl]propyl]-7-azaspiro[3.5]nonan-2-yl] 2-cyclopentyl-2-hydroxy-2-(2-thienyl)acetate (9B) (1.0 g, 1.17 mmol) was dissolved in tetrahydrofuran (5 ml), and triethylamine trihydrofluoride (1.89 g, 11.7 mmol) was added thereto, followed by reaction at room temperature for 24 hours. A 10% (v/v) methanol/methylene chloride solution (50 ml) was added, followed by addition of a saturated solution of sodium bicarbonate to adjust the pH to about 8, and then the reaction solution was partitioned. The aqueous phase was extracted with a 10% (v/v) methanol/methylene chloride solution (50 mL×2). The organic phases were combined. The organic phase was washed with saturated brine (20 mL×1), dried over anhydrous sodium sulfate, filtered, and the filtrate was concentrated under reduced pressure. The residue was purified by preparative liquid column chromatography (preparation condition: C18 reverse preparative column; Mobile phase: 0.05% TFA in deionized water (A) and 0.05% TFA in acetonitrile (B); Gradient elution with A:B=5%-100% for 10 min, followed by elution with 100% B for 5 min; Flow rate: 1.0 ml/min; Column temperature: 40°C) to obtain the title compound Isomer 2 of [7-[3-[5-[[[(2R)-2-hydroxy-2-(8-hydroxy-2-oxo-1H-quinolin-5-yl)ethyl]amino]methyl ]benzotriazol-1-yl]propyl]-7-azaspiro[3.5]nonan-2-yl] 2-cyclopentyl-2-hydroxy-2-(2-thienyl)acetate; ditrifluoroacetic acid (Compound 9) as a white solid (0.500g, yield 44.1%).
¹H NMR (400 MHz, CD₃OD) δ 8.27 - 8.16 (m, 2H), 7.89 (d, 1H), 7.71 (m, 1H), 7.32 - 7.22 (m, 2H), 7.12 (d, 1H), 7.01 (d, 1H), 6.95 (dd, 1H), 6.55 (d, 1H), 5.44 (m, 1H), 5.09 - 5.00 (m, 1H), 4.88-4.83 (m, 2H), 4.55 - 4.45 (m, 2H), 3.53-3.38 (d, 2H), 3.29 - 3.18 (m, 4H), 3.02 - 2.78 (m, 3H), 2.58 - 2.24 (m, 4H), 2.04 - 1.76 (m, 6H), 1.70 - 1.38 (m, 8H).
¹⁹F NMR (376 MHz, CD₃OD) δ -74.88.
LCMS m/z=371.3[(M+2)/2].

### Example 11:

### [7-[3-[3-[5-[[[(2R)-2-hydroxy-2-(8-hydroxy-2-oxo-1H-quinolin-5-yl)ethyl]amino]met hyl]benzotriazol-1-yl]propyl-methyl-amino]-3-oxo-propyl]-7-azaspiro[3.5]nonan-2-yl] 2-hydroxy-2,2-bis(2-thienyl)acetate;ditrifluoroacetic acid (Compound 11)

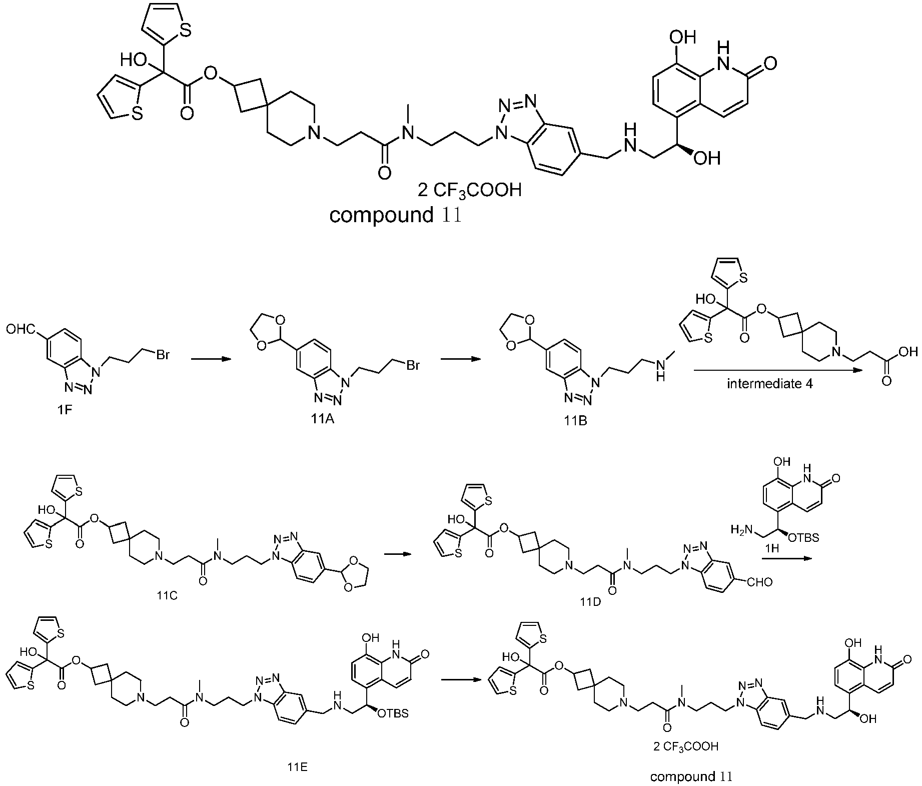

### Step 1: 1-(3-bromopropyl)-5-(1,3-dioxolan-2-yl)benzotriazole (11A)

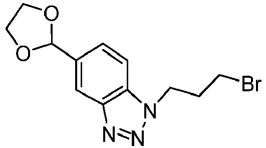

1-(3-bromopropyl)benzotriazole-5-carbaldehyde (**1F**) (1.5 g, 5.6 mmol) was dissolved in toluene (40 ml), to which ethylene glycol (0.38 g, 6.2 mmol) and p-toluene sulfonic acid (0.011 g, 0.056 mmol) were added, followed by reaction at 120°C for 3 hours. The reaction solution was extracted after addition of ethyl acetate (30 ml) and water (30 ml). The aqueous phase was extracted with ethyl acetate (30 mL×1). The organic phases were combined, dried over anhydrous sodium sulfate, filtered, and the filtrate was concentrated under reduced pressure. The residue was purified by silica gel column chromatography (ethyl acetate : petroleum ether (v/v) = 0:1 to 1:4) to give the colorless oily title compound 1-(3-bromopropyl)-5-(1,3-dioxolan-2-yl)benzotriazole (11A) (1.2 g, yield: 69%).
¹H NMR (400 MHz, CDCl₃) δ 8.17 (s, 1H), 7.69-7.60 (m, 2H), 5.97 (s, 1H), 4.82 (t, 2H), 4.21 - 4.04 (m, 4H), 3.37 (t, 2H), 2.62 - 2.53 (m, 2H).
LCMS m/z=312.1[M+1].

### Step 2: 3-[5-(1,3-dioxolan-2-yl)benzotriazol-1-yl]-N-methyl-propan-1-amine (11B)

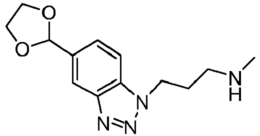

1-(3-bromopropyl)-5-(1,3-dioxolan-2-yl)benzotriazole (11A) (1.2 g, 3.8 mmol) was added into a 50-ml single-neck flask, to which a solution of 2N methylamine in ethanol (15 ml) was added, and the flask was sealed to carry out a reaction at room temperature for 12 hours. The reaction solution was extracted after addition of methylene chloride (30 ml) and saturated brine (20 ml). The organic phase was dried over anhydrous sodium sulfate, filtered, and the filtrate was concentrated under reduced pressure, to obtain the yellow oily title compound 3-[5-(1,3-dioxolan-2-yl)benzotriazol-1-yl]-N-methyl-propan-1-amine (11B) (1.0 g, yield 99%).
¹H NMR (400 MHz, CDCl₃) δ 8.16 (s, 1H), 7.65-7.56 (m, 2H), 5.97 (s, 1H), 4.75 (t, 2H), 4.23 - 4.02 (m, 4H), 2.58 (t, 2H), 2.41 (s, 3H), 2.24 - 2.10 (m, 2H).
LCMS m/z=263.2[M+1].

### Step 3: [7-[3-[3-[5-(1,3-dioxolan-2-yl)benzotriazol-1-yl]propyl-methyl-amino]-3-oxo-propyl] -7-azaspiro[3.5]nonan-2-yl] 2-hydroxy-2,2-bis(2-thienyl)acetate (11C)

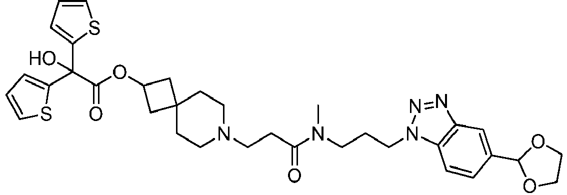

3-[5-(1,3-dioxolan-2-yl)benzotriazol-1-yl]-N-methyl-propan-1-amine (11B) (0.397 g, 1.52 mmol) was dissolved in methylene chloride (40 ml), to which 3-[2-[2-hydroxy-2,2-bis(2-thienyl)acetyl]oxy-7-azaspiro[3.5]nonan-7-yl]propanoic acid (Intermediate 4) (0.660 g, 1.52 mmol), HATU (2-(7-azobenzotriazole)-N,N,N',N'-tetramethylureahexafluorophosphate) (0.864 g, 2.27 mmol) and diisopropylethylamine (1.57 g, 12.1 mmol) were added, followed by reaction at room temperature for 3 hours. The reaction solution was extracted after addition of water (50 ml) and methylene chloride (50 ml). The organic phase was dried over anhydrous sodium sulfate, filtered, and the filtrate was concentrated under reduced pressure. The residue was purified by silica gel column chromatography (methanol:methylene chloride (v/v) = 1:99 to 1:19), to obtain the yellow oily title compound [7-[3-[3-[5-(1,3-dioxolan-2-yl)benzotriazol-1-yl]propyl-methyl-amino]-3-oxo-propyl] -7-azaspiro[3.5]nonan-2-yl] 2-hydroxy-2,2-bis(2-thienyl)acetate (11C) (0.900 g, yield: 87.4%).
¹H NMR (400 MHz, CDCl₃) δ 8.15 (t, 1H), 7.70 - 7.60 (m, 1H), 7.59 - 7.51 (m, 1H), 7.31 - 7.27 (m, 2H), 7.21-7.13 (m, 2H), 7.02-6.93 (m, 2H), 5.96 (s, 1H), 5.18 - 5.07 (m, 1H), 4.70-4.58(m, 2H), 4.20 - 4.13 (m, 2H), 4.13 - 4.04 (m, 2H), 3.54-3.42(m, 2H), 3.04 - 2.88 (m, 3H), 2.77 - 2.66 (m, 2H), 2.55 - 2.22 (m, 10H), 1.92-1.80 (m, 2H), 1.70 - 1.53 (m, 4H).
LCMS m/z=680.3[M+1].

### Step 4: [7-[3-[3-(5-formylbenzotriazol-1-yl)propyl-methyl-amino]-3-oxo-propyl]-7-azaspiro[ 3.5]nonan-2-yl] 2-hydroxy-2,2-bis(2-thienyl)acetate (11D)

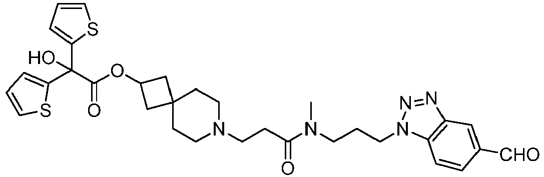

[7-[3-[3-[5-(1,3-dioxolan-2-yl)benzotriazol-1-yl]propyl-methyl-amino]-3-oxo-propyl] -7-azaspiro[3.5]nonan-2-yl] 2-hydroxy-2,2-bis(2-thienyl)acetate (11C) (0.100 g, 0.147 mmol) was dissolved in acetone (4 ml) and water (1 ml), to which p-toluene sulfonic acid (0.280 g, 0.147 mmol) was added, followed by reaction at 70°C for 3 hours. The reaction solution was extracted after addition of methylene chloride (20 ml) and a saturated solution of sodium bicarbonate (10 ml). The organic phase was dried over anhydrous sodium sulfate, filtered, and the filtrate was concentrated under reduced pressure, to obtain the yellow oily title compound [7-[3-[3-(5-formylbenzotriazol-1-yl)propyl-methyl-amino]-3-oxo-propyl]-7-azaspiro[ 3.5]nonan-2-yl] 2-hydroxy-2,2-bis(2-thienyl)acetate (11D) (0.065 g, yield 69%).
LCMS m/z=636.3[M+1].

### Step 5: [7-[3-[3-[5-[[[(2R)-2-[tert-butyl(dimethyl)silyl]oxy-2-(8-hydroxy-2-oxo-1H-quinolin-5-yl)ethyl]amino]methyl]benzotriazol-1-yl]propyl-methyl-amino]-3-oxo-propyl]-7-az aspiro[3.5]nonan-2-yl]2-hydroxy-2,2-bis(2-thienyl)acetate (11E)

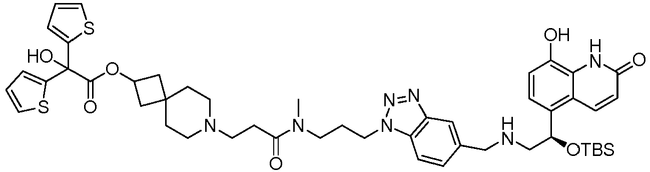

[7-[3-[3-(5-formylbenzotriazol-1-yl)propyl-methyl-amino]-3-oxo-propyl]-7-azaspiro[ 3.5]nonan-2-yl] 2-hydroxy-2,2-bis(2-thienyl)acetate (11D) (0.665 g, 1.05 mmol) was dissolved in methanol (10 ml), to which 5-[(1R)-2-amino-1-[t-butyl(dimethyl)silyl]oxymethyl]-8-hydroxy-1H-quinolin-2-one (1H) (0.445 g, 1.36 mmol) and anhydrous zinc chloride (0.570 g, 4.18 mmol) were added, followed by reaction at 55°C for 1 h. Sodium cyanoborohydride (0.197 g, 3.14 mmol) was added, followed by reaction at 55°C for 2 h. The reaction solution was extracted after addition of methylene chloride (50 ml) and a saturated solution of sodium bicarbonate (20 ml). The aqueous phase was extracted with methylene chloride (30 mL×2). The organic phases were combined, dried over anhydrous sodium sulfate, filtered, and the filtrate was concentrated under reduced pressure, to obtain the title compound [7-[3-[3-[5-[[[(2R)-2-[tert-butyl(dimethyl)silyl]oxy-2-(8-hydroxy-2-oxo-1H-quinolin-5-yl)ethyl]amino]methyl]benzotriazol-1-yl]propyl-methyl-amino]-3-oxo-propyl]-7-az aspiro[3.5]nonan-2-yl] 2-hydroxy-2,2-bis(2-thienyl)acetate (11E) as a yellow solid (0.60g, yield 60.1%).
LCMS m/z=477.8[(M+2)/2].

### Step 6: [7-[3-[3-[5-[[[(2R)-2-hydroxy-2-(8-hydroxy-2-oxo-1H-quinolin-5-yl)ethyl]amino]met hyl]benzotriazol-1-yl]propyl-methyl-amino]-3-oxo-propyl]-7-azaspiro[3.5]nonan-2-yl] 2-hydroxy-2,2-bis(2-thienyl)acetate;ditrifluoroacetic acid (Compound 11)

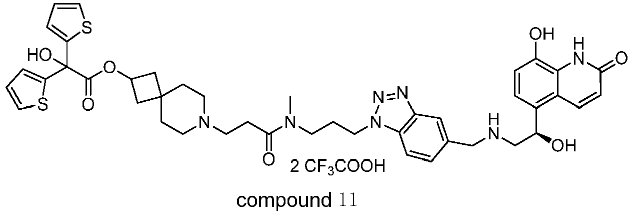

[7-[3-[3-[5-[[[(2R)-2-[tert-butyl(dimethyl)silyl]oxy-2-(8-hydroxy-2-oxo-1H-quinolin-5-yl)ethyl]amino]methyl]benzotriazol-1-yl]propyl-methyl-amino]-3-oxo-propyl]-7-az aspiro[3.5]nonan-2-yl] 2-hydroxy-2,2-bis(2-thienyl)acetate (11E) (0.600 g, 0.629 mmol) was dissolved in tetrahydrofuran (5 ml), and triethylamine trihydrofluoride (1.01 g, 6.29 mmol) was added thereto, followed by reaction at room temperature for 24 hours. A 10% (v/v) methanol/methylene chloride solution (50 ml) was added, followed by addition of a saturated solution of sodium bicarbonate to adjust the pH to about 8, and then the reaction solution was partitioned. The aqueous phase was extracted with a 10% (v/v) methanol/methylene chloride solution (50 mL×2). The organic phases were combined. The organic phase was washed with saturated brine (20 mL×1), dried over anhydrous sodium sulfate, filtered, and the filtrate was concentrated under reduced pressure. The residue was purified by preparative liquid column chromatography (preparation condition: C18 reverse preparative column; Mobile phase: 0.05% TFA in deionized water (A) and 0.05% TFA in acetonitrile (B); Gradient elution with A:B=5%-100% for 10 min, followed by elution with 100% B for 5 min; Flow rate: 1.0 ml/min; Column temperature: 40°C) to obtain the title compound [7-[3-[3-[5-[[[(2R)-2-hydroxy-2-(8-hydroxy-2-oxo-1H-quinolin-5-yl)ethyl]amino]met hyl]benzotriazol-1-yl]propyl-methyl-amino]-3-oxo-propyl]-7-azaspiro[3.5]nonan-2-yl] 2-hydroxy-2,2-bis(2-thienyl)acetate; ditrifluoroacetic acid (Compound 11) as a white solid (0.200g, yield 29.8%).
¹H NMR (400 MHz, CD₃OD) δ 8.28-8.19 (m, 2H), 7.96-7.88 (m, 1H), 7.78-7.68 (m, 1H), 7.39 (dd, 2H), 7.29 (d, 1H), 7.15 (s, 2H), 7.07 - 6.95 (m, 3H), 6.63-6.53(m, 1H), 5.49-5.41 (m, 1H), 5.20-5.11 (m, 1H), 4.83-4.75 (m, 2H), 4.53 (s, 2H), 3.58 - 3.35 (m, 6H), 3.31 - 3.27 (m, 2H), 3.07 - 3.00 (m, 3H), 3.00 - 2.92 (m, 2H), 2.91 - 2.86 (m, 2H), 2.60 - 2.27 (m, 4H), 2.04-1.94 (m, 3H), 1.91 - 1.75 (m, 3H).
¹⁹F NMR (376 MHz, CD₃OD) δ -75.47.
LCMS m/z=420.8[(M+2)/2].

### Example 12:

### [7-[3-[5-[[[(2R)-2-hydroxy-2-(4-hydroxy-2-oxo-3H-1,3-benzothiazol-7-yl)ethyl]amin o]methyl]benzotriazol-1-yl]propyl]-7-azaspiro[3.5]nonan-2-yl] 2-hydroxy-2,2-bis(2-thienyl)acetate; ditrifluoroacetic acid (Compound 12)

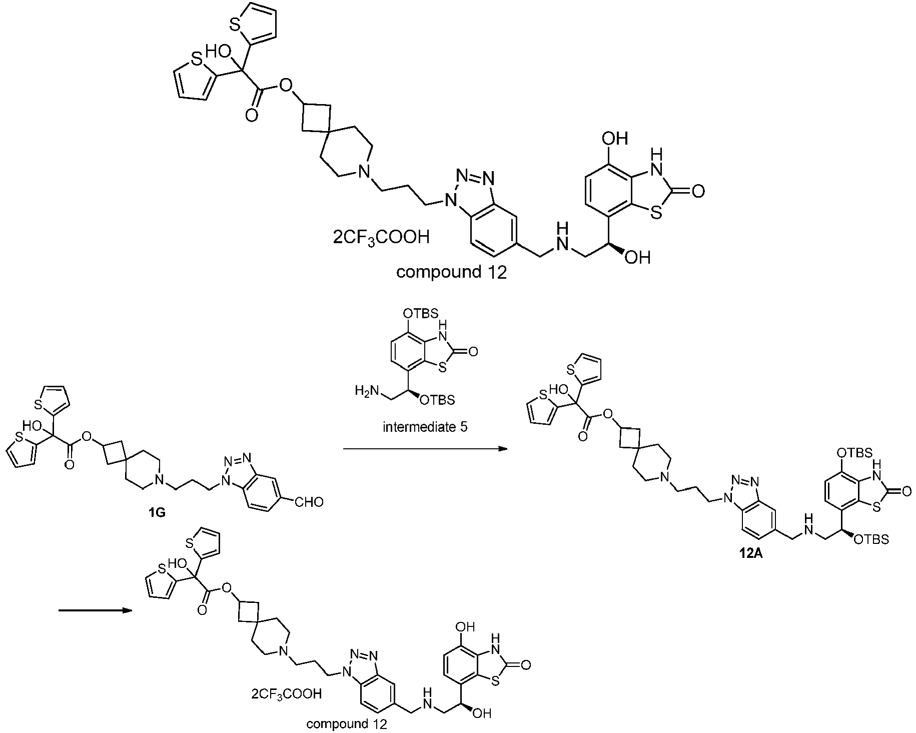

### Step 1: [7-[3-[5-[[[(2R)-2-[tert-butyl(dimethyl)silyl]oxy-2-[4-[tert-butyl(dimethyl)silyl]oxy-2 -oxo-3H-1,3-benzothiazol-7-yl]ethyl]amino]methyl]benzotriazol-1-yl]propyl]-7-azasp iro[3.5]nonan-2-yl] 2-hydroxy-2,2-bis(2-thienyl)acetate (12A)

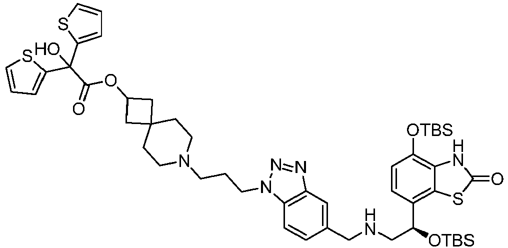

[7-[3-(5-formylbenzotriazol-1-yl)propyl]-7-azaspiro[3 .5]nonan-2-yl] 2-hydroxy-2,2-bis(2- thienyl)acetate (1G) (0.55 g, 1.0 mmol) and 7-[(1R)-2-amino-1-[tert-butyl(dimethyl)silyl]oxy-ethyl]-4-[tert-butyl(dimethyl)silyl]o xy-3H-1,3-benzothiazol-2-one (Intermediate 5) (0.55 g, 1.2 mmol) were dissolved in methanol (10 ml), zinc chloride (0.54 g, 4.0 mmol) was added thereto, the temperature was elevated to 55°C to carry out a reaction for 1 hour, and then sodium cyanoborohydride (0.188 g, 3.0 mmol) was added, followed by further reaction for 2 hours. After the reaction was complete, water (20 ml) was added to the reaction solution, which was then extracted with methylene chloride (50 mL×2), washed with saturated brine (30 mL×1), dried over anhydrous sodium sulfate, and concentrated under reduced pressure. The residue was purified by silica gel column chromatography (with methylene chloride/methanol (v/v) = 100/1) to give the title compound [7-[3-[5-[[[(2R)-2-[tert-butyl(dimethyl)silyl]oxy-2-[4-[tert-butyl(dimethyl)silyl]oxy-2 -oxo-3H-1,3-benzothiazol-7-yl]ethyl]amino]methyl]benzotriazol-1-yl]propyl]-7-azasp iro[3.5]nonan-2-yl] 2-hydroxy-2,2-bis(2-thienyl)acetate (12A) as a yellow solid (0.43 g, yield 44%).
LCMS m/z = 989.4[M+1].

### Step 2: [7-[3-[5-[[[(2R)-2-hydroxy-2-(4-hydroxy-2-oxo-3H-1,3-benzothiazol-7-yl)ethyl]amin o]methyl]benzotriazol-1-yl]propyl]-7-azaspiro[3.5]nonan-2-yl] 2-hydroxy-2,2-bis(-2-thienyl)acetate; ditrifluoroacetic acid (Compound 12)

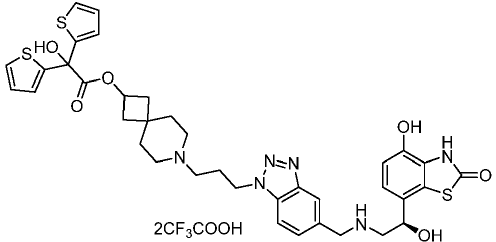

[7-[3-[5-[[[(2R)-2-[tert-butyl(dimethyl)silyl]oxy-2-[4-[tert-butyl(dimethyl)silyl]oxy-2 -oxo-3H-1,3-benzothiazol-7-yl]ethyl]amino]methyl]benzotriazol-1-yl]propyl]-7-azasp iro[3.5]nonan-2-yl] 2-hydroxy-2,2-bis(2-thienyl)acetate (12A) (0.27 g, 0.27 mmol) was dissolved in tetrahydrofuran (5 ml), and triethylamine trihydrofluoride (0.18 g, 1.1 mmol) was added thereto, followed by reaction at room temperature overnight. The pH was adjusted to about 9 with a saturated aqueous solution of sodium bicarbonate. The resultant was extracted with methylene chloride (30 mL×1), and the organic phase was washed with a saturated sodium chloride solution (30 mL×1), dried over anhydrous sodium sulfate, and filtered, and the filtrate was concentrated under reduced pressure. The residue was purified by preparative liquid column chromatography (preparation condition: C18 reverse preparative column; Mobile phase: 0.05% TFA in deionized water (A) and acetonitrile (B); Isocratic elution with 25% acetonitrile for 20 min) to obtain the title compound [7-[3-[5-[[[(2R)-2-hydroxy-2-(4-hydroxy-2-oxo-3H-1,3-benzothiazol-7-yl)ethyl]amin o]methyl]benzotriazol-1-yl]propyl]-7azaspiro[3.5]nonan-2-yl] 2-hydroxy-2,2-bis(-2-thienyl)acetate; ditrifluoroacetic acid (Compound 12) as an off-white solid (0.09 g, yield 40%).
¹H NMR (400 MHz, CD₃OD) δ 8.23 (s, 1H), 7.95 (d, 1H), 7.75 (dd, 1H), 7.39 (dd, 2H), 7.19 - 7.12 (m, 2H), 7.06 - 6.93 (m, 3H), 6.77 (d, 1H), 5.20 - 5.09 (m, 1H), 5.01 (t, 1H), 4.88 (d, 2H), 4.57 - 4.44 (m, 2H), 3.56 - 3.37 (m, 2H), 3.29 - 3.21 (m, 2H), 3.19 (d, 2H), 2.94 (s, 2H), 2.57 - 2.46 (m, 3H), 2.37 (s, 1H), 1.95 (s, 3H), 1.80 (d, 3H).
LCMS m/z =761.3[M+1].

### Free base of Compound 12:

### [7-[3-[5-[[[(2R)-2-hydroxy-2-(4-hydroxy-2-oxo-3H-1,3-benzothiazol-7-yl)ethyl]amin o]methyl]benzotriazol-1-yl]propyl]-7-azaspiro[3.5]nonan-2-yl] 2-hydroxy-2,2-bis(2-thienyl)acetate

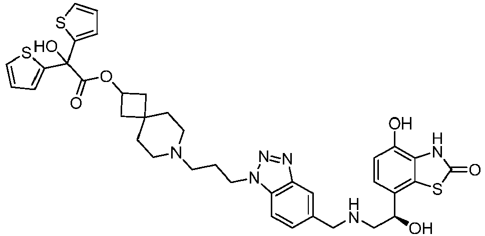

[7-[3-[5-[[[(2R)-2-[tert-butyl(dimethyl)silyl]oxy-2-[4-[tert-butyl(dimethyl)silyl]oxy-2 -oxo-3H-1,3-benzothiazol-7-yl]ethyl]amino]methyl]benzotriazol-1-yl]propyl]-7-azasp iro[3.5]nonan-2-yl] 2-hydroxy-2,2-bis(2-thienyl)acetate (12A) (30.0 g, 30.3 mmol) was dissolved in tetrahydrofuran (120 ml), and triethylamine trihydrofluoride (19.56 g, 0.12 mol) was added thereto, followed by reaction at room temperature overnight. The pH was adjusted to about 9 with a saturated aqueous solution of sodium bicarbonate. The resultant was extracted with methylene chloride:methanol (2:1) (300 mL×1). The organic phase was dried over anhydrous sodium sulfate, and filtered, and the filtrate was concentrated under reduced pressure. The residue was purified by silica gel column chromatography (with methylene chloride/methanol (v/v) = 100/1 to 7/1) to give the title compound [7-[3-[5-[[[(2R)-2-hydroxy-2-(4-hydroxy-2-oxo-3H-1,3-benzothiazol-7-yl)ethyl]amin o]methyl]benzotriazol-1-yl]propyl]-7-azaspiro[3.5]nonan-2-yl] 2-hydroxy-2,2-bis(2-thienyl)acetate (Free base of Compound 12) as a white solid (11.0 g, yield 47.7%).
¹H NMR (400 MHz, DMSO-d6) δ 7.88 (s, 1H), 7.75 (d, 1H), 7.50-7.48 (dd, 1H), 7.47-7.46 (dd, 2H), 7.09-7.07 (dd, 2H), 6.99-6.97 (dd, 2H), 6.86 (d, 1H), 6.68 (d, 1H), 5.09 - 4.89 (m, 1H), 4.75 - 4.50 (m, 3H), 3.86 (s, 2H), 2.71-2.58 (m, 2H), 2.27 - 1.93 (m, 10H), 1.73-1.68m (m, 2H), 1.48-1.42 (m, 4H).
LCMS m/z =761.3[M+1].

### Example 13:

### [7-[3-[5-[[[(2R)-2-hydroxy-2-[4-hydroxy-3-(hydroxymethyl)phenyl]ethyl]amino]met hyl]benzotriazol-1-yl]propyl]-7-azaspiro[3.5]nonan-2-yl] 2-hydroxy-2,2-bis(2-thienyl)acetate; ditrifluoroacetic acid (Compound 13)

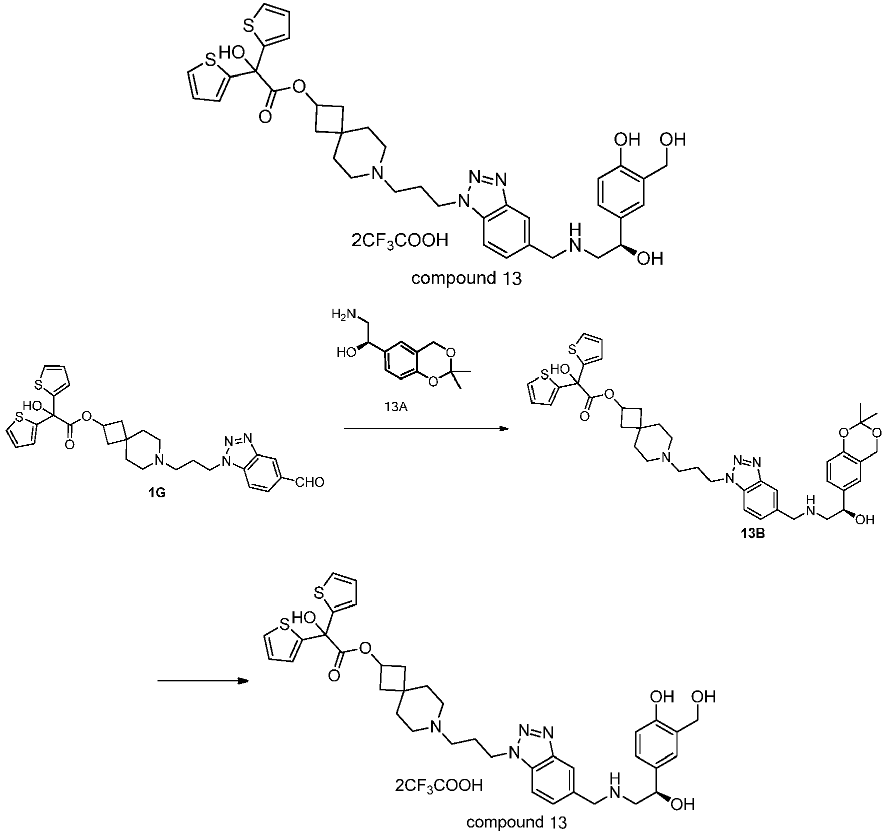

### Step 1: 7-[3-[5-[[[(2R)-2-(2,2-dimethyl-4H-1,3-benzodioxin-6-yl)-2-hydroxy-ethyl]amino]me thyl]benzotriazol-1-yl]propyl]-7-azaspiro[3.5]nonan-2-yl] 2-hydroxy-2,2-bis(2-thienyl)acetate (13B)

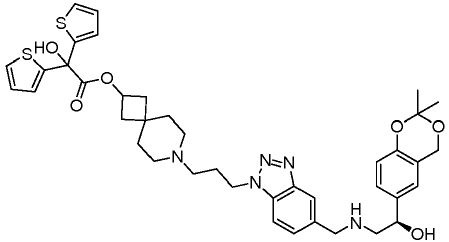

[7-[3-(5-formylbenzotriazol-1-yl)propyl]-7-azaspiro[3 .5]nonan-2-yl] 2-hydroxy-2,2-bis(2-thienyl)acetate (1G) (0.4 g, 0.726 mmol) and (1R)-2-amino-1-(2,2-dimethyl-4H-1,3-benzodioxin-6-yl)ethanol (**13A**) (0.195 g, 0.872 mmol) were added to a reaction flask, to which methanol (10 ml) and zinc chloride (0.396 g, 2.91 mmol) were added, followed by reaction at 55°C for 1 h. Sodium cyanoborohydride (0.137 g, 2.18 mmol) was added, followed by reaction at 55°C for 2 h. After the reaction was complete, water (20 ml) and methylene chloride (30 ml) were added to the reaction solution, the solution was partitioned, and the aqueous phase was extracted with methylene chloride (20 mL×1). The organic phases were combined. The organic phase was dried over anhydrous sodium sulfate, filtered, and the filtrate was concentrated under reduced pressure. The residue was purified by silica gel column chromatography (methanol/methylene chloride (v/v) = 1:49 to 1:19) to give the title compound 7-[3-[5-[[[(2R)-2-(2,2-dimethyl-4H-1,3-benzodioxin-6-yl)-2-hydroxy-ethyl]amino]me thyl]benzotriazol-1-yl]propyl]-7-azaspiro[3.5]nonan-2-yl] 2-hydroxy-2,2-bis(2-thienyl)acetate (13B) as a yellow solid (0.3 g, yield 54%).
LC-MS m/z =758.3[M+1].

### Step 2: [7-[3-[5-[[[(2R)-2-hydroxy-2-[4-hydroxy-3-(hydroxymethyl)phenyl]ethyl]amino]met hyl]benzotriazol-1-yl]propyl]-7-azaspiro[3.5]nonan-2-yl] 2-hydroxy-2,2-bis(2-thienyl)acetate; ditrifluoroacetic acid (Compound 13)

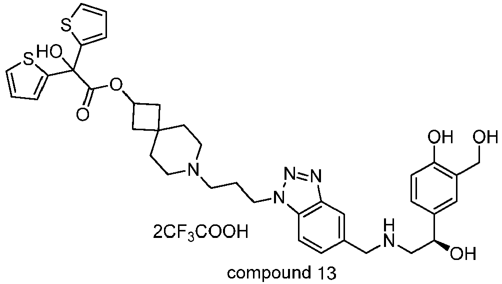

7-[3-[5-[[[(2R)-2-(2,2-dimethyl-4H-1,3-benzodioxin-6-yl)-2-hydroxy-ethyl]amino]me thyl]benzotriazol-1-yl]propyl]-7-azaspiro[3.5]nonan-2-yl] 2-hydroxy-2,2-bis(2-thienyl)acetate (13B) (0.3 g, 0.396 mmol) was dissolved in methylene chloride (15 ml), to which trifluoroacetic acid (0.0903 g, 0.792 mmol) was added dropwise, followed by reaction at room temperature for 2 hours. A saturated solution of sodium bicarbonate (15 ml) was added to the reaction solution, which was extracted with methylene chloride (15 mL×2). The organic phases were combined, dried over anhydrous sodium sulfate, filtered, and the filtrate was concentrated under reduced pressure. The residue was purified by preparative liquid column chromatography (preparation condition: C18 reverse preparative column; Mobile phase: 0.05% TFA in deionized water (A) and acetonitrile (B); Gradient elution with 20%-25% B for 32 min) to obtain the title compound [7-[3-[5-[[[(2R)-2-hydroxy-2-[4-hydroxy-3-(hydroxymethyl)phenyl]ethyl]amino]met hyl]benzotriazol-1-yl]propyl]-7-azaspiro[3.5]nonan-2-yl] 2-hydroxy-2,2-bis(2-thienyl)acetate; ditrifluoroacetic acid (Compound 13) as a white solid (0.2 g, yield 50%).
¹H NMR (400 MHz, CD₃OD) δ 8.23 (s, 1H), 7.94 (d, 1H), 7.75 (d, 1H), 7.39 (d, 2H), 7.33 (s, 1H), 7.16 (dd, 3H), 6.99 (dd, 2H), 6.80 (d, 1H), 5.20 - 5.09 (m, 1H), 4.98 - 4.91 (m, 1H), 4.88 (s, 2H), 4.66 (s, 2H), 4.50 (s, 2H), 3.45 (dd, 2H), 3.28 - 3.20 (m, 2H), 3.16 (d, 2H), 3.00 - 2.82 (m, 2H), 2.58 - 2.44 (m, 3H), 2.37 (s, 1H), 1.95 (d, 3H), 1.90 - 1.76 (m, 3H).
LCMS m/z =718.3[M+1].

### Biological tests

### Test Example 1: Inhibitory activity against human muscarine receptor M3

CHO cells (PerkinElmer, ES-212-AF) that stably express human muscarine receptor M3 (hM3) and apo-Aequorin were cultured at 37°C, 5% CO₂ in Ham's F12 media (Invitrogen 12500-062) containing 10% fetal bovine serum (FBS) (Gibico 10099-141), 400 µg/mL G418 (sigma G5013) and 250 µg/mL Zeocin (Invivogen ant-zn-5p), until a confluency of 90% to 100% was reached. Cells were separated by rinsing with PBS/5 mM EDTA, collected by centrifugation, re-suspended in phenol red-free Ham's F12 media (Invitrogen 12500-062) containing 0.1 BSA (BOVOGEN BSAS 100), and counted. The cell concentration was adjusted to 1×10⁶ cells/ml. 15 ml of the cell suspension was added to a 50-ml centrifuge tube, to which Coelenterazine-h (Promega S2011) was added to a final concentration of 5 µM. The tube was wrapped with a tin foil to shade light, and incubated on a rotary shaker at 20°C for 4 hours. The cells were diluted with a phenol red-free/0.1% BSA Ham's F12 medium to a final concentration of 5.0×10⁵ cells/ml, placed on a rotary shaker in low speed rotation, and incubated at room temperature for at least 1 hour. The compounds of the Examples were formulated with DMSO into 10 mM stock solutions, diluted in series with a phenol red-free/0.1% BSA Ham's F12 media (log(M): -7, -8, -9, -10, -11), and added to a 96-well plate at 50 µl/well. 50 µl cell suspension was further added to each well (25,000 cells/well) and incubated for 15 min at room temperature. The 96-well plate was placed in a microplate reader (Perkin Elmer, Envision), and a 50 µl solution of acetylcholine chloride (Sigma A6625) was added to each well at a concentration of 112.92 nM (hM3) with a microplate reader sample injector. The luminescence was recorded over 20 seconds, and IC₅₀ values were calculated and analyzed by Origin 7.5. The inhibitory activity of the compounds of the present invention against human muscarine receptor was measured by the above experiments, and the IC₅₀ values obtained are shown in Table 1 below.

**Table 1. Inhibitory activity of test compounds against human muscarine receptor M3.**

| Examples | IC₅₀ for hM3 receptor (nM) |
|---|---|
| Compound 1 | 0.43 |
| Compound 2 | 0.59 |
| Compound 3 | 0.22 |
| Compound 4 | 2.46 |
| Isomer 1 of Compound 4 | 2.36 |
| Isomer 2 of Compound 4 | 1.77 |
| Compound 7 | 1.29 |
| Compound 8 | 0.65 |
| Isomer 1 of Compound 9 | 2.47 |
| Isomer 2 of Compound 9 | 2.76 |
| Compound 11 | 0.46 |
| Compound 12 | 0.52 |
| Compound 13 | 0.31 |

Conclusion: the compounds of the present invention showed significant inhibitory activity against the human muscarine receptor M3.

### Test Example 2: Agonist activity on human β₂-adrenergic receptor

The agonist activity of the compounds of the Examples on the human adrenergic receptor was measured by the LANCE Ultra cAMP Assay.

CHO cells (PerkinElmer, ES-034-AF) that stably express human adrenergic receptor (hβ₂) were cultured at 37°C, 5% CO₂ in MEM-alpha media (Invitrogen 12561-056) containing 10% fetal bovine serum (FBS) (Gibico 10099-141) and 250 µg/ml Zeocin (InvivoGen ant-zn-5p), until a confluency of 90% to 100% was reached. Then the agonism on cAMP was tested by using a LANCE Ultra cAMP Assay kit (PerkinElmer TRF0263). Cells were separated with PBS/5 mM EDTA, collected by centrifugation, re-suspended in Stimulation Buffer (1 x HBSS, 5 mM HEPES, 0.5 mM IBMX, 0.1% BSA, pH7.4), and adjusted to a cell concentration of 6×10⁵ cells/ml. The compounds of the Examples were formulated with DMSO into 10 mM stock solutions, diluted in series with Stimulation Buffer, and added to a 384-well plate at 5 µl/well. 5 µl cell suspension was further added to each well (3,000 cells/well), incubated for 30 min at room temperature, then 5µl of a 4 x Eu-cAMP trace working solution was added to each well, and then 5 µl of a 4 x Ulight-anti-cAMP working solution was added to each well, followed by incubation at room temperature for 1 hour. The 384-well plate was detected for TR-FRET with a microplate reader (Perkin Elmer, Envision), and EC₅₀ values were calculated and analyzed by Origin 7.5. The agonist activity of the compounds of the present invention on the human adrenergic receptor was measured by the above experiments, and the IC₅₀ values obtained are shown in Table 2 below.

**Table 2. Agonist activity of test compounds on human β₂-adrenergic receptor**

| Example | EC₅₀ for hβ₂ receptor (nM) |
|---|---|
| Compound 1 | 4.45 |
| Compound 2 | 1.3 |
| Compound 7 | 4 |
| Compound 8 | 6.13 |
| Compound 12 | 0.47 |

Conclusion: the compounds of the present invention showed significant agonist activity on the β₂-adrenergic receptor.

## Claims

1. A compound represented by general formula (I), 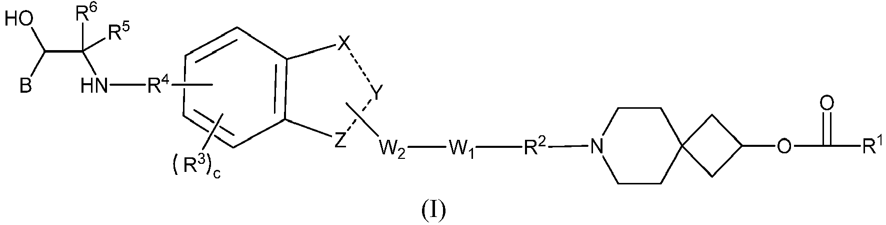 wherein
R¹ is selected from the group consisting of 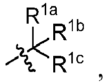 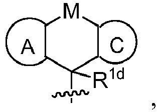 or 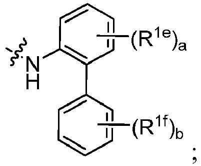
R^{1a} and R^{1b} are each independently selected from the group consisting of H, a C₂₋₆ alkenyl, a C₂₋₆ alkynyl, a C₃₋₁₀ carbocycle, a 3- to 8-membered heterocycle, a C₃₋₁₀ carbocyclyl-C₁₋₄ alkylene, or a 3- to 8-membered heterocyclyl-C₁₋₄ alkylene, wherein the alkenyl, alkynyl, alkylene, carbocycle or heterocycle is optionally further substituted with 0 to 4 substituents selected from the group consisting of F, Cl, Br, I, CH₂F, CHF₂, CF₃, OH, OCH₂F, OCHF₂, OCF₃, NH₂, carboxyl, cyano, nitro, a C₁₋₄ alkyl, a C₁₋₄ alkoxy, a -O(=O)C₁₋₄ alkyl, a -(=O)C₁₋₄ alkyl, a -OC₃₋₆ cycloalkyl or a C₁₋₄ alkylthio, wherein the heterocycle contains 1 to 3 hetero atoms selected from the group consisting of N, O or S; with the proviso that R^{1a} and R^{1b} are not both H;
R^{1c} is selected from the group consisting of H, hydroxy, cyano, NH₂, a C₁₋₆ alkyl, a C₁₋₆ alkoxy, a C₁₋₆ alkylthio or -C(=O)NH₂, wherein the NH₂, -C(=O)NH₂, or alkyl is optionally further substituted with 0 to 2 substituents selected from the group consisting of F, Cl, Br, I, hydroxy, cyano or a C₁₋₄ alkyl;
M is selected from the group consisting of a bond, -CH₂-, -CH₂CH₂-, -O-, -S-, -OCH₂-, -SCH₂- or -CH=CH-;
R^{1d} is selected from the group consisting of H, hydroxy, -CH₂OH or a C₁₋₄ alkyl;
ring A and ring C are each independently selected from the group consisting of a C₆₋₁₀ carbocycle or a 5- to 10-membered heterocycle which is optionally further substituted with 0 to 4 substituents selected from the group consisting of F, Cl, Br, I, a C₁₋₄ alkyl or a C₁₋₄ alkoxy, wherein the heterocycle contains 1 to 3 heteroatoms selected from the group consisting of N, O or S;
R^{1e} and R^{1f} are each independently selected from the group consisting of F, Cl, Br, I, CF₃, NH₂, OH, carboxyl, cyano, a C₁₋₄ alkyl, a C₁₋₄ alkoxy, a C₁₋₄ alkylthio, -NHC₁₋₄ alkyl, -N(C₁₋₄ alkyl)₂, -S(=O)-C₁₋₄ alkyl, -S(=O)₂-C₁₋₄ alkyl, or -C(=O)O-C₁₋₄ alkyl;
R² is selected from the group consisting of a C₁₋₆ alkylene, a C₂₋₆ alkenylene or a C₂₋₆ alkynylene, wherein the alkylene, alkenylene or alkynylene is optionally further substituted with 0 to 5 substituents selected from the group consisting of F, Cl, Br, I, OH, cyano, a C₁₋₄ alkyl, a C₁₋₄ alkoxy, phenyl or phenyl-C₁₋₄ alkylene;
W₁ is selected from the group consisting of a bond, -O-, -C(=O)O-, -OC(=O)-, -S-, -S(=O)-, -S(=O)₂-, -C(=O)NR^{w}-, -NR^{w}C(=O)-, -OC(=O)NR^{w}-, -NR^{w}C(=O)O-, -NR^{w}C(=O)NR^{w}-, -NR^{w}S(=O)₂-, -S(=O)₂NR^{w}-, -NR^{w}S(=O)₂NR^{w}-, or -NR^{w}-;
W₂ is selected from the group consisting of a bond or 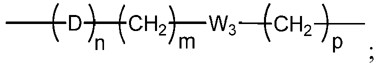
D is selected from the group consisting of a C₆₋₁₀ carbocyclene group or a 5- to 10-membered heterocyclene group, wherein the heterocyclene group contains 1 to 3 heteroatoms selected from the group consisting of N, O or S, and the carbocyclene or heterocyclene group is optionally further substituted with 0 to 4 substituents selected from the group consisting of F, Cl, Br, I, OH, NH₂, carboxyl, cyano, nitro, a C₁₋₄ alkyl, a C₂₋₄ alkynyl, a C₁₋₄ alkoxy, a -OC₃₋₆ cycloalkyl, a C₁₋₄ alkylthio, a -S(=O)-C₁₋₄ alkyl, a -S(=O)₂-C₁₋₄ alkyl, a -C(=O)-C₁₋₄ alkyl, a -C(=O)O-C₁₋₄ alkyl, a -OC(=O)-C₁₋₄ alkyl or a -C(=O)NH₂, where the alkyl, alkoxy, cycloalkyl, alkynyl, NH₂ and -C(=O)NH₂ are optionally further substituted with 0 to 4 substituents selected from the group consisting of F, Cl, Br, I, CF₃, a C₁₋₄ alkyl, a C₁₋₄ alkoxy or a -C(=O)-C₁₋₄ alkyl;
W₃ is selected from the group consisting of a bond, -O-, -C(=O)O-, -OC(=O)-, -S-, -S(=O)-, -S(=O)₂-, -C(=O)NR^{w}-, -NR^{w}C(=O)-, -OC(=O)NR^{w}-, -NR^{w}C(=O)O-, -NR^{w}C(=O)NR^{w}-, -NR^{w}S(=O)₂-, -S(=O)₂NR^{w}-, -NR^{w}S(=O)₂NR^{w}-, or -NR^{w}-;
R^{W} is selected from the group consisting of H or a C₁₋₄ alkyl;
X, Y and Z are each independently selected from the group consisting of -N-, -NR^{x1}-, -CR^{x1}R^{x2}-, -CR^{x1}R^{x2}CR^{x3}R^{x4}-, -CR^{x1}=CR^{x2}-, -S-, -O-, and -C(O)-;
R^{x1}, R^{x2}, R^{x3} or R^{x4} is each independently selected from the group consisting of H or a C₁₋₄ alkyl;
R³ is each independently selected from the group consisting of F, Cl, Br, I, OH, NH₂, carboxy, cyano, nitro, a C₁₋₄ alkyl, a C₂₋₄ alkenyl, a C₂₋₄ alkynyl, a C₁₋₄ alkoxy, a -OC₃₋₆ cycloalkyl, a C₁₋₄ alkylthio, a -S(=O)-C₁₋₄ alkyl, a -S(=O)₂-C₁₋₄ alkyl, a -C(=O)-C₁₋₄ alkyl, a -C(=O)O-C₁₋₄ alkyl, a -OC(=O)-C₁₋₄ alkyl or a -C(=O)NH₂, wherein the alkyl, alkoxy, cycloalkyl, alkenyl, alkynyl, NH₂ and -C(=O)NH₂ are optionally further substituted with 0 to 4 substituents selected from the group consisting of F, Cl, Br, I, CF₃, a C₁₋₄ alkyl, a C₁₋₄ alkoxy or a -C(=O)-C₁₋₄ alkyl;
R⁴ is selected from a C₁₋₆ alkylene which is optionally further substituted with 0 to 5 substituents selected from R^{4a};
R^{4a} is selected from the group consisting of F, Cl, Br, I, cyano, OH, a C₁₋₄ alkyl, a C₁₋₄ alkoxy, phenyl or phenyl-C₁₋₄ alkylene;
alternatively, two R^{4a}s may form a 3- to 6-membered carbocycle together with the atoms to which they are attached, wherein the carbocycle is optionally further substituted with 0 to 5 substituents selected from F, Cl, Br, I, cyano, OH, a C₁₋₄ alkyl or a C₁₋₄ alkoxy;
R⁵ and R⁶ are each independently selected from the group consisting of H or a C₁₋₄ alkyl; 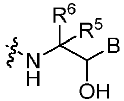 represents a β-adrenergic receptor binding group;
in general formula (I), "------" is a single or double bond;
a is 0, 1, 2, 3, 4 or 5;
b is 0, 1, 2, 3 or 4;
c is 0, 1, 2, 3 or 4;
n is 0 or 1;
m is 0, 1, 2, 3, 4, 5 or 6, with the proviso that when n is 0, m is 1, 2, 3, 4, 5 or 6; and
p is 0, 1, 2, 3, 4, 5 or 6,
or a stereoisomer, a hydrate, a metabolite, a solvate, a pharmaceutically acceptable salt, a cocrystal, or a prodrug thereof.

2. The compound according to claim 1, wherein
B is selected from the group consisting of 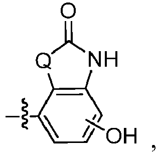 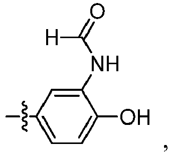 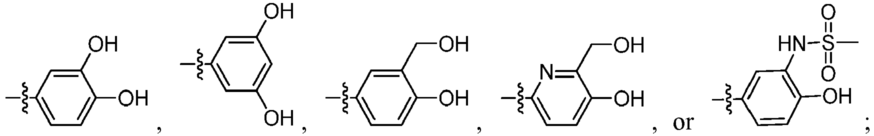 where Q is selected from the group consisting of -CH=CH-, -CH₂CH₂-, -O-, -S-, -CH₂O-, -OCH₂-, -C(CH₃)₂O- or -OC(CH₃)₂-,
or a stereoisomer, a hydrate, a metabolite, a solvate, a pharmaceutically acceptable salt, a cocrystal, or a prodrug thereof.

3. The compound according to claim 2, wherein
R^{1a} and R^{1b} are each independently selected from the group consisting of H, a C₂₋₄ alkenyl, a C₂₋₄ alkynyl, a C₃₋₇ carbocycle, a 3- to 6-membered heterocycle, a C₃₋₇ carbocyclyl-C₁₋₄ alkylene, or a 3- to 6-membered heterocyclyl-C₁₋₄ alkylene, wherein the alkenyl, alkynyl, alkylene, carbocycle or heterocycle is optionally further substituted with 0 to 4 substituents selected from the group consisting of F, Cl, Br, I, CF₃, OH, NH₂, carboxyl, cyano, nitro, a C₁₋₄ alkyl, a C₁₋₄ alkoxy, a -O(=O)C₁₋₄ alkyl, a -(=O)C₁₋₄ alkyl, a -OC₃₋₆ cycloalkyl or a C₁₋₄ alkylthio, wherein the heterocycle contains 1 to 3 hetero atoms selected from the group consisting of N, O or S; with the proviso that R^{1a} and R^{1b} are not both H;
R^{1c} is selected from the group consisting of H, hydroxy, cyano, NH₂, a C₁₋₄ alkyl, a C₁₋₄ alkoxy, a C₁₋₄ alkylthio or -C(=O)NH₂, wherein the NH₂, -C(=O)NH₂, or alkyl is optionally further substituted with 0 to 2 substituents selected from the group consisting of F, Cl, Br, I, hydroxy, cyano or a C₁₋₄ alkyl;
ring A and ring C are each independently selected from a benzene ring, wherein the benzene ring is optionally further substituted with 0 to 4 substituents selected from the group consisting of F, Cl, Br, I, a C₁₋₄ alkyl or a C₁₋₄ alkoxy;
R^{1e} and R^{1f} are each independently selected from the group consisting of F, Cl, Br, I, CF₃, NH₂, OH, cyano, a C₁₋₄ alkyl, a C₁₋₄ alkoxy, a C₁₋₄ alkylthio, -NHC₁₋₄ alkyl, or -N(C₁₋₄ alkyl)₂;
D is a phenylene which is optionally further substituted with 0 to 4 substituents selected from the group consisting of F, Cl, Br, I, OH, NH₂, carboxyl, cyano, nitro, a C₁₋₄ alkyl, a C₂₋₄ alkynyl, a C₁₋₄ alkoxy, a -OC₃₋₆ cycloalkyl, a C₁₋₄ alkylthio, a -S(=O)-C₁₋₄ alkyl, a -S(=O)₂-C₁₋₄ alkyl, a -C(=O)-C₁₋₄ alkyl, or a -C(=O)O-C₁₋₄ alkyl, where the alkyl, alkynyl, alkoxy, cycloalkyl, and NH₂ are optionally further substituted with 0 to 4 substituents selected from the group consisting of F, Cl, Br, I, CF₃, a C₁₋₄ alkyl, a C₁₋₄ alkoxy or a -C(=O)-C₁₋₄ alkyl; and
R³ is each independently selected from the group consisting of F, Cl, Br, I, OH, NH₂, carboxy, cyano, nitro, a C₁₋₄ alkyl, a C₂₋₄ alkynyl, a C₁₋₄ alkoxy, a -OC₃₋₆ cycloalkyl, a C₁₋₄ alkylthio, a -S(=O)-C₁₋₄ alkyl, a -S(=O)₂-C₁₋₄ alkyl, a -C(=O)-C₁₋₄ alkyl, or a -C(=O)O-C₁₋₄ alkyl, wherein the alkyl, alkynyl, alkoxy, cycloalkyl, and NH₂ are optionally further substituted with 0 to 4 substituents selected from the group consisting of F, Cl, Br, I, CF₃, a C₁₋₄ alkyl, a C₁₋₄ alkoxy or a -C(=O)-C₁₋₄ alkyl,
or a stereoisomer, a hydrate, a metabolite, a solvate, a pharmaceutically acceptable salt, a cocrystal, or a prodrug thereof.

4. The compound according to claim 3, wherein
R¹ is selected from the group consisting of 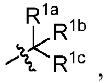 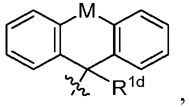 or 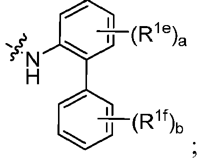
R^{1a} and R^{1b} are each independently selected from the group consisting of H, vinyl, ethynyl, propynyl, phenyl, cyclopropyl, cyclobutyl, cyclopentyl, cyclohexyl, cycloheptyl, benzyl, thienyl, furyl or pyridyl, wherein the vinyl, ethynyl, propynyl, phenyl, cyclopropyl, cyclobutyl, cyclopentyl, cyclohexyl, cycloheptyl, benzyl, thienyl, furyl or pyridyl is optionally further substituted with 0 to 4 substituents selected from the group consisting of F, Cl, Br, I, CF₃, OH, NH₂, carboxyl, cyano, nitro, a C₁₋₄ alkyl, a C₁₋₄ alkoxy, or a -O(=O)C₁₋₄ alkyl; with the proviso that R^{1a} and R^{1b} are not both H;
R^{1c} is selected from the group consisting of H, hydroxy, NH₂, methyl, ethyl, cyano, methylamino, dimethylamino, methoxy, ethoxy, -CH₂OH or -C(=O)NH₂;
R^{1d} is selected from the group consisting of H, hydroxy, -CH₂OH, methyl or ethyl;
R^{1e} and R^{1f} are each independently selected from the group consisting of F, Cl, Br, I, CF₃, NH₂, OH, cyano, methyl, ethyl, methoxy, ethoxy, -NHCH₃ or -N(CH₃)₂;
M is selected from the group consisting of a bond, -O- or -S-;
R² is selected from the group consisting of methylene, ethylene, propylene, butylene or pentylene which is optionally further substituted with 0 to 5 substituents selected from the group consisting of F, Cl, Br, I, OH, cyano, methyl, ethyl, methoxy, or ethoxy;
W₁ is selected from the group consisting of a bond, -O-, -C(=O)NR^{w}-, -NR^{w}C(=O)-, -OC(=O)NR^{w}- or -NR^{w}C(=O)O-;
D is selected from the group consisting of 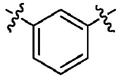 or 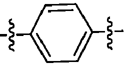 which is optionally further substituted with 0 to 4 substituents selected from the group consisting of F, Cl, Br, cyano, methyl, ethyl, propyl, isopropyl, ethynyl, CHF₂, CF₃, methoxy, ethoxy, -OCHF₂ or -OCF₃;
W₃ is selected from the group consisting of a bond, -O-, -C(=O)NR^{w}-, -NR^{w}C(=O)-, -OC(=O)NR^{w}- or -NR^{w}C(=O)O-;
R^{W} is selected from the group consisting of H, methyl, ethyl, or propyl;
X, Y and Z are each independently selected from the group consisting of -NH-, -N-, -CH₂-, -CH₂CH₂-, -CH-, -CH=CH-, -S-, -O- and -C(O)-;
R³ is each independently selected from the group consisting of F, Cl, Br, cyano, methyl, ethyl, propyl, isopropyl, ethynyl, CHF₂, CF₃, methoxy, ethoxy, -OCHF₂ or -OCF₃;
R⁴ is selected from the group consisting of methylene, ethylene, propylene, butylene,
pentylene or 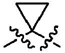 which is optionally further substituted with 0 to 5 substituents selected from the group consisting of F, Cl, Br, I, cyano, OH, methyl, ethyl, methoxy or ethoxy;
R⁵ and R⁶ are each independently selected from the group consisting of H, methyl or ethyl;
B is selected from the group consisting of 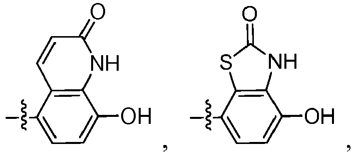 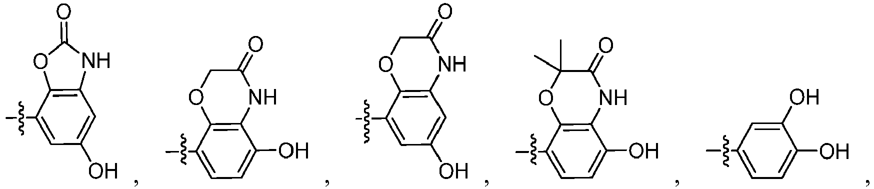 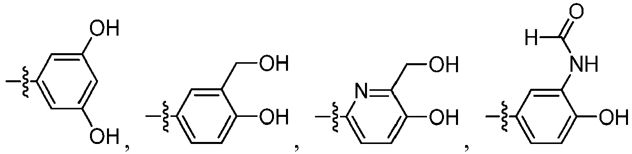 or 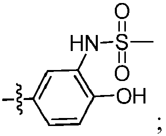
a is 0 or 1;
b is 0, 1, or 2;
c is 0, 1, or 2;
n is 0 or 1;
m is 0, 1, 2, 3, 4, or 5, with the proviso that when n is 0, m is 1, 2, 3, 4, or 5; and
p is 0, 1, 2, 3, or 4,
or a stereoisomer, a hydrate, a metabolite, a solvate, a pharmaceutically acceptable salt, a cocrystal, or a prodrug thereof.

5. The compound according to claim 4, wherein
R¹ is selected from the group consisting of 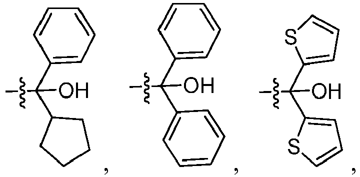 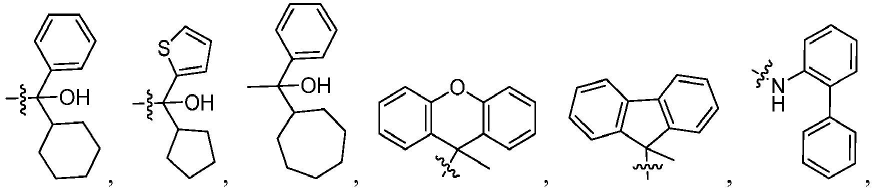 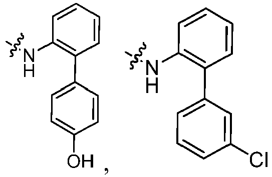 or 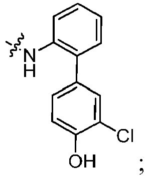
R² is selected from the group consisting of methylene, ethylene, propylene, -CH₂CH(CH₃)-, -CH(CH₃)CH₂-, butylene, -CH(CH₃)CH₂CH₂-, -CH₂CH(CH₃)CH₂-, -CH₂CH(CH₃)CH₂- or pentylene;
D is selected from the group consisting of 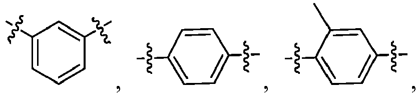 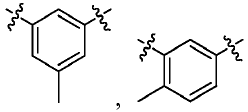 or 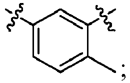
R³ is each independently selected from the group consisting of F, Cl, Br, CHF₂, CF₃, cyano, methyl, ethyl, ethynyl, methoxy, ethoxy, -OCHF₂ or -OCF₃;
R⁴ is selected from the group consisting of methylene, ethylene, propylene, -CH₂CH(CH₃)-, -CH(CH₃)CH₂-, butylene, -CH(CH₃)CH₂CH₂-, -CH₂CH(CH₃)CH₂-, -CH₂CH(CH₃)CH₂-, pentylene or 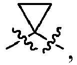
or a stereoisomer, a hydrate, a metabolite, a solvate, a pharmaceutically acceptable salt, a cocrystal, or a prodrug thereof.

6. The compound according to claim 5, wherein the compound is selected from 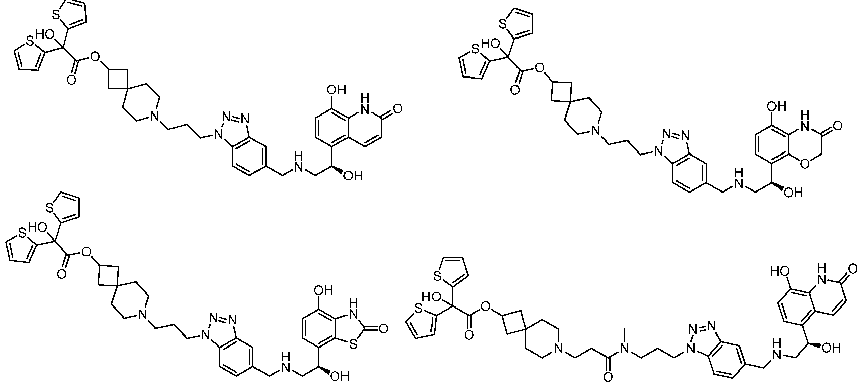 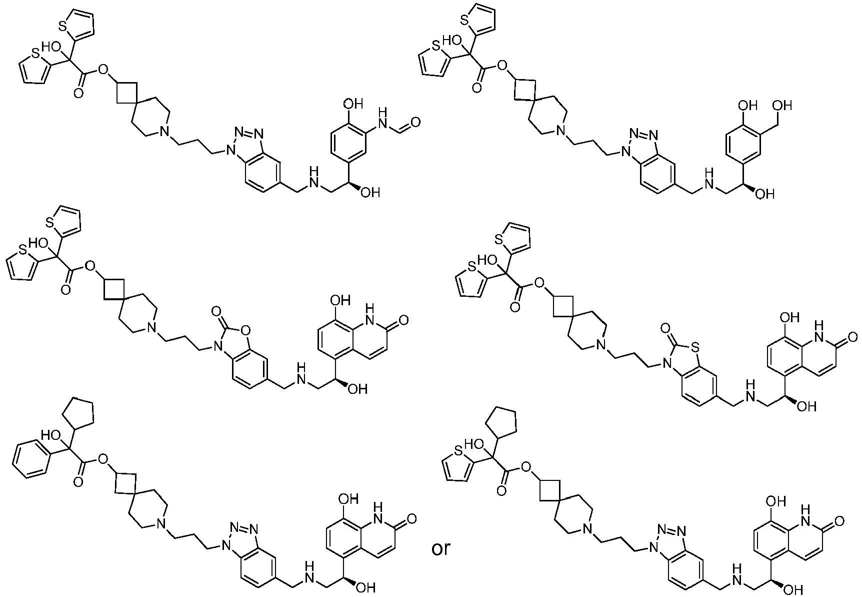 or a stereoisomer, a hydrate, a metabolite, a solvate, a pharmaceutically acceptable salt, a cocrystal, or a prodrug thereof.

7. A pharmaceutical composition comprising: i) a therapeutically effective amount of a compound according to any one of claims 1-6, or a stereoisomer, a hydrate, a metabolite, a solvate, a pharmaceutically acceptable salt, a cocrystal, or a prodrug thereof; and ii) a pharmaceutically acceptable carrier, diluent, adjuvant, vehicle, or excipient; wherein the composition optionally further comprises one or more secondary therapeutic agents.

8. The pharmaceutical composition according to claim 7, wherein the secondary therapeutic agent is selected from the group consisting of one or more of PDE4 inhibitors, muscarinic receptor antagonists, corticosteroids and β-adrenergic receptor agonists.

9. Use of a compound according to any one of claims 1-6, or a stereoisomer, a hydrate, a metabolite, a solvate, a pharmaceutically acceptable salt, a cocrystal, or a prodrug thereof, or a pharmaceutical composition according to claims 7 or 8, in the manufacture of a medicament for treatment of an airway obstructive disease.

10. Use of a compound according to any one of claims 1-6, or a stereoisomer, a hydrate, a metabolite, a solvate, a pharmaceutically acceptable salt, a cocrystal, or a prodrug thereof, or a pharmaceutical composition according to claims 7 or 8, in the manufacture of a medicament for treatment of asthma, chronic obstructive pulmonary disease (COPD) or bronchitis.

11. A method for treating an airway obstructive disease, comprising administrating a therapeutically effective amount of a compound according to any one of claims 1-6, or a stereoisomer, a hydrate, a metabolite, a solvate, a pharmaceutically acceptable salt, a cocrystal, or a prodrug thereof, or a pharmaceutical composition according to claim 7 or 8.

12. A method for treating asthma, chronic obstructive pulmonary disease (COPD) or bronchitis, comprising administrating a therapeutically effective amount of a compound according to any one of claims 1-6, or a stereoisomer, a hydrate, a metabolite, a solvate, a pharmaceutically acceptable salt, a cocrystal, or a prodrug thereof, or a pharmaceutical composition according to claim 7 or 8.

13. An intermediate for preparing a compound of general formula (I) or a stereoisomer thereof, said intermediate being selected from compounds of general formula (II) or a stereoisomer thereof: wherein: 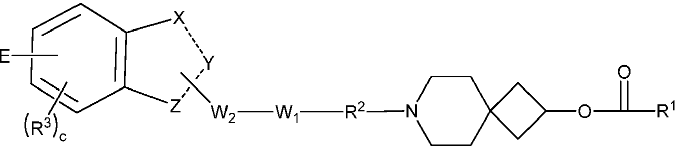
R¹ is selected from the group consisting of 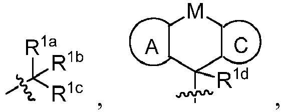 or 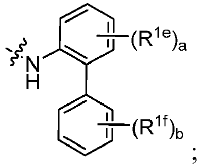
R^{1a} and R^{1b} are each independently selected from the group consisting of H, a C₂₋₆ alkenyl, a C₂₋₆ alkynyl, a C₃₋₁₀ carbocycle, a 3- to 8-membered heterocycle, a C₃₋₁₀ carbocyclyl-C₁₋₄ alkylene, or a 3- to 8-membered heterocyclyl-C₁₋₄ alkylene, wherein the alkenyl, alkynyl, alkylene, carbocycle or heterocycle is optionally further substituted with 0 to 4 substituents selected from the group consisting of F, Cl, Br, I, CH₂F, CHF₂, CF₃, OH, OCH₂F, OCHF₂, OCF₃, NH₂, carboxyl, cyano, nitro, a C₁₋₄ alkyl, a C₁₋₄ alkoxy, a -O(=O)C₁₋₄ alkyl, a -(=O)C₁₋₄ alkyl, a -OC₃₋₆ cycloalkyl or a C₁₋₄ alkylthio, wherein the heterocycle contains 1 to 3 hetero-atoms selected from the group consisting of N, O or S; with the proviso that R^{1a} and R^{1b} are not both H;
R^{1c} is selected from the group consisting of H, hydroxy, cyano, NH₂, a C₁₋₆ alkyl, a C₁₋₆ alkoxy, a C₁₋₆ alkylthio or -C(=O)NH₂, wherein the NH₂, -C(=O)NH₂, or alkyl is optionally further substituted with 0 to 2 substituents selected from the group consisting of F, Cl, Br, I, hydroxy, cyano or a C₁₋₄ alkyl;
M is selected from the group consisting of a bond, -CH₂-, -CH₂CH₂-, -O-, -S-, -OCH₂-, -SCH₂- or -CH=CH-;
R^{1d} is selected from the group consisting of H, hydroxy, -CH₂OH or a C₁₋₄ alkyl;
ring A and ring C are each independently selected from the group consisting of a C₆₋₁₀ carbocycle or a 5- to 10-membered heterocycle which is optionally further substituted with 0 to 4 substituents selected from the group consisting of F, Cl, Br, I, a C₁₋₄ alkyl or a C₁₋₄ alkoxy, wherein the heterocycle contains 1 to 3 heteroatoms selected from N, O or S;
R^{1e} and R^{1f} are each independently selected from the group consisting of F, Cl, Br, I, CF₃, NH₂, OH, carboxyl, cyano, a C₁₋₄ alkyl, a C₁₋₄ alkoxy, a C₁₋₄ alkylthio, -NHC₁₋₄ alkyl, -N(C₁₋₄ alkyl)₂, -S(=O)-C₁₋₄ alkyl, -S(=O)₂-C₁₋₄ alkyl, or -C(=O)O-C₁₋₄ alkyl;
R² is selected from the group consisting of a C₁₋₆ alkylene, a C₂₋₆ alkenylene or a C₂₋₆ alkynylene, wherein the alkylene, alkenylene or alkynylene is optionally further substituted with 0 to 5 substituents selected from the group consisting of F, Cl, Br, I, OH, cyano, a C₁₋₄ alkyl, a C₁₋₄ alkoxy, phenyl or phenyl-C₁₋₄ alkylene;
W₁ is selected from the group consisting of a bond, -O-, -C(=O)O-, -OC(=O)-, -S-, -S(=O)-, -S(=O)₂-, -C(=O)NR^{w}-, -NR^{w}C(=O)-, -OC(=O)NR^{w}-, -NR^{w}C(=O)O-, -NR^{w}C(=O)NR^{w}-, -NR^{w}S(=O)₂-, -S(=O)₂NR^{w}-, -NR^{w}S(=O)₂NR^{w}-, or -NR^{w}-;
W₂ is selected from the group consisting of a bond or 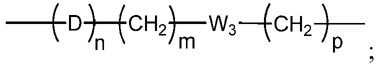
D is selected from the group consisting of a C₆₋₁₀ carbocyclene group or a 5- to 10-membered heterocyclene group, wherein the heterocyclene group contains 1 to 3 heteroatoms selected from N, O or S, and the carbocyclene or heterocyclene group is optionally further substituted with 0 to 4 substituents selected from the group consisting of F, Cl, Br, I, OH, NH₂, carboxyl, cyano, nitro, a C₁₋₄ alkyl, a C₂₋₄ alkynyl, a C₁₋₄ alkoxy, a -OC₃₋₆ cycloalkyl, a C₁₋₄ alkylthio, a -S(=O)-C₁₋₄ alkyl, a -S(=O)₂-C₁₋₄ alkyl, a -C(=O)-C₁₋₄ alkyl, a -C(=O)O-C₁₋₄ alkyl, a -OC(=O)-C₁₋₄ alkyl or a -C(=O)NH₂, where the alkyl, alkoxy, cycloalkyl, alkynyl, NH₂ and -C(=O)NH₂ are optionally further substituted with 0 to 4 substituents selected from the group consisting of F, Cl, Br, I, CF₃, a C₁₋₄ alkyl, a C₁₋₄ alkoxy or a -C(=O)-C₁₋₄ alkyl;
W₃ is selected from the group consisting of a bond, -O-, -C(=O)O-, -OC(=O)-, -S-, -S(=O)-, -S(=O)₂-, -C(=O)NR^{w}-, -NR^{w}C(=O)-, -OC(=O)NR^{w}-, -NR^{w}C(=O)O-, -NR^{w}C(=O)NR^{w}-, -NR^{w}S(=O)₂-, -S(=O)₂NR^{w}-, -NR^{w}S(=O)₂NR^{w}-, or -NR^{w}-;
R^{W} is selected from the group consisting of H or a C₁₋₄ alkyl;
X, Y and Z are each independently selected from the group consisting of -N-, -NR^{x1}-, -CR^{x1}R^{x2}-, -CR^{x1}R^{x2}CR^{x3}R^{x4}-, -CR^{x1}=CR^{x2}-, -S-, -O-, and -C(O)-;
R^{x1}, R^{x2}, R^{x3} or R^{x4} is each independently selected from the group consisting of H or a C₁₋₄ alkyl;
R³ is each independently selected from the group consisting of F, Cl, Br, I, OH, NH₂, carboxy, cyano, nitro, a C₁₋₄ alkyl, a C₂₋₄ alkenyl, a C₂₋₄ alkynyl, a C₁₋₄ alkoxy, a -OC₃₋₆ cycloalkyl, a C₁₋₄ alkylthio, a -S(=O)-C₁₋₄ alkyl, a -S(=O)₂-C₁₋₄ alkyl, a -C(=O)-C₁₋₄ alkyl, a -C(=O)O-C₁₋₄ alkyl, a -OC(=O)-C₁₋₄ alkyl or a -C(=O)NH₂, wherein the alkyl, alkoxy, cycloalkyl, alkenyl, alkynyl, NH₂ and -C(=O)NH₂ are optionally further substituted with 0 to 4 substituents selected from the group consisting of F, Cl, Br, I, CF₃, a C₁₋₄ alkyl, a C₁₋₄ alkoxy or a -C(=O)-C₁₋₄ alkyl;
E is selected from the group consisting of formyl, 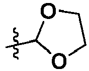 or 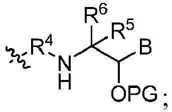
R⁴ is selected from a C₁₋₆ alkylene which is optionally further substituted with 0 to 5 substituents selected from R^{4a};
R^{4a} is selected from the group consisting of F, Cl, Br, I, cyano, OH, a C₁₋₄ alkyl, a C₁₋₄ alkoxy, phenyl or phenyl-C₁₋₄ alkylene;
alternatively, two R^{4a}s may form a 3- to 6-membered carbocycle together with the atoms to which they are attached, wherein the carbocycle is optionally further substituted with 0 to 5 substituents selected from the group consisting of F, Cl, Br, I, cyano, OH, a C₁₋₄ alkyl or a C₁₋₄ alkoxy;
R⁵ and R⁶ are each independently selected from the group consisting of H or a C₁₋₄ alkyl;
PG is a protective group for hydroxy; 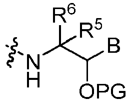 represents a β-adrenergic receptor binding group wherein the hydroxyl is protected;
in formula (II), "------" is a single or double bond;
a is 0, 1, 2, 3, 4 or 5;
b is 0, 1, 2, 3 or 4;
c is 0, 1, 2, 3 or 4;
n is 0 or 1;
m is 0, 1, 2, 3, 4, 5 or 6, with the proviso that when n is 0, m is 1, 2, 3, 4, 5 or 6; and
p is 0, 1, 2, 3, 4, 5 or 6.

14. The intermediate according to claim 13, wherein R¹ is selected from the group consisting of 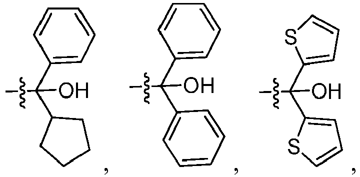 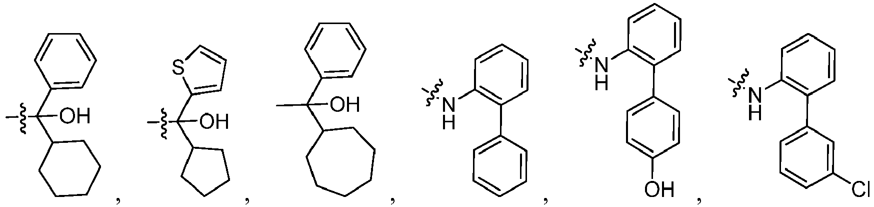 or 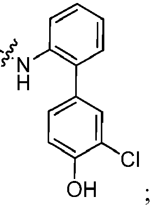
R² is selected from the group consisting of methylene, ethylene, propylene, -CH₂CH(CH₃)-, -CH(CH₃)CH₂-, butylene, -CH(CH₃)CH₂CH₂-, -CH₂CH(CH₃)CH₂-, -CH₂CH(CH₃)CH₂- or pentylene;
W₁ is selected from the group consisting of a bond, -O-, -C(=O)NR^{w}-, -NR^{w}C(=O)-, -OC(=O)NR^{w}- or -NR^{w}C(=O)O-;
D is selected from the group consisting of 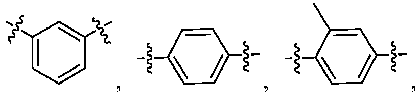 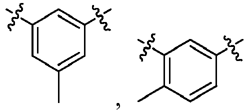 or 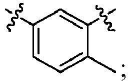
W₃ is selected from the group consisting of a bond, -O-, -C(=O)NR^{w}-, -NR^{w}C(=O)-, -OC(=O)NR^{w}- or -NR^{w}C(=O)O-;
R^{W} is selected from the group consisting of H, methyl, ethyl, or propyl;
X, Y and Z are each independently selected from the group consisting of -NH-, -N-, -CH₂-, -CH₂CH₂-, -CH-, -CH=CH-, -S-, -O- and -C(O)-;
R³ is selected from the group consisting of F, Cl, Br, CHF₂, CF₃, cyano, methyl, ethyl, ethynyl, methoxy, ethoxy, -OCHF₂ or -OCF₃;
R⁴ is selected from the group consisting of methylene, ethylene, propylene, -CH₂CH(CH₃)-, -CH(CH₃)CH₂-, butylene, -CH(CH₃)CH₂CH₂-, -CH₂CH(CH₃)CH₂-, -CH₂CH(CH₃)CH₂-, 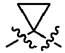 or pentylene;
R⁵ and R⁶ are each independently selected from the group consisting of H, methyl or ethyl;
B is selected from the group consisting of 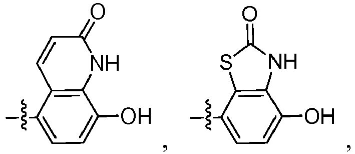 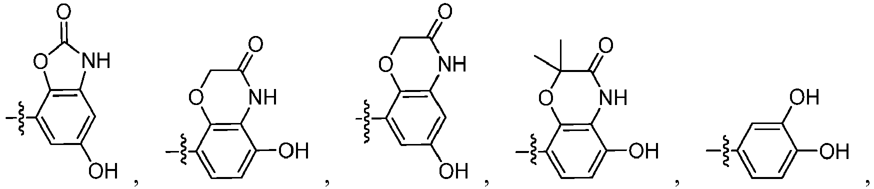 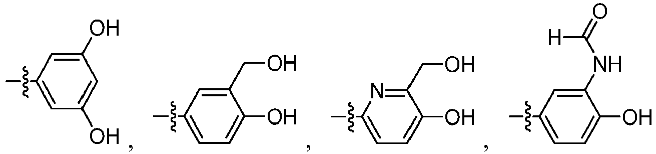 or 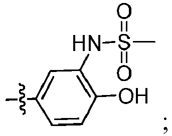 and
PG is TBS.

15. The intermediate according to claim 14, selected from
